# EUROPEAN PATENT APPLICATION

(11) **EP 4 303 233 A1**
(43) Date of publication of application: **10.01.2024**
(21) Application number: 22745373.5
(22) Date of filing: 01.02.2022
(51) Int. Cl.: C07K 16/28, C07K 14/705, C07K 14/725

(54) **TARGETED PROTEIN DEGRADATION SYSTEM AND USE THEREOF**

(30) Priority: 01.02.2021 US 202117164834; 17.02.2021 US 202163150577 P; 30.07.2021 CN 202110877843
(71) Applicant: ST PHI THERAPEUTICS CO., LTD., Hangzhou, Zhejiang 310056 (CN)
(72) Inventor: LIU, Lingfeng, Hangzhou, Zhejiang 310056 (CN); ZHONG, Wenting, Hangzhou, Zhejiang 310056 (CN); LIU, Liling, Hangzhou, Zhejiang 310056 (CN)
(74) Representative: Hoffmann Eitle
(86) International application number: PCT/CN2022/075302
(87) International publication number: WO 2022/161502

(57) **Abstract**

An endoplasmic reticulum-based chimeric protein construct for targeted protein degradation and the use thereof, wherein the chimeric protein construct is used for treating cancers, viral infection diseases, autoimmune diseases, neurodegenerative diseases, etc. The chimeric protein construct includes a protein binding domain based on an endoplasmic reticulum-associated degradation mechanism and a targeting domain. The protein binding domain of the chimeric protein construct based on the ERAD mechanism can be the transmembrane domain of the endoplasmic reticulum resident protein of a virus or a functional variant thereof and an endoplasmic reticulum resident domain or a functional variant thereof. The targeting domain of the chimeric protein construct can target any target proteins of interest, and can be the natural ligand of the target protein, an antibody that specifically recognizes the target protein, or an antigen-binding fragment thereof. The targeting domain of the chimeric protein construct can also be an antigen that can be specifically recognized by an antibody.

## Description

### TECHNICAL FIELD

The present disclosure relates to a targeted protein degradation system and use thereof, and in particular, to use thereof in the treatment of diseases (including but not limited to cancer, viral infectious diseases, autoimmune diseases, neurodegenerative diseases).

### BACKGROUND

Targeted protein removal is desirable for at least the following reasons: 1) to determine the function of a protein in basic research; 2) to validate a therapeutic target; 3) to block a pathogenic protein (e.g., tumor proteins or proteins causing neurodegeneration) action; 4) to enhance the degradation of proteins accumulated during aging; and 5) to reduce the side effects of cell therapy (e.g., preventing the expression of immunogenic proteins). However, existing targeted protein removal technologies all have their own inherent technical problems

Gene editing technologies (for example, CRISPR technologies) are capable of target-knocking out genes encoding any target protein at gene level, but there are off-target problems, including low specificity in identifying and cutting desired DNA sites, chromosomal instability, and other side effects.

Small interfering RNA (siRNA) can prevent the synthesis of a target protein at RNA level. However, siRNA also has potential off-target effects. For example, a sense strand of a siRNA may mediate expression silencing of a cognate gene, causing off-target effects mediated by the sense strand. In addition, small mismatches in small nucleic acid sequences may also lead to other gene silencing, resulting in off-target effects and toxic side effects.

Protein degradation targeting chimera technologies (PROTACs) are capable of degrading target proteins at protein level, and usually added to cells or administered to animals or humans as a biochemical reagent to induce degradation of target proteins in cells. Early PROTACs are based on polypeptide ligand molecules bound by E3 ligase, but PROTACs prepared by using polypeptide ligand molecules has low cell permeability and are unstable, resulting in low degradation efficiency. Although PROTACs using small molecules as E3 ligase ligands have shown remarkable effectiveness, their membrane permeability and bioavailability are poor.

Therefore, there is an urgent need for a new targeted protein degradation system to overcome at least one problem existing in various prior art for targeted removal of target protein.

### SUMMARY

In one aspect, the present application provides a chimeric protein construct that degrades a target protein through an endoplasmic reticulum-associated degradation (ER-associated degradation, ERAD) mechanism, including an ERAD mechanism protein binding domain and a targeting domain. In some embodiments, the present application provides a general target protein degradation component that may hijack the ERAD mechanism to prevent transport of target proteins in ER and facilitate their translocation into cytoplasm, to conduct lysosome endocytosis and degradation of a target protein through an ubiquitin-based proteasome (UPS) mechanism, proteasome ubiquitination and degradation of the target protein, and/or an ALP mechanism of the autophagy lysosome. This design may target any protein, including endogenous or exogenous proteins, to effectively inhibit expression and rapidly degrade for therapeutic purposes. The present application targets various endogenous target proteins or exogenous target proteins by redesigning viral elements to achieve retention and directional degradation of target proteins in the endoplasmic reticulum.

In another aspect, the present application provides a chimeric protein construct synergistically degrading a target protein through an ERAD mechanism and a ubiquitination mechanism, including an ERAD mechanism protein binding domain, a targeting domain, as well as an additional protein degradation pathway member-binding domain. This design can greatly improve degradation efficiency of a target protein, and is suitable for many targets that cannot be degraded by PROTAC technologies.

The above two chimeric protein constructs are also referred to as TPD (Targeted Protein Degradation) chimeric protein constructs in the present application The present application also provides a nucleic acid, nucleic acid vector, oncolytic virus that encode the above-mentioned chimeric protein construct, and cells expressing the above-mentioned chimeric protein construct, and the use thereof for treating diseases.

Embodiment1. A chimeric protein construct comprising an endoplasmic reticulum-associated degradation (ER-associated degradation, ERAD) mechanism protein binding domain and a targeting domain.

Embodiment 2. The chimeric protein construct of Embodiment 1, wherein the ERAD mechanism protein binding domain comprises: a transmembrane domain of a viral endoplasmic reticulum resident protein or a functional variant thereof, and, an endoplasmic reticulum resident domain or a functional variant thereof.

Embodiment 3. The chimeric protein construct of Embodiment 1 or 2, wherein the viral endoplasmic reticulum resident protein is adenovirus E3-19K.

Embodiment 4. The chimeric protein construct of Embodiment 1 or 2, wherein the viral endoplasmic reticulum resident protein is not adenovirus E3-19K.

Embodiment 5. The chimeric protein construct of Embodiment 4, wherein the viral endoplasmic reticulum resident protein is selected from at least one of the group consisting of: HCMV glycoprotein US2, US11, US3, US10, US6, HSV ICP47, CPXV12, BHV UL49.5, EBV BNFL2a, HCMV UL16, UL141, UL142, HIV Nef, HIV Vpu, HHV-7 U21, HHV-8 KK3, HHV-8 KK5, MHV-68 MK3, HTLV-1 p12, and Cowpox Virus protein CPXV203.

Embodiment 6. The chimeric protein construct of any one of the preceding Embodiments, further comprising a protein degradation pathway member (e.g. E3 ubiquitin ligase, proteasome, lysosome) binding domain, and optionally, the protein degradation pathway member binding domain being linked to the ERAD mechanism protein binding domain.

Embodiment 7. The chimeric protein construct of any one of the preceding Embodiments, wherein the targeting domain comprises an antibody specifically targeting a target protein or a functional fragment thereof (e.g., Fd, Fv, Fab, Fab', F(ab')2, Fv(scFv), single chain antibody (scFv), nanobody, diabody, triabody or terabody).

Embodiment 8. The chimeric protein construct of Embodiment 7, wherein the target protein is a pathogenic protein, optionally, being a tumor-associated protein, a virus-associated protein, an immune function-related protein (including immunosuppressive and immune stimulatory proteins), an autoantigen protein, or a protein associated with neurodegenerative diseases.

Embodiment 9. The chimeric protein construct of Embodiment 8, wherein the autoantigen protein is selected from a group consisting of: an autoantigen associated with type 1 diabete, e.g., an islet cell antigen (ICA), an insulin (IAA), a glutamine Acid decarboxylase 65 (GAD65), an insulinoma antigen-2 (IA-2); an autoantigen associated with rheumatoid arthritis (RA), e.g., a citrullinated protein/peptide antibody, a heteroribonucleoprotein (heterogeneous nuclear ribonucleoprotein A2/B1), an aldolase, an alpha-enolase, a calreticulin, a heat-activated protein (HSP60), BiP, PGK1, a stress-induced phosphoprotein 1, FUSE-BP1/2; an autoantigen associated with systemic lupus erythematosus (SLE), e.g., a deoxyribonucleoprotein, SmD1, SmD3, Clq, a sore anticoagulant (LA), cardiolipin (CL), β2 glycoprotein I (β2 GPI), a prothrombin (PT) and phosphatidylserine (PS); an autoantigen associated with Systemic Sclerosis (SSc)/scleroderma (SD), e.g., Scl-70, SSA, Ro52; an antigen associated with autoimmune liver diseases, e.g., mitochondrial antigen, Spl00, PML, gp210, p62; an autoantigen associated with myasthenia gravis, e.g., an acetylcholine receptor; an autoantigen associated with central nervous system autoimmune disease (limbic encephalitis, encephalomyelitis, cerebellar ataxia), e.g., voltage-gated potassium channels (VGKC) complex, voltage-gated calcium channel receptor, a-amino-3-hydroxy-5-methylisoxazole-4-propionic acid (AMPA) receptor, γ-aminobutyric acid-B (GABAB ) receptors, glycine receptors; an autoantigen associated with multiple sclerosis, e.g., a myelin basic protein (MBP), a myelin oligodendrocyte glycoprotein (MOG); an autoantigen associated with polymyositis(PM) anddermatomyositis (DM), e.g., Jo-1, Mi-2, PM-Scl, Ro-52; an autoantigen associated with gluten-sensitive enteropathy, e.g., endomysial antibody (EMA), tissue transglutaminase (tTG); an autoantigen associated with anti-NMDAR antibody encephalitis, e.g., N-methyl-D-aspartate receptor; an autoantigen associated with neuromyelitis optica (NMO), e.g., aquaporin 4 (AQP4); and, an autoantigen associated with reproduction, e.g., ovarian antigen, sperm antigen.

Embodiment 10. The chimeric protein construct of Embodiment 9, wherein the tumor-associated protein is encoded by an oncogene selected from a group consisting of: BCL-2, c-MYC, Ras, HER2, BCR/ABL, ABL1/BCR, TGFB1, TLX1, P53, WNT1, WNT2, WT1, αv-β3, PKCa, ABL, BCL1, CD24, CDK4, EGFR/ERBB- 1. HSTF1, INT1/WNT1, INT2, MDM2, MET, MYB, MYC, MYCN, MYCL1, RAFI, NRAS, REL, AKT2, APC, BCL2-ALPHA, BCL2-BETA, BCL3, BCR, BRCA1, BRCA2, CBL, CCND1, CDKN1A, CDKN1C, CDKN2A, CDKN2B, CRK, CRK-II, CSF1R/FMS, DBL, DDOST, PMS-2, PRAD-1, RAF, RHOM-1, RHOM-2, SIS, TAL2, TANI, TIAM1, TSC2, TRK, TSC1, STK11, PTCH, MEN1, MEN2, P57/KIP2, PTEN, HPC1, ATM, XPA/XPG, BCL6, DEK, AKAP13, CDH1, BLM, EWSR1/FLI1, FES, FGF3, FER, FGR, FLI1/ERGB2, FOS, FPS/FES, FRA1, FRA2, FYN, HCK, HEK, HER3/ERBB-2, ERBB-3, HER4/ERBB-4, HST2, INK4A, INK4B, JUN, JUNB, JUND, KIP2, KIT, KRAS2A, KRAS2B, LCK, LYN, MAS, MAX, MCC, MLH1, MOS, MSH2, MYBA, MYBB, NF1, NF2, P53, PDGFB, PIM1, PTC, RBI, RET, ROS1, SKI, SRC1, TALI, TGFBR2, THRA1, THRB, TIAM1, TRK, VAV, VHL, WAF1, WNT2, WT1, YES1, ALK/NPM1, AMI1, AXL, FMS, GIP, GLI, GSP, HOX11, HST, IL3, INT2, KS3, K- SAM, LBC, DCC, DPC4/SMAD4, E-CAD, E2F1/RBAP, ELK1, ELK3, EPH, EPHA1, E2F1, EPHA3, ERG, ETS1, ETS2, LMO-1, LMO-2, L-MYC, LYL1, LYT- 10, MDM-2, MLH1, MLL, MLM, N-MYC, OST, PAX-5, PMS-1, FGF4, FGF6, FANCA, FLI1/ERGB2, FOSL1, FOSL2, GLI, HRAS1, HRX/MLLT1, HRX/MLLT2, KRAS2, MADH4, MASI, MCF2, MLLT1/MLL, MLLT2/HRX, MTG8/RUNX1, MYCLK1, MYH11/CBFB, NFKB2, NOTCH1, NPM1/ALK, NRG/REL, NTRK1, PBX1/TCF3, PML/ RARA, PRCA1, RUNX1, RUNX1/CBFA2T1, SET, SHP2, TCF3/PBX1, TNFa, Clusterin, Survivin, TOEβ, c-fos, c-SRC,Estrogen receptor gene (estrogen receptor ER-α), androgen receptor gene (Androgen receptor, AR) and INT-1, optionally, the tumor-associated protein is selected from the group consisting of: Bcl-2 family members ( Such as: Bcl-2, Bcl-xL and Bcl-w), VEGF/VEGFR, PDGFRβ, EGFR, EGFR mutants, IGF-1R, HDACs, HER2, MYC, KRAS, AFP, CEA, CA199, estrogen receptor ( estrogen receptor ER-α), androgen receptor (Androgen receptor, AR), tyrosine kinases (c-ABL, BCR-ABL, BTK, FAK, PTK6, Wee1, TRK transmembrane receptors), serine/threonine Acid kinase receptors (IRAK4, LRRK2, B-Raf, RIPK2, CDK4/6, CDK7, CDK8, CDK8/19, CDK9, TBK1), protein kinase II (CK2), epigenetic related proteins (BRD2, BRD3, BRD4 , BRDT, TRIM24, BRD9, PBRM1, SMARCA2, SMARCA4, EP300, EZH2, WDR5), adrenomedullin (ADM), DPP3.

Embodiment 11. The chimeric protein construct of Embodiment 8, wherein the pathogenic protein is a virus-associated protein selected from the group consisting of: HBV surface antigen, HBV capsid glycoprotein, HBeAg, HBV DNA polymerase, HBV encoded X protein ( HBx), HIV Gag protein, HIV Env protein, HIV gp120, HIV-1 reverse transcriptase, HIV gp120, HCVNS3-4A protease, HCV RNA polymerase, HCV envelope protein, EBV DNA polymerase, EBV EBNA1 , coronavirus RNA synthetase, coronavirus spike protein, coronavirus envelope protein, coronavirus membrane protein, coronavirus nucleocapsid protein, RNA-dependent RNA polymerase (RdRp), such as the new coronavirus RNA Dependent RNA polymerase, Herpesviruses DNA and RNA polymerase, Herpesvirus capsid glycoprotein, CMV DNA polymerase, CMV capsid glycoprotein, RSV envelope protein, RSV capsid protein, RSV RNA polymerase, Influenza virus RNA polymerase, influenza virus envelope protein, HPV DNA polymerase, or HPV capsid protein.

Embodiment 12. The chimeric protein construct of Embodiment 7, wherein the target protein is an immune function-related protein selected from the group consisting of: an antigen-presenting molecule (e.g., a MHC class I molecule, a MHC class II molecule, a MICA/B molecule, etc.), an antigen recognition molecule (e.g., TCR, CD123, NKG2D, etc.), an immune checkpoint molecule (e.g., PD-1, PD-L1, CTLA4, TIM3, TIGIT, LAG3, A2AR, BTLA, IDO1, IDO2, TDO, KIR, NOX2, VISTA , SIGLEC7, PVR, etc.), an immune stimulatory/co-stimulatory molecule (e.g., CD3, CD80/86, CD28, etc.).

Embodiment 13. The chimeric protein construct of Embodiment 7, wherein the target protein is a target protein related to nervous system disease, and is selected from the group consisting of: Tau, β-amyloid protein (amyloid-β (Aβ)), α Synuclein, mutant huntingtin (mHTT), α-synuclein, TAR RNA binding protein (TARDBP) and FUS RNA binding protein

### (FUS).

Embodiment 14. The chimeric protein construct of Embodiment 2, 4 or 5, wherein the targeting domain does not specifically binding TCR.

Embodiment 15. The chimeric protein construct of Embodiment 14, wherein the targeting domain specifically binds MHC I, MHC II, MICA, MICB, ULBP1, ULBP2, ULBP3, ULBP4, ULBP5 and/or ULBP6; and wherein preferably, MHC I is HLA I; and wherein, preferably, MHC II is HLA II.

Embodiment 16. The chimeric protein construct of Embodiment 4, wherein the targeting domain specifically binds a TCR.

Embodiment 17. The chimeric protein construct of Embodiment 16, wherein the viral endoplasmic reticulum resident protein is selected from the at least one of the group consisting of: HCMV glycoprotein US2, US11, US3, US10, US6, HSV ICP47, CPXV12, BHV UL49.5, EBV BNFL2a, HCMV UL16, UL141, UL142, HIV Nef, HIV Vpu, HHV-7 U21, HHV-8 KK3, HHV-8 KK5, MHV-68 MK3, HTLV-1 p12, and Cowpox Virus protein CPXV203.

Embodiment 18. The chimeric protein construct of any one of the preceding Embodiments, which is linked to at least one co-expression moiety.

Embodiment 19. The chimeric protein construct of Embodiment 18, wherein the at least one co-expression moiety is independently selected from the group consisting of: a complete viral ER resident glycoprotein (e.g., HCMV US2, US3, US 11, US10, adenovirus E3-Kl 9, HCMV US6, HSV ICP47), a chimeric antigen receptor (CAR), a functional T cell receptor (TCR), a chemokine receptor, or a NK-cell-activating receptor.

Embodiment 20. The chimeric protein construct of Embodiment 19, wherein the chemokine receptor is selected from a group consisting of: CCR4, CCR5, CCR6, CCR7, CCR9, CCR2b, CXCR1, CXCR2, or CXCR4.

Embodiment 21. The chimeric protein construct of Embodiment 20, wherein the NK-cell-activating receptor comprises: (a) an extracellular domain (ED) of the NK-cell-activating receptor or a functional variant thereof, (b) a transmembrane domain (TMD) of the NK-cell-activating receptor or a functional variant thereof, and (c) an intracellular domain (ICD) of the NK-cell-activating receptor or a functional variant thereof; and wherein, optionally, a hinge or linker is included among the extracellular domain or the NK-cell-activating receptor or a functional variant thereof, the transmembrane domain of NK-cell-activating receptor or a functional variant thereof, and/or the intracellular domain of the NK-cell-activating receptor or a functional variant thereof.

Embodiment 22. The chimeric protein construct of Embodiment 21, wherein the NK-cell-activating receptor is selected from NKG2D, NKG2C, NKG2E, NKG2F, NKG2H, CD94, KIR2DL4, KIR2DS1, KIR2DS2, KIR2DS4, KIR3DS1, a natural cytotoxic receptor, TRAIL, DNAM-1, CD16a, 2B4, NTB-A, CRACC, orNKp80.

Embodiment 23. The chimeric protein construct of Embodiment 21 or 22, wherein the NK-cell-activating receptor is complexed with a CNK signal transduction module.

Embodiment 24. The chimeric protein construct of any one of Embodiments 18-23, wherein the at least one co-expression moiety is linked to the chimeric protein construct by a cleavable linker.

Embodiment 25. A nucleic acid molecule encoding the chimeric protein construct of any one of Embodiments 1-24.

Embodiment 26. The nucleic acid molecule of Embodiment 25, wherein the nucleic acid molecule is deoxyribonucleic acid (DNA), ribonucleic acid (RNA) (for example, mRNA, circular RNA, ccRNA), threose nucleic acid (TNA), glycol nucleic acid ( GNA), peptide nucleic acid (PNA), locked nucleic acid (LNA, including LNA with β-D-ribose configuration, α-LNA with α-L-ribose configuration (diastereoisomers of LNA), with 2'-amino-functionalized 2'-amino-LNA and 2'-amino-α-LNA with 2'-amino-functionalization), ethylene nucleic acid (ENA), cyclohexenyl nucleic acid (CeNA) and/or chimeras and/or combinations thereof.

Embodiment 27. A vector comprising the nucleic acid molecule of Embodiment 25 or 26, wherein the nucleic acid molecule is operably linked to at least one polynucleotide regulatory element to express a chimeric protein construct encoded by the nucleic acid molecule.

Embodiment 28. The vector of Embodiment 27, wherein the vector is selected from a group consisting of: plasmid, nanoplasmid, cosmid, viral vector (e.g., oncolytic viral vectors), minicircle, RNA vector, or linear or circular DNA (e.g., transposon DNA) or RNA molecules.

Embodiment 29. The vector of Embodiment 28, wherein the vector is a viral vector selected from a group consisting of retrovirus, lentiviral vector, adenovirus, parvoviruse (e.g., adeno-associated virus), adeno-associated virus (AAV) vector, coronavirus, negative-strand RNA virus such as orthomyxovirus (e.g., influenza virus), rhabdovirus (e.g., rabies and vesicular stomatitis viruses), paramyxovirus (e.g., Machi and Sendai), positive-strand RNA Virus such as picornavirus and alphavirus, and double-stranded DNA virus, wherein the double-stranded DNA virus, comprises adenovirus, herpesvirus (e.g., herpes simplex virus types I and II, Epstein-Barr virus, cytomegalovirus) and pox Virus (e.g., vaccinia, fowlpox, and canarypox), Norwalk, togavirus, flavivirus, reovirus, papovavirus, hepadnavirus, baculovirus, and hepatitis virus , Virus-like particle (VLP).

Embodiment 30. The vector of Embodiment 29, wherein the viral vector is a retroviral vector.

Embodiment 31. The vector of Embodiment 30, wherein the retroviral vector is selected from: avian leukoproliferative-sarcoma, mammalian C-type, B-type virus, D-type virus, HTLV-BLV collection, Lentivirus, bubble virus.

Embodiment 32. The vector of Embodiment 29, wherein the viral vector is a lentiviral vector.

Embodiment 33. The vector of Embodiment 32, wherein the lentiviral vector is selected from HIV-1, HIV-2, SIV, FIV, BIV, EIAV, CAEV or ovine demyelinating leukoencephalitis lentivirus.

Embodiment 34. The vector of any one of Embodiments 27-33, wherein the vector is further combined with other vectors comprising other nucleic acid molecules encoding at least one co-expression moiety.

Embodiment 35. An engineered cell expressing the chimeric protein construct of any one of Embodiments 1-25, or comprising the nucleic acid molecule of Embodiment 26, or comprising the vector of any one of Embodiments 27-33.

Embodiment 36. The engineered cell of Embodiment 35 expressing the chimeric protein construct of any one of Embodiments 1-18, and a co-expression moiety.

Embodiment 37. The engineered cell of Embodiment 35 or 36, wherein the cell is an immune cell selected from: T cell, natural killer (NK) cell, B cell, macrophage, monocyte, dendrites cell, neutrophil, or γδT cell.

Embodiment 38. The engineered cell of Embodiment 37, wherein the T cell is selected from the group consisting of: CD8+ T cell, CD4+ T cell, cytotoxic T cell, terminal effector T cell, memory T cell, naive T cell, cell group consisting of regulatory T cell, natural killer T cell, gamma-delta T cell, cytokine-induced killer (CIK) T cell, and tumor infiltrating lymphocyte.

Embodiment 39. A method for producing the engineered cell of any one of Embodiments 35-38, comprising introducing the vector of any one of Embodiments 27 to 34 into a starting cell, under a condition suitable for expressing the nucleic acid molecule of Embodiment 25 or 26.

Embodiment 40. The method of Embodiment 39, wherein the starting cell is a stem cell or a cell differentiated from a stem cell.

Embodiment 41. The method of Embodiment 38, wherein the stem cell is a hematopoietic progenitor cell (e.g. T cell progenitor, NK cell progenitor, macrophage progenitor), a hematopoietic stem cell (HSC), a CD34+ cell, an embryonic cell line, a mesenchymal stem cell or an iPSC cell.

Embodiment 42. The method of Embodiment 37, wherein the cell is an immune cell.

Embodiment 43. The method of Embodiment 40, wherein the immune cell is a T cell, a natural killer (NK) cell, a B cell, a macrophage, a monocyte, or a dendritic cell.

Embodiment 44. The method of Embodiment 41, wherein the cell is a T cell selected from the group consisting of: CD4+ T cell, CD8+ T cell, cytotoxic T cell, terminal effector T cell, memory T cell, naive T cell, cell group consisting of regulatory T cell, natural killer T cell, gamma-delta T cell, cytokine-induced killer (CIK) T cell, and tumor infiltrating lymphocyte.

Embodiment 45. A cell population produced ex vivo by the method of any one of Embodiments 39-44.

Embodiment 46. A pharmaceutical composition or kit, comprising: (i) the chimeric protein construct of any one of Embodiments 1-24, or the nucleic acid molecule of Embodiment 25 or 26, or the vector of any one of Embodiments 27-34, or the engineered cell of any one of Embodiments 35-38 or the cell population of Embodiment 45, and (ii) pharmaceutically acceptable medium.

Embodiment 47. A method for degrading a target protein, comprising delivering the vector of any one of Embodiments 27-34 into a cell expressing the target protein.

Embodiment 48. The method of Embodiment 47, wherein the cell is selected from: a tumor cell, a virus-infected cell, a neural cell, a transplanted cell, or an immune cell.

Embodiment 49. The method of Embodiment 47 or 48, wherein the delivery comprises delivery by a viral vector (e.g., an oncolytic virus).

Embodiment 50. The method of Embodiment 49, wherein the vector expresses the chimeric protein construct of any one of Embodiments 1-24 in the cell.

Embodiment 51. The method of Embodiment 50, wherein the chimeric protein construct is capable of simultaneously binding a target protein and an ERAD mechanism protein, thereby degrading the target protein.

Embodiment 52. A method of treating a condition or disease in a subject in need thereof, comprising:
administering a therapeutically effective amount of the pharmaceutical composition of Embodiment 46 to the subject.

Embodiment 53. The method of Embodiment 52, wherein the disease comprises various solid tumors and hematological tumors, viral infectious diseases, autoimmune diseases, and neurological and degenerative diseases (for example, Alzheimer's disease), metabolic diseases (e.g., diabetes (e.g., type II diabete), lipid metabolism-related diseases (e.g., tumor lipid metabolism, non-alcoholic fatty liver), atherosclerosis (AS), tumor glucose metabolism);
preferably, the solid tumor is selected from nervous system tumors, head and neck tumors, chest tumors, digestive system tumors, genitourinary system tumors, soft tissue and skin tumors, bone tumors, etc.;
preferably, the nervous system tumors include diffuse glioma, diffuse astrocytoma and anaplastic astrocytoma, glioblastoma, oligodendroglioma, oligoastrocytoma, diffuse Gliomas, other astrocytomas, ependymomas, neuronal and mixed neuronal-glial tumors, medulloblastoma, other embryonal tumors, schwannomas, meningiomas, solitary fibrous tumors and hemangiopericytoma, etc.;
preferably, the head and neck tumors comprise malignant tumors of the nasal luminal and sinuses, nasopharyngeal cancer, oral cancer, laryngeal cancer, salivary gland tumors, intracranial tumors, thyroid cancer, tongue cancer, etc.;
preferably, the thoracic tumors comprise lung cancer, esophageal cancer, cardia cancer, breast cancer, mediastinal tumors, etc.;
preferably, the digestive system tumors comprise gastric cancer, colorectal cancer, sigmoid colon and rectal cancer, liver cancer, pancreatic cancer and periampullary cancer, biliary tract cancer, malignant tumors of the small intestine, etc.;
preferably, the genitourinary system tumors comprise kidney cancer, prostate cancer, bladder cancer, testicular cancer, penile cancer, cervical cancer, endometrial cancer, ovarian cancer, etc.;
preferably, the soft tissue and skin tumors comprise malignant fibrous histiocytoma, rhabdomyosarcoma, synovial sarcoma, malignant melanoma of the skin, etc.;
preferably, the bone tumors comprise osteosarcoma, Ewing's sarcoma, etc.;
preferably, the colon cancer is colon adenoma;
preferably, the breast cancer is a triple-negative breast cancer cell;
preferably, the liver cancer is hepatocellular carcinoma;
preferably, the disease is a hematological tumor selected from leukemia, lymphoma (HL), multiple myeloma (MM), myelodysplastic syndrome (MDS), etc.;
preferably, the leukemia is B-cell acute lymphoblastic leukemia, T-cell acute lymphoblastic leukemia, acute myeloid leukemia, etc.;
preferably, viral infectious diseases comprise: respiratory viral diseases, gastrointestinal viral diseases, liver viral diseases, skin and mucous membrane viral diseases, ocular viral diseases, central nervous system viral diseases, lymphocytic viral diseases, Insect-borne viral diseases, lentivirus infection diseases, etc.;
preferably, the respiratory viral diseases comprise infections of rhinovirus, adenovirus, respiratory syncytial virus, parainfluenza virus, and coronavirus; influenza; mumps, etc.;
preferably, the gastrointestinal viral diseases comprise polio; Cooksackie virus infection; ECHO virus infection; and viral gastroenteritis, including rotavirus gastroenteritis, Norwalk virus gastroenteritis, adenovirus gastroenteritis, astrovirus gastroenteritis, coronavirus gastroenteritis and calicivirus gastroenteritis, etc.;
preferably, the liver viral diseases comprise viral hepatitis A, viral hepatitis B, viral hepatitis C, viral hepatitis D, viral hepatitis E, Epstein-Barr viral hepatitis, and cytomegalovirus hepatitis etc.;
preferably, the skin and mucous membrane viral diseases comprise measles, rubella, infantile acute rash, chickenpox and herpes zoster, smallpox, herpes simplex virus infection, rabies and foot-and-mouth disease, etc.;
preferably, the ocular viral diseases comprise epidemic keratoconjunctivitis, follicular conjunctivitis and herpetic keratoconjunctivitis, etc.;
preferably, the central nervous system viral diseases comprise Japanese encephalitis, western equine encephalitis, eastern equine encephalitis, St. Louis encephalitis, Venezuelan equine encephalitis, Murray Valley encephalitis, Californian encephalitis, forest encephalitis and lymphocytic choroid plexus meningitis, etc.;
preferably, the lymphocytic viral diseases comprise infectious mononucleosis, cytomegalovirus infection and acquired immunodeficiency syndrome, etc.;
preferably, the insect-borne viral diseases comprise: viral hemorrhagic fevers, including epidemic hemorrhagic fever, yellow fever, Crimean-Congo hemorrhagic fever, Rift Valley fever, Argentine hemorrhagic fever, Bolivian hemorrhagic fever, Lassa fever, Omu Scrubs hemorrhagic fever, Marburg disease and Ebola hemorrhagic fever, etc.; dengue fever and dengue hemorrhagic fever; West Nile fever; Colorado tick heat transfer; and sandfly fever, etc.;
preferably, the lentiviral infection diseases comprise subacute sclerosing panencephalitis, Kuru disease, progressive multifocal leukoencephalopathy and subacute spongiform encephalopathy (corticostriatal spinal cord degeneration), etc.;
preferably, the autoimmune diseases comprise organ-specific autoimmune diseases and systemic autoimmune diseases;
preferably, the organ-specific autoimmune diseases comprise chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritic syndrome, vulgaris Pemphigus, pemphigoid, primary biliary cirrhosis, multiple sclerosis, acute idiopathic polyneuritis, etc.;
preferably, the systemic autoimmune diseases comprise systemic lupus erythematosus, rheumatoid arthritis, systemic vasculitis, scleroderma, pemphigus, dermatomyositis, mixed connective tissue disease, autoimmune hemolytic anemia, thyroid Autoimmune diseases, ulcerative colitis, etc.;
preferably, neurological diseases comprise nervous system diseases, peripheral nerve diseases such as trigeminal neuralgia, facial paralysis, hemifacial spasm, vestibular neuronitis, glossopharyngeal neuralgia, mononerve disease, brachial plexus neuralgia, multiple mononeuropathy, multiple Neuropathy, acute inflammatory demyelinating polyneuropathy, chronic inflammatory demyelinating polyneuropathy;
spinal cord diseases, e.g., myelitis, compressive myelopathy, subacute combined degeneration of the spinal cord, syringomyelia, spinal cord vascular disease, spinal cord arachnoiditis, etc.;
cerebrovascular diseases, e.g., transient ischemic attack, cerebral infarction, cerebral hemorrhage, subarachnoid hemorrhage, intracranial venous system thrombosis, etc.;
infectious diseases of the central nervous system, e.g., meningitis, encephalitis, etc. caused by infectionsof viruses, bacteria, fungi, or parasites, etc, and lentiviral encephalitis caused by lentiviral infections;
central nervous system demyelinating diseases, e.g., multiple sclerosis, neuromyelitis optica, acute disseminated encephalomyelitis, leukodystrophy, etc.;
movement disorders, e.g., Parkinson's disease, chorea, hepatolenticular degeneration, dystonia, essential tremor, tardive dyskinesia, etc.;
epilepsy;
headaches, e.g., migraines, tension headaches, cluster headaches, etc.;
nervous system degenerative diseases, e.g., motor neuron disease, Alzheimer's disease, Lewy body dementia, frontotemporal dementia, multiple system atrophy, etc.;
inherited nervous system diseases such as hereditary ataxia, hereditary spastic paraplegia, Charcot-Marie-Tooth disease, neurofibromatosis, tuberous sclerosis, cerebral hemangiomatosis, etc.;
neurodevelopmental disorders such as congenital hydrocephalus, cerebral palsy, basilar depression, cerebellar subtonsillar disease, etc.;
neuromuscular junction and muscle diseases, e.g., myasthenia gravis, periodic paralysis, polymyositis, progressive muscular dystrophy, myotonic myopathy (myotonic dystrophy, myotonia congenita), metabolic Myopathy (mitochondrial myopathy, lipid storage myopathy, glycogenosis), etc.;
autonomic nervous system diseases, e.g., Raynaud's disease, erythromelalgia, hemifacial atrophy, generalized autonomic insufficiency, spontaneous hyperhidrosis, progressive lipodystrophy, etc.;
nervous system tumors, e.g., glioma, lymphoma, meningioma, etc.;
paraneoplastic: paraneoplastic syndromes of the nervous system, e.g., paraneoplastic cerebellar degeneration, paraneoplastic encephalomyelitis, subacute necrotizing myelopathy, subacute motor neuron disease, paraneoplastic sensory neuron disease, etc.;
preferably, metabolic diseases comprise diabetes, hyperlipidemia, gout, etc.

Embodiment 54. A method of stimulating an immune response in a subject, said method comprises administering an effective amount of the pharmaceutical composition of embodiment 46 to the subject.

### BRIEF DESCRIPTION OF THE DRAWINGS

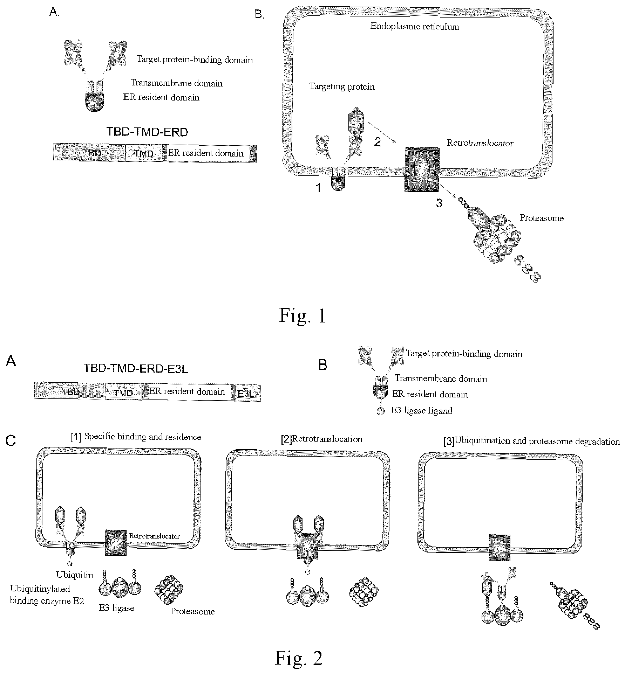

Fig. 1 illustrates a chimeric protein construct based on endoplasmic reticulum-based targeted protein degradation technologies, wherein Fig. 1A shows a linear schematic diagram and a structural schematic diagram of the basic ER-TPD chimeric protein construct TBD-TMD-ERD, and Fig. 1B shows the working structural principle of the ER-TPD chimeric protein construct TBD-TMD-ERD.
Fig. 2 illustrates an ER-TPD chimeric protein construct TBD-TMD-ERD-E3L with E3 ubiquitin ligase ligand domain, wherein, Fig. 2A shows its linear schematic diagram,
Fig. 2B shows its structural schematic diagram, and Fig. 2C shows the working structural principle of the ER-TPD chimeric protein construct TBD-TMD-ERD-E3L.

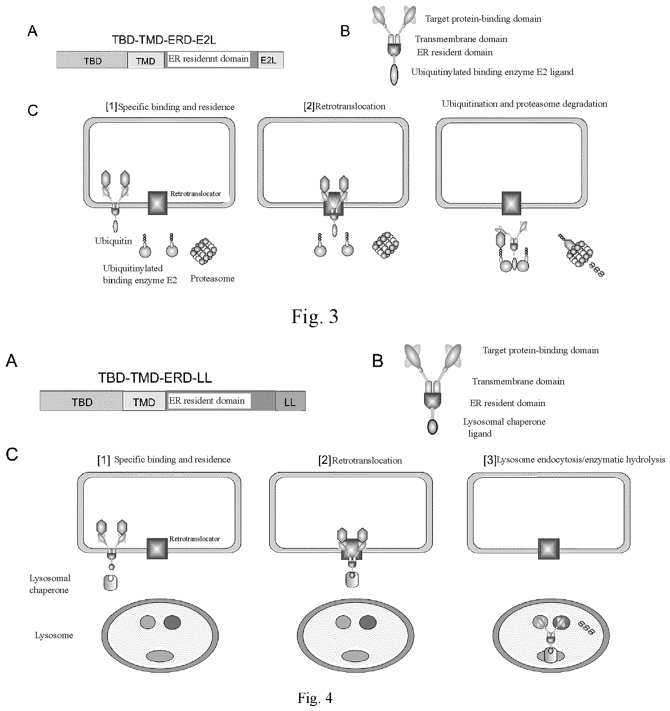

Fig. 3 illustrates an ER-TPD chimeric protein construct TBD-TMD-ERD-E2L with E2 ubiquitin-binding enzyme ligand domain, wherein, Fig. 3A shows its linear schematic diagram, Fig. 3B shows its structural schematic diagram, and Fig. 3C shows the working structural principle of the ER-TPD chimeric protein construct TBD-TMD-ERD-E2L.
Fig. 4 illustrates an ER-TPD chimeric protein construct TBD-TMD-ERD-LL with lysosome ligand domain, wherein, Fig. 4A shows its linear schematic diagram, Fig. 4B shows its structural schematic diagram, and Fig. 4C shows the working structural principle of the ER-TPD chimeric protein construct TBD-TMD-ERD-LL.

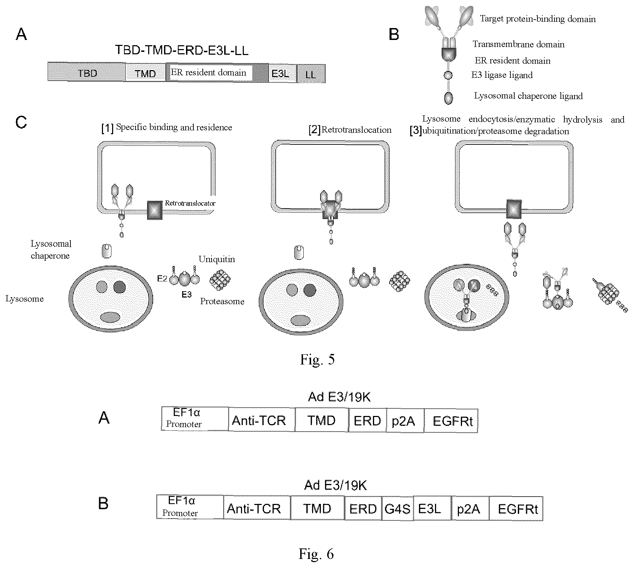

Fig. 5 illustrates an ER-TPD chimeric protein construct TBD-TMD-ERD-E3L-LL with combination of E3 ubiquitinated ligase ligand and lysosome ligand, wherein, Fig. 5A shows its linear schematic diagram, Fig. 5B shows its structural schematic diagram, and Fig. 5C shows the working structural principle of the ER-TPD chimeric protein construct TBD-TMD-ERD-E3L-LL.
Fig. 6 illustrates an ER-TPD chimeric nucleic acid construct (TCR-ER-TPD) targeting TCR, wherein, Fig. 6A shows the chimeric nucleic acid construct TCR-TPD1, and
Fig. 6B shows the chimeric nucleic acid construct TCR -TPD2.

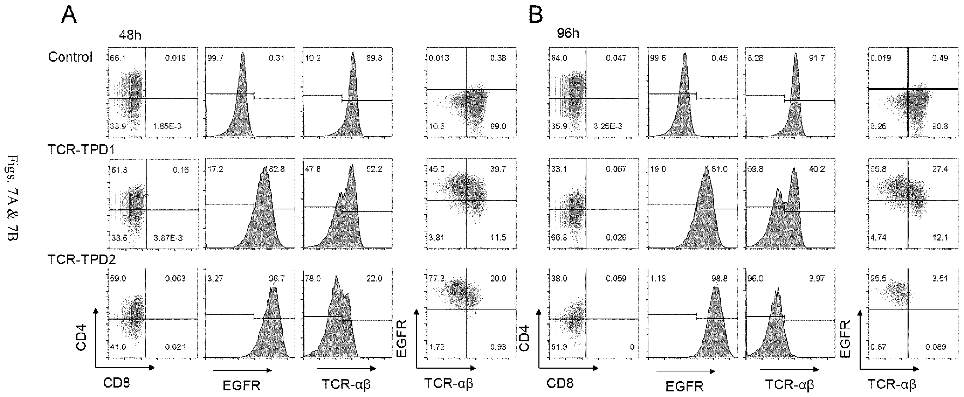

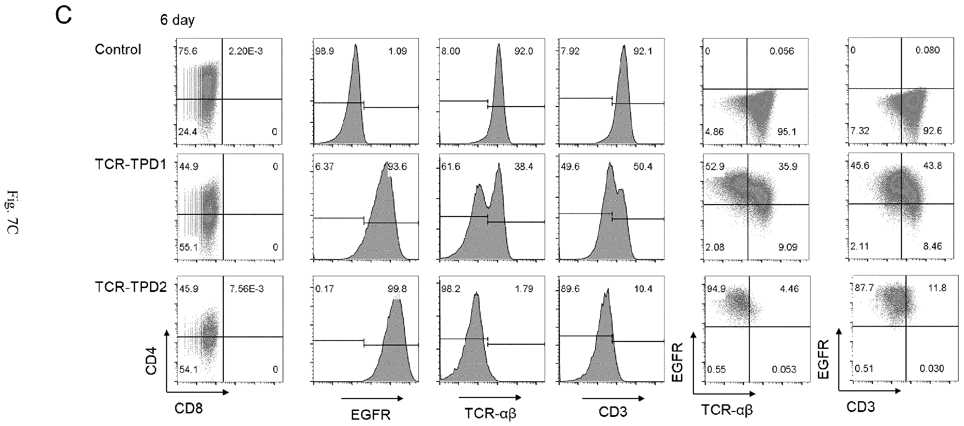

Fig. 7 illustrates results of cell phenotype detection of TCR ER-TPD Jurkat cells at 48h (A), 96h (B) and 6th day (C).

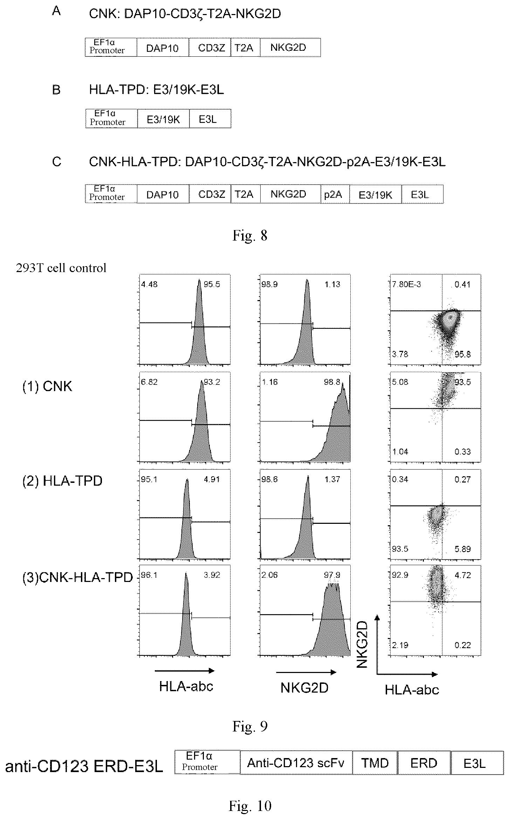

Fig. 8 illustrates an expression vector designed using a single lentiviral expression vector and the EF1α promoter as a promoter, wherein, Fig. 8A shows the CNK expression vector, Fig. 8B shows the chimeric protein construct HLA-ER- TPD, and Fig. 8C shows the chimeric protein construct CNK-HLA-ER-TPD.
Fig. 9 illustrates 48h flow cytometry results of cell phenotype of the 293 cells transfected with the ER-TPD chimeric nucleic acid construct targeting HLA-abc combined with the CNK expression vector.
Fig. 10 illustrates an ER-TPD chimeric nucleic acid construct targeting CD123, wherein Fig. 10A shows the CNK expression vector, and Fig. 10B shows the chimeric protein construct HLA-TPD.

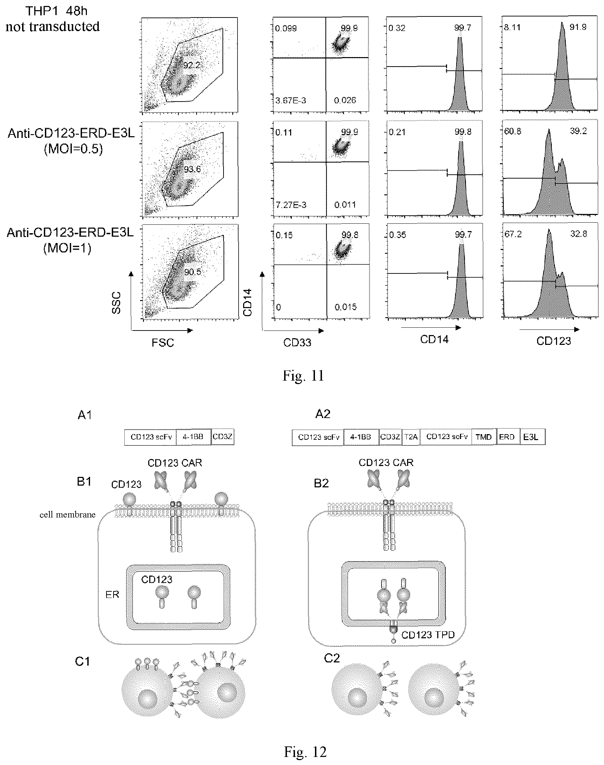

Fig. 11 illustrates 48h flow cytometry results of cell phenotype of the acute myeloid leukemia cell line THP1 transfected with scFv TPD chimeric nucleic acid construct targeting CD123.
Fig. 12 illustrates application of ER-TPD-CD123 CAR-T cells targeting CD123, wherein Fig. 12A1 shows CD123 CAR, Fig. 12B1 shows the expression of CD123 on activated T cells, and Fig. 12C1 shows CAR-T cells Targeting CD123 may produce a self-killing effect in the process of activation, culture and killing tumor cells, Fig. 12A2 shows the CD123 CAR-T-T2A-CD123 ER-TPD chimeric protein construct, and Fig. 12B2 shows the expression of the chimeric protein construct may degrade the self-expressed CD123, and Fig. 12C2 shows the killing effect on tumor cells after expressing the chimeric protein construct.

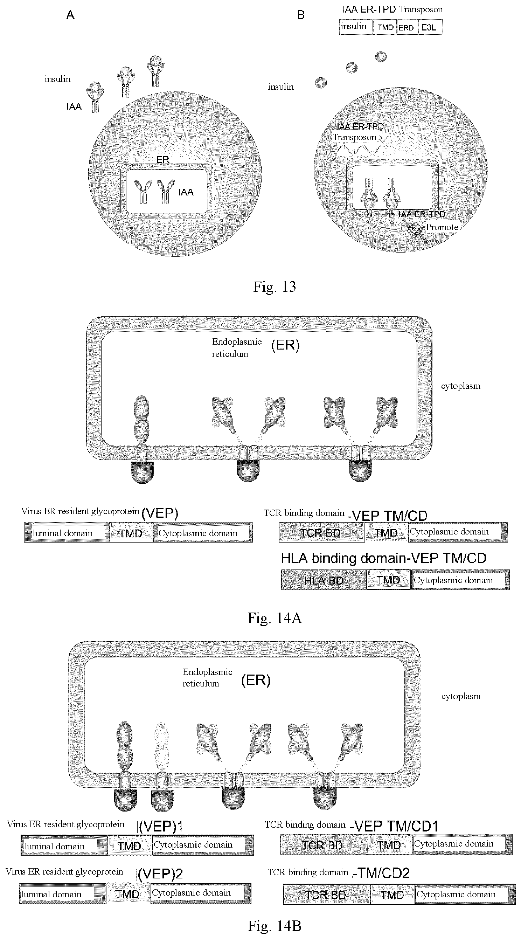

Fig. 13 illustrates the application of the ER-TPD chimeric protein construct targeting autoantibodies in the treatment of autoimmune diseases, wherein, Fig. 13A shows that plasma cells in patients with type 1 diabetes express and release insulin antibodies, which bind to Insulin, and clear insulin in the peripheral blood, resulting in a severe decrease in insulin usage in patients; Fig. 13B shows the delivery of ER-TPD transposon DNA targeting insulin antibodies to plasma cells using liposomes targeting plasma cells intracellular, which achieves integration and long-term expression of the IAA ER-TPD chimeric protein construct.

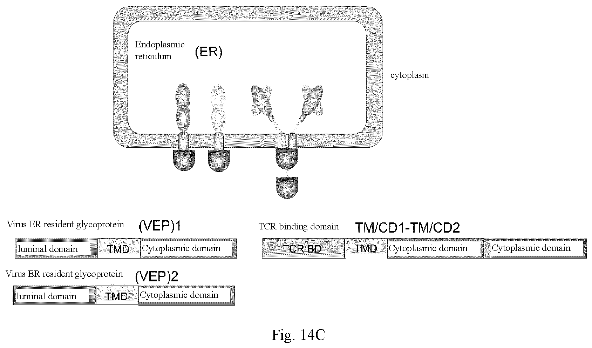

Fig. 14 illustrates exemplary chimeric fusion proteins containing one or more transmembrane and cytoplasmic domains of TCRαβ or HLA molecule binding molecules and viral ER-resident proteins. Fig. 14A shows a schematic diagram and composition of UT chimeric multi-domain. Broad structure of a viral ER-resident glycoprotein (VEP) is consisted of a luminal domain (LD), a transmembrane domain (TMD), and a cytoplasmic functional domain; a UT chimeric multidomain is consisted of a TCR or HLA-binding domain, a hinge / spacer, VEP transmembrane and cytoplasmic domains. Fig. 14B shows a schematic diagram and composition of UT chimeric multi-domains. Broad structure of different viral ER-resident glycoproteins (VEPs), is consisted of distinct luminal domains (LD), transmembrane domains (TMD), and cytoplasmic functional domains; a UT chimeric multidomain is consisted of TCR or HLA domain, a hinge/spacer, which is different from VEP transmembrane and cytoplasmic domains. Fig. 14C shows a schematic diagram and composition of UT chimeric multidomain. Broad structure of different viral ER-resident glycoproteins (VEPs) is consisted of distinct luminal domains (LDs), transmembrane domains (TMDs), and cytoplasmic functional domains; a UT chimeric multidomains is consisted of a TCR or HLA-binding domain, a Hinge/spacer, wherein different VEP transmembrane and chimeric cytoplasmic domains are derived from different VEPs.

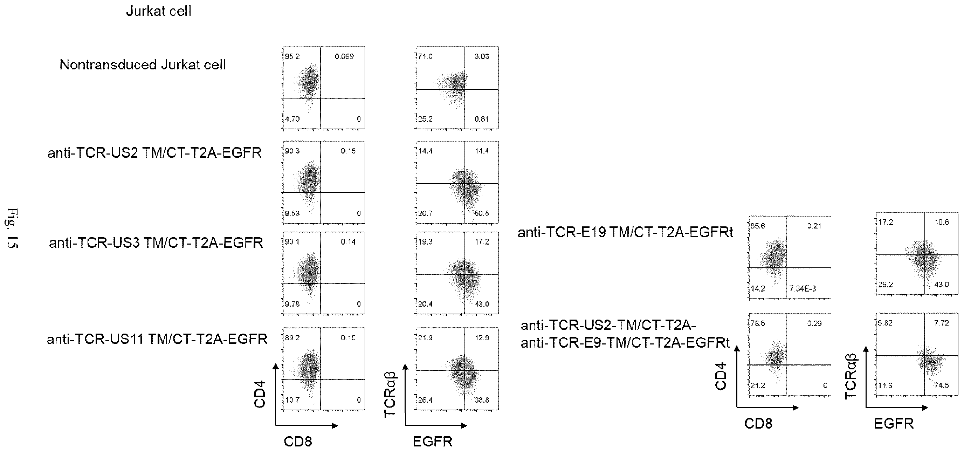

Fig. 15 illustrates that the forced expression of UT elements may effectively inhibit the expression of TCR in Jurkat cells. A lentiviral vector with truncated EGFR was constructed, encoding an anti-TCR scFv fused to different viral ER resident proteins, HCMV US2, US3, US11, adenovirus E19 TM/CT domains, and simultaneously encoding anti-Lentiviral vector of TCR scFv fused to US2 and adenovirus E19 TM/CT. Jurkat cells were transduced with this lentivirus, and non-transduced Jurkat cells were used as a control. Forty-eight hours after transduction, the cells were sent to a flow cytometer to detect the expression of TCRαβ in the transduced cells (EGFR+ cells). The results showed that all EGFR(+) Jurkats showed a significant downregulation of TCRαβ expression, and cells transduced with combined UT components (US2/E19) could effectively block the expression of TCRαβ. All tested UT chimeric elements (US2, US3, US11, E19) were able to block the expression of TCRαβ. Combining UT elements (US2 and E19) may significantly enhance the inhibitory effect on the expression of TCRαβ on the surface of Jurkat cells.

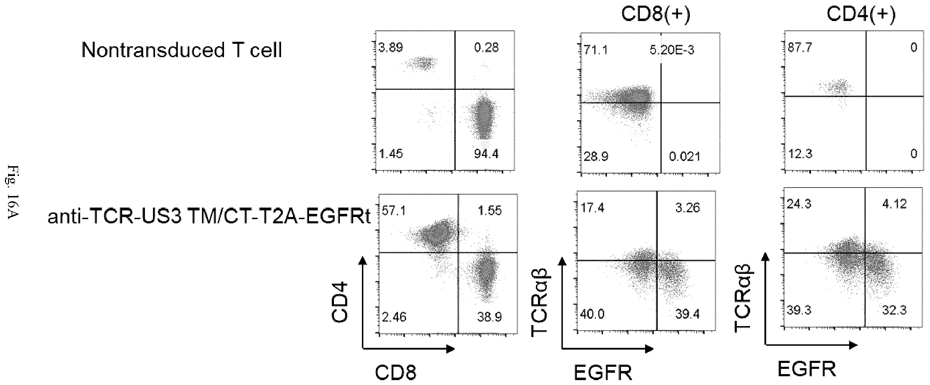

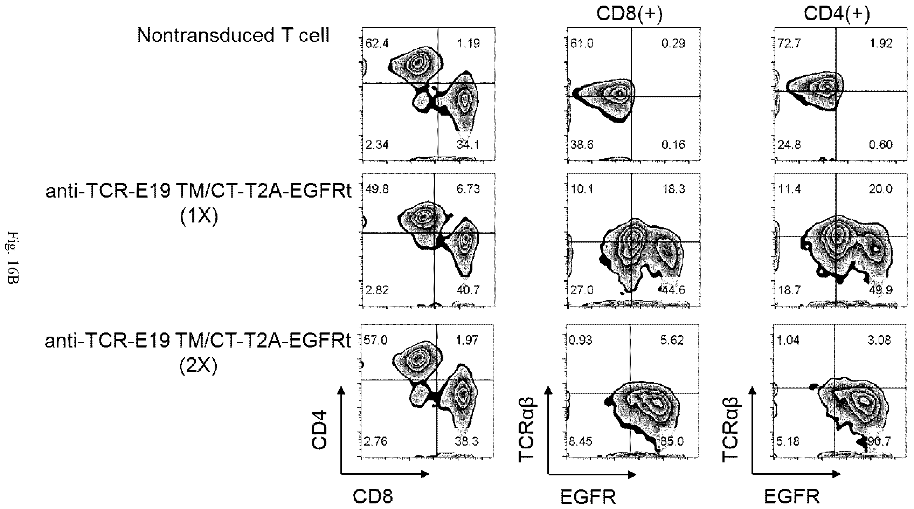

Fig. 16 illustrates that forced expression of UT elements may effectively inhibit TCR expression in human T cells. Fig. 16A shows that the forced expression of UT elements may effectively inhibit the expression of TCR in human T cells. The lentiviral vector with the expression cassette including the truncated EGFR encodes an anti-TCR scFv fused with the HCMV US3 TM/CT domain (anti-TCR-US2 TM/CT-T2A-EGFRt). Human T cells were stimulated and transduced with this lentivirus, and untransduced T cells were used as controls. After 5 days of transduction, the cells were sent to a flow cytometer to detect the expression of TCRαβ in the transduced T cells (EGFR+ cells). The results showed that EGFR(+) T cells showed a significant downregulation of TCRαβ expression compared with non-transduced T cells. This indicates that the US element containing anti-TCR-US3 TM/CT may effectively inhibit the expression of TCRαβ in human T cells. Fig. 16B shows that forced expression of UT elements can effectively inhibit TCR expression in human T cells. The lentiviral vector with the expression cassette including the truncated EGFR encoded an anti-TCR scFv fused with the HCMV E19 TM/CT domain (anti-TCR-E19 TM/CT-T2A-EGFRt). Human T cells were stimulated and transduced with this lentivirus, and non-transduced T cells were used as controls. After 5 days of transduction, the cells were sent to a flow cytometer to detect the expression of TCRαβ in the transduced T cells (EGFR+ cells). The results showed that EGFR(+) T cells showed a significant down-regulation of TCRαβ expression compared with non-transduced T cells. Increased viral transduction may further enhance the inhibition of TCRαβ expression on the T cell surface. This indicates that the US element containing anti-TCR-E19 TM/CT may efficiently express TCRαβ in human T cells.

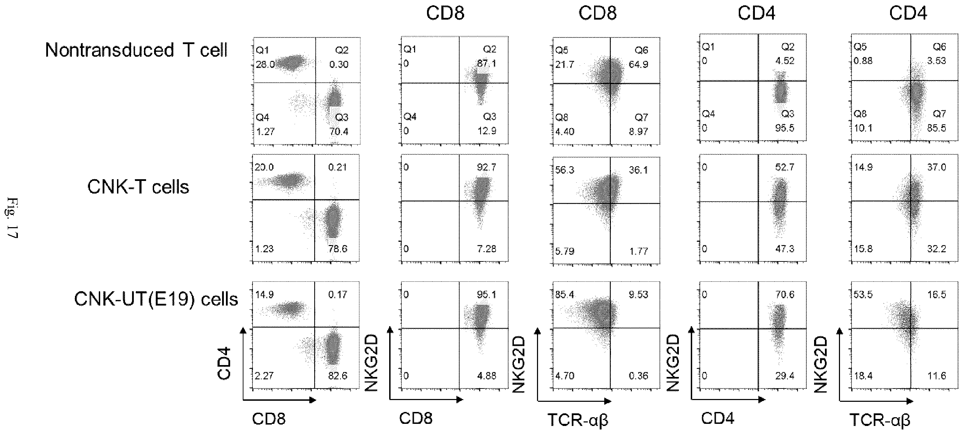

Fig. 17 illustrates that CNK-T cells with UT elements down regulate TCR expression on CD8/CD4 cells. The lentiviral vector contains an expression cassette (CNK-UT-E19) encoding a chimeric NK receptor and an anti-TCR scFv fused with an E19 TM/CT domain. Human T cells were transduced with this lentivirus. After five days of transduction, the cells were sent to flow cytometry to detect the expression of TCRαβ in transduced cells (NKG2D+ cells), conventional chimeric NK receptor-transduced T cells, and non-transduced T cells. The results illustrate that the introduction of UT elements in the CNK expression cassette does not affect the expression of chimeric NK receptors. In addition, it may significantly inhibit the expression of TCR on CD8+ and CD4+ CNK-T cells.

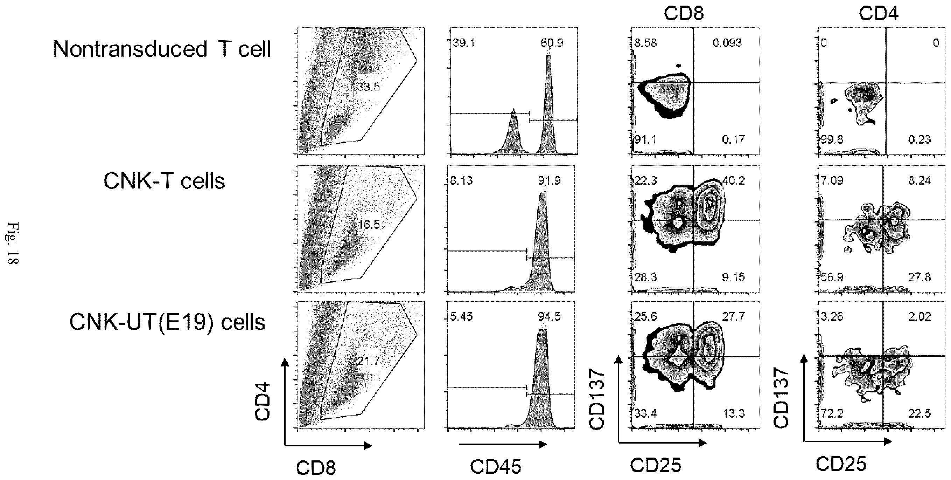

Fig. 18 illustrates that CNK-T cells with UT elements, similar to normal CNK-T cells, exhibit strong cytotoxicity against tumor cells. Non-transgenic T cells, CNK-T cells and CNK-UT (E19) cells were co-cultured with HepG2 cells at an E: T ratio of 1:5. After culturing for 24 hours, cells were harvested and analyzed by flow cytometry to detect tumor lysis and T cell activation. The data indicates that CNK-UT cells exhibited comparable potent cytotoxicity to HepG2 cells as normal CNK-T cells. After co-culturing for 24 hours, CD45(-) tumor cells were significantly reduced in both CNK-T and CNK-UT co-cultures. In addition, both CD8 and CD4 CNK-UT cells could up-regulate CD25 and CD137 after co-cultured with HepG2 cells.

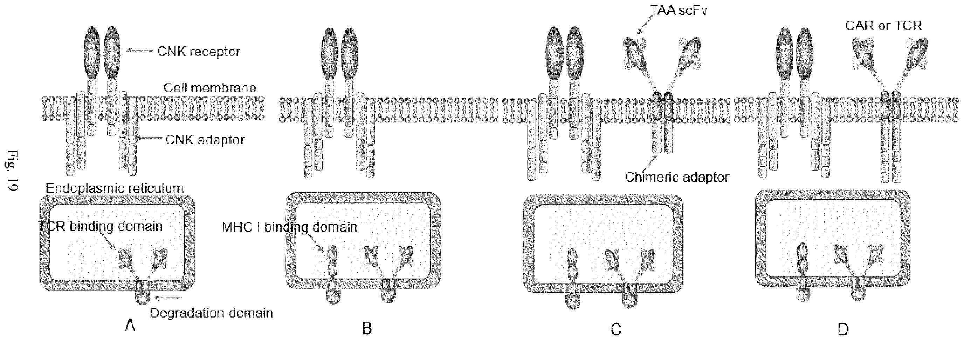

Fig. 19 illustrates the CNK-UT multi-functional complex of four structures, wherein, Fig. 19A shows a basic CNK-UT multi-functional complex, Fig. 19B shows a CNK-UT multifunctional complex with a MHC I target binding protein molecular domain, Fig. 19C shows a CNK-UT multifunctional complex with a MHC I target binding protein molecular domain and a chimeric adapter, Fig. 19D shows a CNK-UT multifunctional complex with a MHC I target binding protein molecular domain and a receptor, e.g., CAR or TCR or etc., targeting and killing a tumor cell.

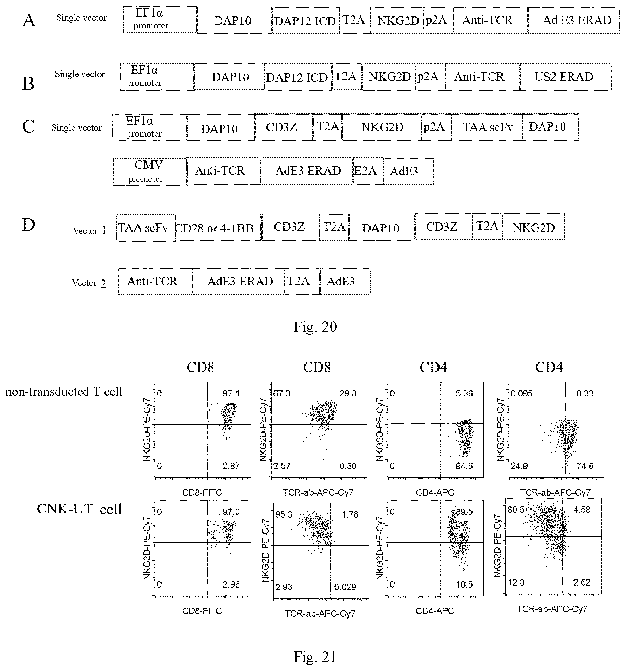

Fig. 20 illustrates the structures of four CNK-UT elements expressing CNK-UT multifunctional complexes. As shown in Fig. 20A, a single lentiviral EF1α promoter expression vector promotes the expression of one of the combinations of CNK-UT elements : DAP10-DAP12 ICD-T2A-NKG2D-p2A-anti-TCR-AdE3 ERAD; as shown in Fig. 20B, a single lentiviral EF1α promoter expression vector is used to promote the expression of one of the combinations of CNK-UT elements: DAP10-DAP12 ICD -T2A-NKG2D-p2A-anti-TCR-US2 ERAD; as shown in Fig. 20C, a single lentiviral expression vector, and EF1α and CMV promoters, are used to regulate the multi-gene expressions of DAP10-CD3ζ-T2A-NKG2D-p2A-ant-TAA scFv-DAP10, and anti-TCR-AdE3 ERAD-E2A-AdE3, respectively, realizing the expression of multiple CNK-UT functional elements on a single vector; as shown in Fig. 20D, two different lentiviral expression vectors are used to regulate expressions of anti-TAA scFv-CD28/4-1BB-CD3ζ-T2A-CD3ζ-p2A-NKG2D and anti-TCR-AdE3 ERAD-T2A-AdE3, respectively, realizing the expressions of multiple function elements for the CNK-UT on one same T cell after co-transfection with T cells.
Fig. 21 illustrates the results of flow cytometry detection of a basic phenotype of CNK-UT (DAP10-CD3ζ-T2A-NKG2D-p2A-anti-TCR scFv-AdE3) cells.

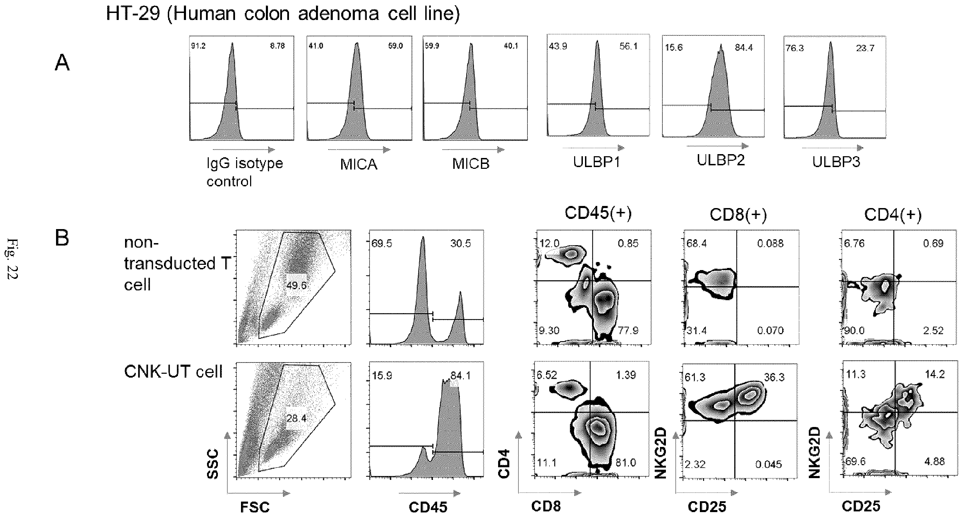

Fig. 22 illustrates the results of recognition and specific killing of CNK-UT cells on the human colon adenoma cell line HT29, wherein Fig. 22A shows the expression of different NK target proteins in the human colon adenoma cell line HT29, and Fig. 22B shows that CNK-UT cells have efficient killing and activating functions on HT29.

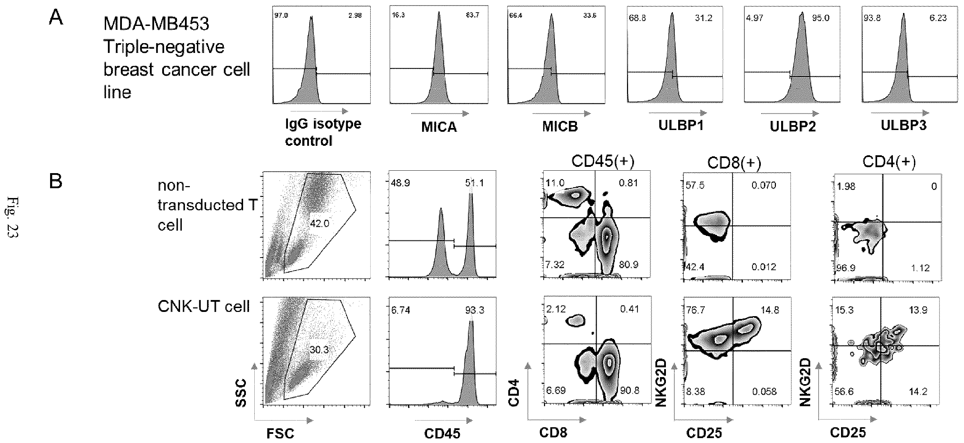

Fig. 23 illustrates the recognition and specific killing of MDA-MB453 by CNK-UT cells, wherein Fig. 23A shows the expression of different NK target proteins in the triple negative breast cancer cell line MDA-MB453, and Fig. 23B shows that CNK-UT cells have efficient killing and activating functions on THP1.

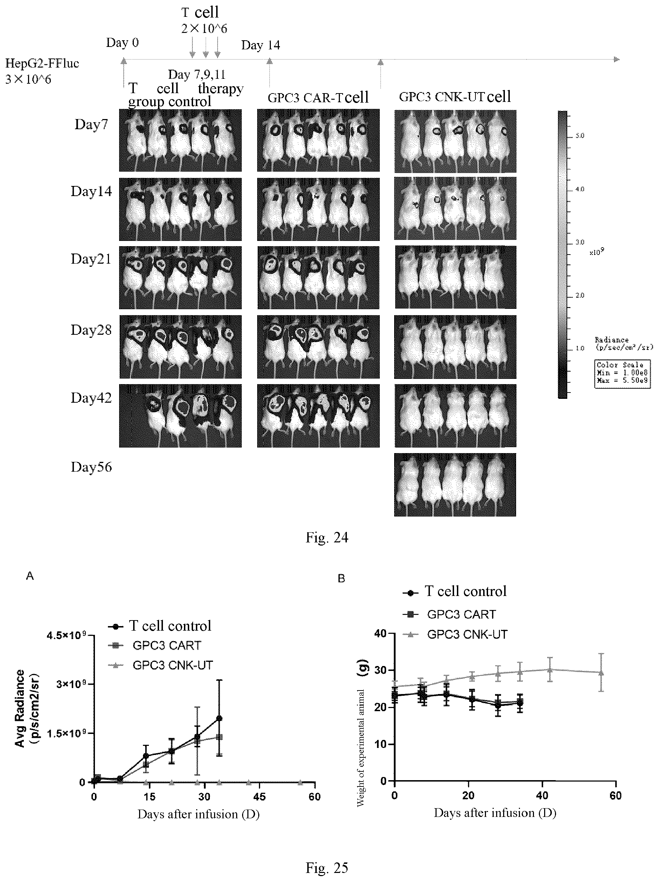

Fig. 24 illustrates that GPC3 CAR/CNK-UT cells have more efficient tumor clearance ability in mice than GPC3 CAR-T cells.
Fig. 25 illustrates that GPC3 CAR/CNK-UT cells have more efficient tumor clearance ability than GPC3 CAR-T.

### DETAILED DESCRIPTION

Before the present disclosure is described in greater detail, it is to be understood that this disclosure is not limited to particular embodiments described, and as such may, of course, vary. It is also to be understood that the terminology used herein is for the purpose of describing particular embodiments only, and is not intended to be limiting, since the scope of the present disclosure will be limited only by the appended claims.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present disclosure is not entitled to antedate such publication by virtue of prior disclosure. Further, the dates of publication provided could be different from the actual publication dates that may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope or spirit of the present disclosure. Any recited method can be carried out in the order of events recited or in any other order that is logically possible.

### I. Definition

The following definitions are provided to assist a reader. Unless otherwise defined, all terms of art, notations and other scientific or medical terms or terminology used herein are intended to have the meanings commonly understood by those of skill in the art. In some cases, terms with commonly understood meanings are defined herein for clarity and/or for ready reference, and the inclusion of such definitions herein should not necessarily be construed to represent a substantial difference over the definition of the term as generally understood in the art.

It is noted that in the present disclosure, terms such as "comprises", "comprised", "comprising", "contains", "containing" and the like have the meaning attributed in United States Patent law; they are inclusive or open-ended and do not exclude additional, un-recited elements or method steps. Terms such as "consisting essentially of" and "consists essentially of" have the meaning attributed in United States Patent law; they allow for the inclusion of additional ingredients or steps that do not substantially affect the basic and novel characteristics of the claimed invention. The terms "consists of" and "consisting of" have the meaning ascribed to them in United States Patent law; namely that these terms are close ended.

As used herein, the singular forms "a", "an" and "the" include plural references unless the context clearly dictates otherwise.

As used herein, the term "about" refers to a measurable value such as an amount, a time duration, and the like, and encompasses variations of ±20%, ±10%, ±5%, ±1%, ±0.5% or ±0.1% from the specified value.

"Antigen" refers to a molecule capable of being specifically recognized and bounded by an antibody. In some embodiments, the antigen(s) described herein may be a molecule provoking an immune response. This immune response may be either humoral, or cell-mediated response, or both. The skilled artisan will understand that any macromolecule, including virtually all proteins or peptides, can serve as an antigen. It is readily apparent that the present disclosure includes therapeutic antibodies acting as antigen eliciting immune response.

"Antibody" refers to a polypeptide of the immunoglobulin (Ig) family that binds with an antigen. For example, a naturally occurring "antibody" of the IgG type is a tetramer comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain (abbreviated herein as CL). The VH and VL regions can be further subdivided into regions of hypervariability, termed complementarity determining regions (CDR) (a light chain CDR includes LCDR1, LCDR2, and LCDR3, and a heavy chain CDR includes HCDR1, HCDR2, and HCDR3), interspersed with regions that are more conserved, termed framework regions (FR). Boundaries defining CDRs of the antibodies disclosed herein may be defined or identified according to the conventions of Kabat, IMGT, Chothia, or Al-Lazikani (Al-Lazikani, B., Chothia, C., Lesk, A. M., J. Mol. Biol.), 273(4), 927(1997); Chothia, C. et al., Journal of Molecular Biology, Dec. 5;186(3):651-63(1985); Chothia, C. and Lesk, A.M., Journal of Molecular Biology, 196, 901 (1987); Chothia, C. et al., Nature, Dec. 21-28;342(6252):877-83 (1989); Kabat E.A. et al., National Institutes of Health, Bethesda, Md. (1991); Marie-Paule Lefranc et al., Developmental and Comparative Immunology, 27: 55- 77 (2003); Marie-Paule Lefranc et al., Immunome Research, 1(3), (2005); Marie-Paule Lefranc, Molecular Biology of B cells (Second Edition), Chapter 26, 481-514, (2015)). VH and VL are composed of three CDRs and four FRs, respectively, arranged in the following order from the amino terminus to the carboxyl terminus: FR1, CDR1, FR2, CDR2, FR3, CDR3, and FR4. The variable regions of the heavy and light chains contain binding domains that interact with an antigen.

As used herein, a "functional fragment" of an antibody is used interchangeably with "antigen-binding fragment" and refers to a fragment of an antibody that includes one or more CDRs, or an antibody fragment formed by any other antibody portion that binds to an antigen rather than including a complete native antibody structure. Examples of antigen binding fragments include, but are not limited to, diabodies, Fab, Fab', F(ab')2, Fd, Fv fragments, disulfide bond stabilized Fv fragments (dsFv), (dsFv)2, bispecific dsFv (dsFv-dsFv'), disulfide bond stabilized diabodies (ds diabodies), single chain antibody molecules (scFv), scFv dimers (bivalent diabodies), multispecific antibodies, camelized single domain antibodies, nanobodies, domain antibodies, and bivalent domain antibodies. Antigen-binding fragments are capable of binding the same antigen to which the parent antibody binds. In certain embodiments, an antigen-binding fragment may include one or more CDRs from a particular parent antibody.

"Fab" with regard to an antibody refers to a monovalent antigen-binding fragment of the antibody consisting of a single light chain (both variable and constant regions) bound to the variable region and first constant region of a single heavy chain by a disulfide bond. Fab may be obtained by papain digestion of an antibody at the residues proximal to the N-terminus of the disulfide bond between the heavy chains of the hinge region.

"Fab‴ refers to a Fab fragment that includes a portion of the hinge region, which may be obtained by pepsin digestion of an antibody at the residues proximal to the C-terminus of the disulfide bond between the heavy chains of the hinge region and thus is different from Fab in a small number of residues (including one or more cysteines) in the hinge region.

"F(ab)2" refers to a dimer of Fab' that includes two light chains and part of two heavy chains.

"Fv" with regard to an antibody refers to the smallest fragment of the antibody to bear the complete antigen binding site. A Fv fragment consists of the variable region of a single light chain bound to the variable region of a single heavy chain. A "dsFv" refers to a disulfide-stabilized Fv fragment that the linkage between the variable region of a single light chain and the variable region of a single heavy chain is a disulfide bond.

"Single-chain Fv antibody" or "scFv" refers to an engineered antibody consisting of a light chain variable region and a heavy chain variable region connected to one another directly or via a peptide linker sequence (Huston J S et al. Proc Natl Acad Sci USA, 85:5879(1988)). A "scFv dimer" refers to a single chain comprising two heavy chain variable regions and two light chain variable regions with a linker. In certain embodiments, an "scFv dimer" is a bivalent diabody or bivalent ScFv (BsFv) comprising VH-VL (linked by a peptide linker) dimerized with another VH-VL moiety such that VH's of one moiety coordinate with the VL's of the other moiety and form two binding sites which can target the same antigens (or eptipoes) or different antigens (or eptipoes). In other embodiments, a "scFv dimer" is a bispecific diabody comprising VH1-VL2 (linked by a peptide linker) associated with VL1-VH2 (also linked by a peptide linker) such that VH1 and VL1 coordinate and VH2 and VL2 coordinate and each coordinated pair has a different antigen specificity.

"Single-chain Fv-Fc antibody" or "scFv-Fc" refers to an engineered antibody consisting of a scFv connected to the Fc region of an antibody.

"Camelized single domain antibody," "heavy chain antibody," "nanobody" or "HCAb" refers to an antibody that contains two VH domains and no light chains (Riechmann L. and Muyldermans S., J Immunol Methods. December 10; 231(1-2):25-38 (1999); Muyldermans S., JBiotechnol. June; 74(4):277-302 (2001); WO94/04678; WO94/25591; U.S. Pat. No. 6,005,079). Heavy chain antibodies were originally obtained from Camelidae (camels, dromedaries, and llamas) Although devoid of light chains, camelized antibodies have an authentic antigen-binding repertoire (Hamers-Casterman C. et al., Nature. June 3; 363(6428):446-8 (1993); Nguyen V K. et al. "Heavy-chain antibodies in Camelidae; a case of evolutionary innovation," Immunogenetics. April; 54(1):39-47 (2002); Nguyen V K. Et al. Immunology. May; 109(1):93-101 (2003)). The variable domain of a heavy chain antibody (VHH domain) represents the smallest known antigen-binding unit generated by adaptive immune responses (Koch-Nolte F. et al., FASEB J. November; 21(13):3490-8. Epub 2007 Jun. 15 (2007)). "Diabodies" include small antibody fragments with two antigen-binding sites, wherein the fragments comprise a VH domain connected to a VL domain in a single polypeptide chain (VH-VL or VL-VH) (see, e.g., Holliger P. et al., Proc Natl Acad Sci USA. July 15; 90(14):6444-8 (1993); EP404097; WO93/11161). The two domains on the same chain cannot be paired, because the linker is too short, thus, the domains are forced to pair with the complementary domains of another chain, thereby creating two antigen-binding sites. The antigenbinding sites may target the same or different antigens (or epitopes).

A "domain antibody" refers to an antibody fragment containing only the variable region of a heavy chain or the variable region of a light chain. In certain embodiments, two or more VH domains are covalently joined with a peptide linker to form a bivalent or multivalent domain antibody. The two VH domains of a bivalent domain antibody may target the same or different antigens.

In certain embodiments, a "(dsFv)2" includes three peptide chains: two VH moieties are linked by a peptide linker and bound by disulfide bridges to two VL moieties.

In certain embodiments, a "bispecific ds diabody" includes VH1-VL2 (linked by a peptide linker) bound to VL1-VH2 (also linked by a peptide linker) via a disulfide bridge between VH1 and VL1.

In certain embodiments, a "bispecific dsFv" or "dsFv-dsFv'" includes three peptide chains: a VH1-VH2 moiety wherein the heavy chains are bound by a peptide linker (e.g., a long flexible linker) and paired via disulfide bridges with VL1 and VL2 moieties, respectively. Each disulfide paired heavy and light chain has a different antigen specificity.

An "autologous cell" refers to any cell derived from a same individual as a recipient of the cell, i.e., the recipient itself.

An "allogeneic cell" is any cell derived from another individual of the same species as the recipient of the cell (i.e., not the recipient himself).

As used herein, "construct" and "protein construct" are used interchangeably to refer to a protein or protein complex. The term "complex" refers to an aggregate or component formed by the aggregation or component of more than one protein (e.g., 2, 3, 4 or more proteins). Preferably, the complex is capable of performing functions that the individual proteins within it cannot. In a complex, the constituent proteins may or may not be in contact with each other.

The term "chimeric" as used herein in relation to a construct means that part of the construct is derived from one species and another part of the construct is derived from another species.

The term "vector" as used herein refers to a vehicle into which a polynucleotide encoding a protein may be operably inserted so as to bring about the expression of that protein. A vector may be used to transform, transduce, or transfect a host cell so as to bring about expression of the genetic element it carries within the host cell. Examples of vectors include plasmids, phagemids, cosmids, artificial chromosomes such as yeast artificial chromosome (YAC), bacterial artificial chromosome (BAC), or P1-derived artificial chromosome (PAC), bacteriophages such as lambda phage or M13 phage, and animal viruses. Categories of animal viruses used as vectors include retrovirus (including lentivirus), adenovirus, adeno-associated virus, herpesvirus (e.g., herpes simplex virus), poxvirus, baculovirus, papillomavirus, and papovavirus (e.g., SV40). A vector may contain a variety of elements for controlling expression, including promoter sequences, transcription initiation sequences, enhancer sequences, selectable elements, and reporter genes. In addition, the vector may contain an origin of replication. A vector may also include materials to aid in its entry into the cell, including but not limited to a viral particle, a liposome, or a protein coating. A vector may be an expression vector or a cloning vector. The present disclosure provides vectors (e.g., expression vectors) containing the nucleic acid sequence provided herein encoding the chimeric protein construct, at least one promoter (e.g., SV40, CMV, EF-1α) operably linked to the nucleic acid sequence, and/or at least one selection marker. Examples of vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40), lambda phage, and M13 phage, plasmid pcDNA3.3, pMD18-T, pOptivec, pCMV, pEGFP, pIRES, pQD-Hyg-GSeu, pALTER, pBAD, pcDNA, pCal, pL, pET, pGEMEX, pGEX, pCI, pEGFT, pSV2, pFUSE, pVITRO, pVIVO, pMAL, pMONO, pSELECT, pUNO, pDUO, Psg5L, pBABE, pWPXL, pBI, p15TV-L, pPro18, pTD, pRS10, pLexA, pACT2.2, pCMV-SCRIPT^{®}, pCDM8, pCDNA1.1/amp, pcDNA3.1, pRc/RSV, PCR 2.1, pEF-1, pFB, pSG5, pXT1, pCDEF3, pSVSPORT, pEF-Bos etc.

The phrase "host cell" as used herein refers to a cell into which an exogenous polynucleotide and/or a vector has been or will be introduced.

The term "functional variant" as used herein refers to a polypeptide/protein that has at least 70% sequence identity to a parent polypeptide/protein but still retains the function of the parent polypeptide/protein. A variant may differ from a parent polypeptide/protein by one or more amino acid residues. For example, a functional variant may have a substitution, addition, deletion, insertion or truncation of one or more amino acid residues of a parental polypeptide/protein.

In the present application, percentage of "identity" or "sequence identity" in the context of polypeptide or polynucleotide is determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Alignment for purposes of determining percent amino acid (or nucleic acid) sequence identity can be achieved, for example, using publicly available tools such as BLASTN, BLASTp (available on the website of U.S. National Center for Biotechnology Information (NCBI), see also, Altschul S.F. et al, J. Mol. Biol., 215: 403-410 (1990) ; Stephen F. et al, Nucleic Acids Res., 25: 3389-3402 (1997)), ClustalW2 (available on the website of European Bioinformatics Institute, see also, Higgins D.G. et al, Methods in Enzymology, 266: 383-402 (1996) ; Larkin M.A. et al, Bioinformatics (Oxford, England), 23 (21) : 2947-8 (2007)), and ALIGN or Megalign (DNASTAR) software. Those skilled in the art may use the default parameters provided by the tool, or may customize the parameters as appropriate for the alignment, such as for example, by selecting a suitable algorithm. In certain embodiments, residue positions that are not identical may differ by conservative amino acid substitutions. A "conservative amino acid substitution" is one in which an amino acid residue is replaced with another amino acid residue having a side chain (R group) of similar chemical properties (e.g., charge or hydrophobicity). In general, conservative amino acid substitutions do not substantially alter the functional properties of a protein. Where two or more amino acid sequences differ from each other by conservative substitutions, the percentage or degree of similarity may be adjusted upwards to correct for the conservative nature of the substitutions. The manner in which this adjustment is made is well known to those skilled in the art, seeing, e.g., Pearson (1994) Methods Mol. Biol. 24: 307-331, which is incorporated herein by reference.

"Operatively linked" refers to a functional relationship between two or more polynucleotide sequences. In the context of a polynucleotide encoding a fusion protein (such as the polypeptide chain of a CAR of the present disclosure), the term refers to the joining of two or more polynucleotide sequences such that the two polynucleotides remain in the same expression in frame, allowing it to translate an amino acid sequence. In the context of transcriptional or translational regulation, the term refers to the functional relationship of regulatory sequences to a coding sequence, e.g., a promoter is in the correct position and orientation of a coding sequence so as to regulate transcription.

"Polynucleotide" or "nucleic acid" refers to a chain of nucleotides. As used herein polynucleotides include all polynucleotide sequences which are obtained by any means available in the art, including, without limitation, recombinant means and synthetic means.

"Polypeptide," and "protein" are used interchangeably, and refer to a chain of amino acid residues covalently linked by peptide bonds. The polypeptides include natural peptides, recombinant peptides, synthetic peptides, or a combination thereof.

"T cell receptor" or "TCR" refers to a protein complex on the surface of T cells that is responsible for recognizing fragments of antigen as peptides bound to MHC molecules.

The term "chimeric natural killer receptor" refers to a composition comprising a chimeric NK activating receptor component and a chimeric NK signal transduction component.

The term "CNK T cell" refers to a T cell containing a CNK construct.

The term "chimeric natural killer receptor-universal T cell" (CNK-UT) refers to a T cell expressing of NK-activating receptor module constructs, CNK signal transduction module constructs, and UT module constructs.

The term "CAR/CNK-UT" refers to chimeric antigen receptor & chimeric natural killer receptor-Universal T cell (CNK-UT), which refers to a T cell expressing CAR structure targeting specific Tumor antigen, NK activating receptor module construct, CNK signal transduction module construct and UT module construct.

The term "Co-stimulatory ligand", as used herein, includes a molecule on an antigen presenting cell (e.g., an APC, dendritic cell, B cell, and other immune cells) that specifically binds a cognate co-stimulatory molecule on a T cell, thereby providing a signal which, in addition to the primary signal provided by, for instance, binding of a TCR/CD3 complex with an MHC molecule loaded with peptide, mediates a T cell response, including, but not limited to, proliferation, activation, differentiation, etc. A co-stimulatory ligand can include, but is not limited to, CD7, B7-1 (CD80), B7-2 (CD86), PD-L1, PD-L2, 4-1BBL, OX40L, inducible co stimulatory ligand (ICOS-L), intercellular adhesion molecule (ICAM), CD30L, CD40, CD70, CD83, HLA-G, MICA, MICB, HVEM, lymphotoxin beta receptor, 3/TR6, ILT3, ILT4, HVEM, an agonist or antibody that binds Toll ligand receptor and a ligand that specifically binds with B7-H3. A co-stimulatory ligand also encompasses, inter alia, an antibody that specifically binds with a co-stimulatory molecule present on a T cell, such as, but not limited to, CD27, CD28, 4- 1BB, OX40, CD30, CD40, PD-l, ICOS, lymphocyte function-associated antigen-l (LFA-l), CD2, CD7 LIGHT, NKG2C, B7-H3, and a ligand that specifically binds with CD83.

A "co-stimulatory molecule" or "co-stimulatory receptor" refers to the cognate binding partner on a T cell that specifically binds with a co- stimulatory ligand, thereby mediating a co stimulatory response of the T cell, such as, but not limited to, proliferation. Co-stimulatory molecules include, but are not limited to an MHC class I molecule, BTLA, a Toll ligand receptor. Co-stimulatory molecules also include non-natural engineered proteins.

A "co-stimulatory signal', as used herein, refers to a signal, which in combination with a primary signal, such as TCR/CD3 ligation, leads to T cell proliferation and/or upregulation or down regulation of key molecules.

The term "stimulation" is meant a primary response induced by binding of a
stimulatory molecule (e.g., a TCR/CD3 complex) with its cognate ligand thereby mediating a signal transduction event, such as, but not limited to, signal transduction via the TCR/CD3 complex. Stimulation can mediate altered expression of certain molecules, such as
downregulation of TGF-B, and/or reorganization of cytoskeletal structures, and the like.

The term "stimulatory molecule" as used herein, refers to a molecule on a T cell that specifically binds with a cognate stimulatory ligand present on an antigen presenting cell.

A "stimulatory ligand", as used herein, refers to a ligand that when present on an antigen presenting cell (e.g., an APC, a dendritic cell, a B-cell, and the like) can specifically bind with a cognate binding partner (referred to herein as a "stimulatory molecule') on a T cell, thereby mediating a primary response by the T cell, including, but not limited to, activation, initiation of an immune response, proliferation, and the like. Stimulatory ligands are well-known in the art and encompass, inter cilia, an MHC Class I molecule loaded with a peptide, an anti-CD3 antibody, a Superagonist anti-CD28 antibody, and a Superagonist anti-CD2 antibody.

"Activation", as used herein, refers to the state of a T cell that has been sufficiently stimulated to induce detectable cellular proliferation. Activation can also be associated with induced cytokine production, and detectable effector functions. The term "activated T cells' refers to, among other things, T cells that are undergoing cell division.

"Effective amount" or "therapeutically effective amount" refers to an amount of a cell, composition, formulation or any material as described here effective to achieve a desirable biological result. Such results may include, without limitation, elimination of B cells expressing a specific B-cell receptor (BCR) and the antibodies produced therefrom.

As used herein, "subject" is a mammal, such as a human or other animal, and typically is a human. In some embodiments, the subject, e.g., patient, to whom the cells, cell populations, or compositions are administered is a mammal, typically a primate, such as a human. In some embodiments, the primate is a monkey or an ape. The subject can be male or female and can be of any suitable age, including infant, juvenile, adolescent, adult, and geriatric subjects. In some embodiments, the subject is a non-primate mammal, such as a rodent.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The terms do not imply complete curing of a disease or complete elimination of any symptom or effect(s) on all symptoms or outcomes.

"Preventing," as used herein, includes providing prophylaxis with respect to the occurrence or recurrence of a disease in a subject that may be predisposed to the disease but has not yet been diagnosed with the disease. In some embodiments, the provided cells and compositions are used to delay development of a disease or to slow the progression of a disease.

As used herein, "inhibit" a function or activity is to reduce the function or activity when compared to otherwise same conditions except for a condition or parameter of interest, or alternatively, as compared to another condition. For example, cells that inhibit tumor growth reduce the rate of growth of the tumor compared to the rate of growth of the tumor in the absence of the cells.

An "effective amount" of an agent, e.g., chimeric antigen receptor, polynucleotide, vector, a pharmaceutical formulation, cells, or composition, in the context of administration, refers to an amount effective, at dosages/amounts and for periods of time necessary, to achieve a desired result, such as a therapeutic or prophylactic result.

A "therapeutically effective amount" of an agent, e.g., a pharmaceutical formulation or cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the populations of cells administered. In some embodiments, the provided methods involve administering the cells and/or compositions at effective amounts, e.g., therapeutically effective amounts.

A "prophylactically effective amount" refers to an amount effective, at dosages and for periods of time necessary, to achieve the desired prophylactic result. Typically, but not necessarily, since a prophylactic dose is used in subjects prior to or at an earlier stage of disease, the prophylactically effective amount will be less than the therapeutically effective amount. In the context of lower tumor burden, the prophylactically effective amount in some aspects will be higher than the therapeutically effective amount.

### II. Target Protein Degradation (TPD)

The present disclosure provides a novel system for target protein degradation, used for targeting a specific protein (i.e., a target protein) to block the expression thereof (e.g., surface expression) and accelerate ubiquitination-mediated degradation through an endoplasmic reticulum-associated degradation (ERAD) mechanism.

ERAD refers to a cellular pathway that targets a misfolded protein in the endoplasmic reticulum for ubiquitination and subsequently degradates it by a proteasome. The process of ERAD may include three steps: (1) identificating of misfolded or mutated proteins in the endoplasmic reticulum; (2) reverse-transporting of the identified misfolded or mutated proteins from the endoplasmic reticulum to the cytoplasm, which end misfolded proteins must be transported from the endoplasmic reticulum back into the cytoplasm, which contains the ubiquitin-proteasome system (UPS); (3) using the proteasome to degrade ubiquitination-dependently identified misfolded or mutated proteins (see Annamaria et al., ER-associated degradation: Protein quality control and beyond. J. Cell Biol. Vol. 204 No. 6 869-879).

Herein, the present application provides a novel chimeric strategy that combines a targeting domain (e.g., a specific protein-targeting antibody fragment (ScFv)) with an ERAD functional motif (e.g., viral ER resident proteins TMD and ED return signals domain), and demonstrated that this strategy can effectively inhibit the expression of a specific protein (for example, the expression of specific proteins on the cell surface).

Compared with existing protein degradation/removal technologies (for example, PROTAC, CRISPR, etc.), the ER-TPD technology platform provided by the present application has the following advantages: 1. the ER-TPD chimeric protein constructs delivered into a cell through a vector (e.g., liposome, nano particle, lentivirus, adenovirus, oncolytic virus, etc.) and expressed stablely for functioning. , and compared with PROTAC, there is no problem of low cell permeability and short half-life leading to low degradation efficiency; 2. the ER-TPD technology based on a ERAD principle of the endoplasmic reticulum directly intervenes in protein synthesis and achieves efficient degradation during the process of protein synthesis and assembly in the endoplasmic reticulum, and therefore, has a higher degradation efficiency than exogenous PROTAC technology; 3. an exogenous PROTAC is not effective for degrading membrane proteins and secreted proteins, while the ER-TPD can directly target membrane proteins and secreted proteins and achieve degradation during the synthesis and assembly of endoplasmic reticulum proteins, and therefore, may target a large number of target proteins related to important diseases provide that could not be targeted by conventional TPD technologies, providing a brand-new drug development approach for disease treatment; 4. in the design method of ER-TPD, the targeting domain can adopt various single-chain antibody structures or artificial affinity ligand structures, which greatly improves the ability of specifically targeting and precise recognition and the binding ability of proteins; while conventional PROTAC technologies adopts the chemical structure of small molecules, which is difficult to ensure the specificity of the target structure, such that the ER-TPD technology has strong advantages in targeting accuracy and specificity; 5. the ER-TPD technology can realize the multi-pathway mechanism of target protein degradation through the downstream degradation-guiding ligand structure, including the UPS mechanism based on ubiquitin proteasome and the ALP mechanism of autophagy lysosome, which the downstream degradation-guiding ligand structure design can flexibly connect the degradation system in the cell through genetic engineering methods to achieve efficient targeted degradation, while conventional PROTAC technologies may only achieve degradation by targeting E3 ligase; 6. the ER-TPD technology degrades target proteins at the protein level, and compared with technologies that block the expression of target proteins at the gene level (such as CRISPR technologies), there are no toxic side effects such as chromosome instability caused by off-target effects or gene editing processes; 7. the ER-TPD technology can be combined with genes and cells treatment technologies perfectly, greatly expanding the current clinical indications of gene and cell therapy and enhances the clinical effect; and 8. the development of targeted degradation drugs based on ER-TPD technology is highly feasible and the cycle is short, wherein nucleic acid based on ER-TPD technology cellular drugs are easier to achieve large-scale and industrial production, and are an important basic platform technology for the development of new drugs.

In one aspect, the present disclosure provides a chimeric protein construct of endoplasmic reticulum-based targeted protein degradation technology (ER-TPD), which includes endoplasmic reticulum ER-associated degradation (ERAD) mechanism protein binding domain and targeting domain.

In some embodiments, the chimeric protein construct further includes a protein degradation pathway member (e.g., an ubiquitin-proteasome system pathway member, an endosome-lysosome pathway member, an autophagy pathway member) binding domain. The protein degradation pathway member binding domain may be linked to the constituent elements of the chimeric protein construct in any suitable manner, for example, to the ERAD machinery protein binding domain.

In another aspect, the present disclosure also provides a library of compositions, wherein each composition includes a viral protein or a nucleic acid molecule encoding the same containing a protein capable of hijacking the ERAD machinery to arrest and accelerate proteasome-mediated degradation within the ER. Functional motifs and affinity moieties that target specific proteins for degradation are used for therapeutic purposes to modify cell phenotype, improve cell function or induce apoptosis or inhibit viral replication.

### 1. ERAD Mechanism Protein Binding Domain

The term "ERAD mechanism protein" as used herein refers to a protein involved in the ERAD mechanism or pathway. The term "ERAD mechanism protein binding domain", also known as "ERAD degradation domain", refers to a portion or domain capable of binding and/or utilizing a protein of the ERAD mechanism, for example, a transmembrane domain (or a functional variant thereof) and a cytoplasmic domain (or a functional variant thereof) of a viral endoplasmic reticulum (ER) resident glycoprotein. which is also referred to as an "endoplasmic reticulum resident domain" or "ER resident domain".

In some embodiments, the ERAD mechanism protein binding domain comprises: a transmembrane domain of a viral endoplasmic reticulum resident protein or a functional variant thereof, and, an endoplasmic reticulum resident domain or a functional variant thereof. In some embodiments, the viral ER resident glycoprotein can be any viral ER resident protein capable of hijacking the ERAD machinery and promoting ubiquitination and proteasome-mediated degradation of a target protein.

### 1. ERAD Mechanism Protein Binding Domain Derived from Adenovirus E3-19K

In some embodiments, the viral endoplasmic reticulum resident protein is adenovirus E3-19K. Adenovirus E3-19K (also known as "E19") contains three functional modules: a luminal domain for interaction with MHC-I and MICA/B molecules, a transmembrane domain, and an endoplasmic reticulum resident domain, wherein the ER resident domain contains a dilysine motif in the cytoplasmic tail that may return a Golgi apparatus to the ER, which may therefore also be referred to as an "ER return signaling motif". Studies have shown that a transmembrane domain and ER return signaling motif are required to ensure efficient ER localization, transport-inhibition of histocompatibility complex class I (MHC-I) and MHC-I-associated chains A and B (MICA/B molecules), and proteasomal degradation. Thus, adenovirus E3-19K may reside in MHC class I molecules (e.g., MHC-I and MICA/B molecules) in a secretory pathway and interfere with antigen presentation. The chimeric protein construct provided by the present disclosure utilizes a transmembrane domain and endoplasmic reticulum resident domain (i.e., ERAD degradation domain) of an adenovirus E3-19K to fuse with the targeting protein binding domain, thereby realizing the purpose of degrading a target protein.

In some preferred embodiments, the ERAD degradation domain of the adenovirus E3-19K includes an amino acid sequence having 80% or more identity to the amino acid sequence shown in SEQ ID NO. 60, preferably an amino acid sequence having an identity of 85%, 90%, 95%, 96%, 97%, 98%, or 99% or more, and more preferably an amino acid sequence having an identity of 98%, or 99% or more. The EARD degradation domain of the adenovirus E3-19K has a dilysine motif in the cytoplasmic tail, capable of returning the Golgi apparatus to the endoplasmic reticulum. In some preferred embodiments, the amino acid sequence of the ERAD degradation domain of the adenovirus E3-19K is shown in SEQ ID NO. 60.

### ii. ERAD mechanism protein-binding domains derived from other viral glycoproteins

In some embodiments, the viral endoplasmic reticulum resident protein is not adenovirus E3-19K.

In some embodiments, the viral endoplasmic reticulum resident protein is selected from the at least one of the group consisting of: HCMV glycoprotein US2, US 11, US3, US10, US6, HSV ICP47, CPXV12, BHV UL49.5, EBV BNFL2a, HCMV UL16, UL141, UL142, HIV Nef, HIV Vpu, HHV-7 U21, HHV-8 KK3, HHV-8 KK5, MHV-68 MK3, HTLV-1 p12 and Cowpox Virus protein CPXV203. In some embodiments, the viral endoplasmic reticulum resident protein includes HCMV glycoproteins US2 and US11.

In some embodiments, the viral endoplasmic reticulum resident protein includes E3-19K and is selected from at least one of the group consisting of: HCMV glycoprotein US2, US11, US3, US10, US6, HSV ICP47, CPXV12, BHV UL49.5, EBVBNFL2a, HCMV UL16, UL141, UL142, HIVNef, HIV Vpu, HHV-7 U21, HHV-8 KK3, HHV-8 KK5, MHV-68 MK3, HTLV-1 p12 and Cowpox Virus protein CPXV203.

The Vpu protein of HIV-1 virus is a protein on the ER membrane and targets newly generated CD4 in the endoplasmic reticulum for degradation by the proteasome in the cytoplasm. The Vpu protein retains CD4 in the ER primarily through SCFβ-TrCP-dependent ubiquitination and transmembrane domain (TMD) interactions.

### a) HCMV US2 and US 11

Both HCMV US2 and US 11 proteins are ER-resident type I integral membrane glycoproteins that co-select this ERAD pathway to promote the degradation of MHC class I heavy chains, thereby inhibiting MHC class I antigen presentation. Expression of either protein results in rapid degradation of newly synthesized MHC class I heavy chains. US2 and US11 bind to MHC class I heavy chains through their luminal domains and recruit host cell proteins that extract polypeptides from the endoplasmic reticulum membrane by "pulling" the cytoplasmic tails of the heavy chains. After translocation into the cytoplasm, MHC class I molecules are ubiquitinated and degraded by the proteasome.

In addition to class I molecules, US2 also leads to the degradation of two proteins of the class II pathway, DR-α and DM-α, as well as HFE, a non-canonical major histocompatibility complex (MHC) class I protein involved in iron regulation.

The luminal domain of US2 is responsible for binding MHC class I and II molecules, and the transmembrane domain (TM) and cytoplasmic domain (CT) interact with cellular components of ERAD mechanisms or pathways, and facilitate translocation and promotion of enzymolysis of both MHC class I and class II proteins (Chevalier M S et al., 2002, 2003). The cytoplasmic tail of US2 is sufficient to interact with signal peptide peptidase (SPP), an essential component of the US2-dependent MHC I dislocation complex (Loureiro J et al., 2006), which is necessary for US2-dependent MHC heavy chain translocation (seeing Joana et al., Signal peptide peptidase is required for dislocation from the endoplasmic reticulum. Nature volume 441, pages 894-897 (2006)). In addition, US2 interacts with the endoplasmic reticulum-resident RING-type E3 ligase TRC8 through its TM domain, which also contributes to the ubiquitination and proteasomal degradation of US2 tail-anchored MHC I and II molecules (Stagg H R et al., 2009).

In contrast, US11-induced degradation of MHC-I molecules requires Derlin-1, rather than SPP. The ER luminal domain of US 11 interacts with the luminal domain of the MHC-I heavy chain, while the TM domain of US11 binds Derlin-1. Therefore, the main function of US11 may be to deliver MHC-I molecules to Derlin-1 (Lilley B N et al., 2004; Cho S et al., 2013), and then induce their translocation to the cytosol for proteasomal degradation. Furthermore, US11 activates unfolded proteins. Through Derlin-1, US11 associates with TMEM129 as an ERAD RING E3 ligase and recruits Ube2J2 to ubiquitinate MHC-I prior to US11-induced degradation.

In some preferred embodiments, the ERAD degradation domain of the HCMV glycoprotein US2 includes an amino acid sequence having 80% or more identity to the amino acid sequence shown in SEQ ID NO. 48, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the ERAD degradation domain of the HCMV glycoprotein US2 is shown in SEQ ID NO. 48.

In some preferred embodiments, the ERAD degradation domain of the US2 includes an amino acid sequence having 80% or more identity to the amino acid sequence shown in SEQ ID NO. 76, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the ERAD degradation domain of the US2 is shown in SEQ ID NO. 76.

In some preferred embodiments, the ERAD degradation domain of the HCMV glycoprotein US 11 includes an amino acid sequence having 80% or more identity to the amino acid sequence shown in SEQ ID NO. 54, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the ERAD degradation domain of the HCMV glycoprotein US11 is shown in SEQ ID NO. 54.

In some preferred embodiments, the ERAD degradation domain of the US11 includes an amino acid sequence having 80% or more identity to the amino acid sequence shown in SEQ ID NO. 82, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the ERAD degradation domain of the US 11 is shown in SEQ ID NO. 82.

### b)HCMV US3

Rather than promoting the degradation of MHC proteins, HCMV US3 glycoprotein physically binds to peptide-loaded MHC class I heterodimers, resulting in the retention of class I complexes in ER and inhibition of the binding of invariant chains to class II DR-αβdimers in ER, and resulting in mislocalization of class II complexes and reduced peptide loading. Therefore, US3 is capable of interfering with the intracellular trafficking and maturation of MHC class I molecules during the early stages of HCMV infection. US3 is an endoplasmic reticulum-resident membrane protein. Domain swapping experiments revealed that the luminal domain of US3 is sufficient for US3 to maintain the ER by itself, whereas both the luminal and transmembrane domains are required for the retention of MHC class I molecules in the ER.

In some preferred embodiments, the ERAD degradation domain of the HCMV glycoprotein US3 includes an amino acid sequence having 80% or more identity to the amino acid sequence shown in SEQ ID NO. 51, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the ERAD degradation domain of the HCMV glycoprotein US3 is shown in SEQ ID NO. 51.

In some preferred embodiments, the ERAD degradation domain of the US3 includes an amino acid sequence having 80% or more identity to the amino acid sequence shown in SEQ ID NO. 79, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the ERAD degradation domain of the US3 is shown in SEQ ID NO. 79.

### c)HCMV US10

The HCMV US10 glycoprotein also interacts with components of MHC class I antigen presentation. US 10 binds free class I heavy chains and delays their transport from the ER. However, US 10 does not affect US2 or US11.

In some preferred embodiments, the ERAD degradation domain of the HCMV glycoprotein US 10 includes an amino acid sequence having 80% or more identity to the amino acid sequence shown in SEQ ID NO. 57, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the ERAD degradation domain of the HCMV glycoprotein US10 is shown in SEQ ID NO. 57.

### d)HCMV US6, HSV ICP47, CPXV012, EBV BNFL2a, BHV UL49.5

Non-identical with US2, US3, US10, and US11, the HCMV L protein US6 affects antigen presentation through a completely different strategy. In contrast to interacting with free class I heavy chains or fully assembled class I complexes, US6 inhibits translocation of cytosolic peptides through the TAP complex (TAP1/2). US6 binds to the luminal side of the ER of TAP1 and causes a conformational change that prevents ATP binding. Residues 89-108 in the ER-luminal domain of US6 contribute to the binding of US6 to TAP and are both necessary and sufficient for this inhibition. This inhibition of TAP activity affects not only the expression of the classical MHC class I alleles, but also the expression of the nonclassical alleles HLA-C and HLA-G in fetal cytotrophoblast cells.

Acting as a HCMV US6 protein, HSV ICP47 is expressed early in the infection cycle and dispensable for replication in vitro, and the same strategy could be applied to prevent class I molecular component. ICP47 blocks TAP-mediated peptide transport and binds tightly to the TAP1-TAP2 complex. A clue to the mechanism by which ICP47 blocks TAP is that it exhibits high species selectivity. Both HSV1 and HSV2I CP47 inhibit the TAP in simian, monkey, pig, dog and bovine, and have little effect on the TAP in mouse, rat, guinea pig or rabbit. ICP47 has an approximately 100-fold higher affinity for human TAP than for mouse TAP. ICP47 inhibits peptide binding to TAP but does not affect ATP binding. ICP47 has a 10-1000-fold higher affinity for TAP than most peptides, acts as a competitive inhibitor of peptide binding to TAP, and is thought to bind directly to the peptide-binding site.

In some preferred embodiments, the TAP binding domain of HHV-7 US6 includes an amino acid sequence having 80% or more identity to the amino acid sequence shown in SEQ ID NO. 65, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the TAP binding domain of the HHV-7 US6 is shown in SEQ ID NO. 65.

In some preferred embodiments, the TAP binding domain of HSV ICP47 includes an amino acid sequence having 80% or more identity to the amino acid sequence shown in SEQ ID NO. 67, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the TAP binding domain of the HSV ICP47 is shown in SEQ ID NO. 67.

In some preferred embodiments, the TAP binding domain of the CPXV012 includes an amino acid sequence having 80% or more identity to the amino acid sequence shown in SEQ ID NO. 69, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the TAP binding domain of the CPXV012 is shown in SEQ ID NO. 69.

In some preferred embodiments, the full-length sequence of the CPXV012 includes an amino acid sequence having 80% or more identity to the amino acid sequence shown in SEQ ID NO. 68, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the CPXV012 is shown as in SEQ ID NO.68.

In some preferred embodiments, the TAP binding domain of the EBV BNFL2a includes an amino acid sequence having 80% or more identity to the amino acid sequence shown in SEQ ID NO. 71, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the TAP binding domain of the EBV BNFL2a is shown in SEQ ID NO. 71.

In some preferred embodiments, the full-length sequence of the EBV BNFL2a includes an amino acid sequence having 80% or more identity to the amino acid sequence shown in SEQ ID NO. 70, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the EBV BNFL2a is shown as in SEQ ID NO. 70.

In some preferred embodiments, the TAP binding domain of the BHV UL49.5 includes an amino acid sequence having 80% or more identity to the amino acid sequence shown in SEQ ID NO. 73, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the TAP binding domain of the BHV UL49.5 is shown in SEQ ID NO. 73.

In some preferred embodiments, the full-length sequence of the BHV UL49.5 includes an amino acid sequence having 80% or more identity to the amino acid sequence shown in SEQ ID NO. 72, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the BHV UL49.5 is shown as in SEQ ID NO. 72.

### e) Others

In some preferred embodiments, the ERAD degradation domain of the HHV-7 US21 includes an amino acid sequence having 80% or more identity to the amino acid sequence shown in SEQ ID NO. 63, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the ERAD degradation domain of the HHV-7 US21 is shown in SEQ ID NO. 63.

In some preferred embodiments, the full-length sequence of the HHV-7 US21 includes an amino acid sequence having 80% or more identity to the amino acid sequence shown in SEQ ID NO. 61, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HHV-7 US21 is shown as in SEQ ID NO. 61.

In some preferred embodiments, the ERAD degradation domain of the HCMV UL16 includes an amino acid sequence having 80% or more identity to the amino acid sequence shown in SEQ ID NO. 85, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the ERAD degradation domain of the HCMV UL16 is shown in SEQ ID NO. 85.

In some preferred embodiments, the full-length sequence of the HCMV UL16 includes an amino acid sequence having 80% or more identity to the amino acid sequence shown in SEQ ID NO. 83, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV UL16 is shown as in SEQ ID NO. 83.

In some preferred embodiments, the ERAD degradation domain of the HCMV UL141 includes an amino acid sequence having 80% or more identity to the amino acid sequence shown in SEQ ID NO. 88, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the ERAD degradation domain of the HCMV UL141 is shown in SEQ ID NO. 88.

In some preferred embodiments, the full-length sequence of the HCMV UL141 includes an amino acid sequence having 80% or more identity to the amino acid sequence shown in SEQ ID NO. 86, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMVUL141 is shown as in SEQ ID NO. 86.

In some preferred embodiments, the Golgi-resident domain of the HCMV UL142 includes an amino acid sequence having 80% or more identity to the amino acid sequence shown in SEQ ID NO. 91, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the Golgi-resident domain of HCMV UL142 is shown in SEQ ID NO. 91.

In some preferred embodiments, the HIV Nef includes an amino acid sequence having 80% or more identity to the amino acid sequence shown in SEQ ID NO. 92, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the HIV Nef is shown in SEQ ID NO. 92.

In some preferred embodiments, the full-length sequence of the HCMV UL142 includes an amino acid sequence having 80% or more identity to the amino acid sequence shown in SEQ ID NO. 89, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV UL142 is shown as in SEQ ID NO. 89.

In some preferred embodiments, the HIV Vpu includes an amino acid sequence having 80% or more identity to the amino acid sequence shown in SEQ ID NO. 93, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the HIV Vpu is shown in SEQ ID NO. 93.

In some preferred embodiments, the HHV-8 KK3 includes an amino acid sequence having 80% or more identity to the amino acid sequence shown in SEQ ID NO. 94, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the HHV-8 KK3 is shown in SEQ ID NO. 94.

In some preferred embodiments, the HHV-8 KK5 includes an amino acid sequence having 80% or more identity to the amino acid sequence shown in SEQ ID NO. 95, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the HHV-8 KK5 is shown in SEQ ID NO. 95.

In some preferred embodiments, the MHV-68 MK3 includes an amino acid sequence having 80% or more identity to the amino acid sequence shown in SEQ ID NO. 96, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the MHV-68 MK3 is shown in SEQ ID NO. 96.

In some preferred embodiments, the HTLV-1 p12 includes an amino acid sequence having 80% or more identity to the amino acid sequence shown in SEQ ID NO. 97, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the HTLV-1 p12 is shown in SEQ ID NO. 97.

In some preferred embodiments, the KDEL receptor binding domain of the vaccinia virus protein CPXV203 includes an amino acid sequence having 80% or more identity to the amino acid sequence shown in SEQ ID NO. 100, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the KDEL receptor binding domain of the vaccinia virus protein CPXV203 is shown in SEQ ID NO. 100.

In some preferred embodiments, the KDEL receptor binding domain of the vaccinia virus protein CPXV203 includes an amino acid sequence having 80% or more identity to the amino acid sequence shown in SEQ ID NO. 103, preferably an amino acid sequence having an identity of 85%, 90%, 95%, 96%, 97%, 98%, or 99% or more, and more preferably an amino acid sequence having an identity of 98%, or 99% or more, and has the function of endoplasmic reticulum residence. In some embodiments, the amino acid sequence of the KDEL receptor binding domain of the vaccinia virus protein CPXV203 is shown in SEQ ID NO. 103.

In some preferred embodiments, the full-length sequence of the vaccinia virus protein CPXV203 includes an amino acid sequence having 80% or more identity to the amino acid sequence shown in SEQ ID NO. 98, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the vaccinia virus protein CPXV203 is shown as in SEQ ID NO.98.

In some preferred embodiments, the full-length sequence of the vaccinia virus protein CPXV203 includes an amino acid sequence having 80% or more identity to the amino acid sequence shown in SEQ ID NO. 101, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the vaccinia virus protein CPXV203 is shown as in SEQ ID NO. 101.

### 2. Target binding domain

The target binding domain of the chimeric protein constructs provided in this disclosure may be any structure that recognizes and binds the target protein, e.g., antibodies, antibody fragments, functional motifs derived from natural proteins (for example, natural ligands), artificially synthesized polypeptides or proteins with affinity for a target protein, or various variants such as mutants, fusions, and truncations of the above-mentioned molecules. For example, the targeting domain may be a natural ligand of the target protein, an antibody or an antigen-binding fragment thereof that specifically recognizes the target protein, or an antigen that can be specifically recognized by an antibody.

In some embodiments, the target binding domain includes an antibody of functional fragment thereof for specifically target binding a target protein, e.g., diabodies, Fab, Fab', F(ab')2, Fd, Fv fragments, disulfide stabilized Fv fragments (dsFv), (dsFv)2, bispecific dsFv (dsFv-dsFv'), disulfide bond stabilized diabodies (ds diabodies), single chain antibody molecules (scFv), scFv dimers (bivalent diabodies), Fv(scFv),multispecific antibodies(e.g., bispecific antibodies),, camelized single domain antibodies (e.g., VHH), nanobodies, domain antibodies, bivalent domain antibodies, diabodies, triabodies, and quadruple antibodies. The target binding protein domain may also include any binding ligands capable of binding target proteins, including but not limited to, a ligand, a part of binding a ligand in a receptor, a part of binding a host protein in a viral protein, etc.

A target protein may be any protein of which level or activity is expected to be modulated, e.g., altering activity through the ERAD mechanism to block proteins in the ER and accelerate proteasome-mediated degradation, which may alter the phenotype of the cell and improve cell function, causing cell apoptosis and/or inhibiting virus replication, etc., so as to achieve therapeutic purposes (for example, improve therapeutic effect, reduce side effects, etc.). In some embodiments, the target protein may be a protein involved in cell cycle, apoptosis, signal transduction, cell differentiation, cell dedifferentiation, cell growth, production of cytokines or bioregulators thereof, production of cytokines or bioregulators, production of a regulatory or functional protein, or a protein for pro-inflammatory signaling or glucose-regulation pathways.

In another embodiment, the target protein is a disease-associated protein (also referred to as a "pathogenic protein"), the "disease-associated protein" or "pathogenic protein" described herein refers to the protein any change in which function or activity will lead to the occurrence of the disease, or which function is more important for the development of the disease, exemplary pathogenic protein includes, but is not limited to, oncogenic protein, viral protein, autoimmune disease protein (e.g., antibodies produced by plasma cells).

In some embodiments, the target protein is selected from the group consisting of: immune function-related target proteins, neurological disease-related target proteins, infectious disease-related target proteins (e.g., viral infection-related target proteins), autoantigen-related target proteins or tumor-related target proteins (or oncogenic proteins), metabolic disease-related target proteins.

In some embodiments, the pathogenic protein is an oncogenic protein. The oncogenic protein may be encoded by an oncogene, including but not limited to BCL-2, c-MYC, Ras, HER2, BCR/ABL, ABL1/BCR, TGFB1, TLX1, P53, WNT1, WNT2, WT1, αv-β3, PKCa, ABL, BCL1, CD24, CDK4, EGFR/ERBB- 1. HSTF1, INT1/WNT1, INT2, MDM2, MET, MYB, MYC, MYCN, MYCL1, RAFI, NRAS, REL, AKT2, APC, BCL2-ALPHA, BCL2-BETA, BCL3, BCR, BRCA1, BRCA2, CBL, CCND1, CDKN1A, CDKN1C, CDKN2A, CDKN2B, CRK, CRK-II, CSF1R/FMS, DBL, DDOST, PMS-2, PRAD-1, RAF, RHOM-1, RHOM-2, SIS, TAL2, TANI, TIAM1, TSC2, TRK, TSC1, STK11, PTCH, MEN1, MEN2, P57/KIP2, PTEN, HPC1, ATM, XPA/XPG, BCL6, DEK, AKAP13, CDH1, BLM, EWSR1/FLI1, FES, FGF3, FER, FGR, FLI1/ERGB2, FOS, FPS/FES, FRA1, FRA2, FYN, HCK, HEK, HER3/ERBB-2, ERBB-3, HER4/ERBB-4, HST2, INK4A, INK4B, JUN, JUNB, JUND, KIP2, KIT, KRAS2A, KRAS2B, LCK, LYN, MAS, MAX, MCC, MLH1, MOS, MSH2, MYBA, MYBB, NF1, NF2, P53, PDGFB, PIM1, PTC, RBI, RET, ROS1, SKI, SRC1, TALI, TGFBR2, THRA1, THRB, TIAM1, TRK, VAV, VHL, WAF1, WNT2, WT1, YES1, ALK/NPM1, AMI1, AXL, FMS, GIP, GLI, GSP, HOX11, HST, IL3, INT2, KS3, K- SAM, LBC, DCC, DPC4/SMAD4, E-CAD, E2F1/RBAP, ELK1, ELK3, EPH, EPHA1, E2F1, EPHA3, ERG, ETS1, ETS2, LMO-1, LMO-2, L-MYC, LYL1, LYT-10, MDM-2, MLH1, MLL, MLM, N-MYC, OST, PAX-5, PMS-1, FGF4, FGF6, FANCA, FLI1/ERGB2, FOSL1, FOSL2, GLI, HRAS1, HRX/MLLT1, HRX/MLLT2, KRAS2, MADH4, MASI, MCF2, MLLT1/MLL, MLLT2/HRX, MTG8/RUNX1, MYCLK1, MYH11/CBFB, NFKB2, NOTCH1, NPM1/ALK, NRG/REL, NTRK1, PBX1/TCF3, PML/ RARA, PRCA1, RUNX1, RUNX1/CBFA2T1, SET, SHP2, TCF3/PBX1, TNFa, Clusterin, Survivin, TOEβ, c-fos, c-SRC, and INT-1. In certain embodiments, the oncogenic protein may be a member of the Bcl-2 family (e.g., Bcl-2, Bcl-xL and Bcl-w), VEGF / VEGFR, PDGFRβ, EGFR, EGFR mutants, IGF-1R, HDACs, HER2, MYC, KRAS, AFP, CEA, CA199, estrogen receptor ER-α, androgen receptor (AR), tyrosine kinase (c-ABL, BCR-ABL, BTK, FAK, PTK6, Wee1, TRK transmembrane receptor), serine/threonine kinase receptor (IRAK4, LRRK2, B-Raf, RIPK2, CDK4/6, CDK7, CDK8, CDK8/19, CDK9, TBK1), protein kinase II (CK2), epigenetic related proteins (BRD2, BRD3, BRD4, BRDT, TRIM24, BRD9, PBRM1, SMARCA2, SMARCA4, EP300, EZH2, WDR5), adrenal medullary hormone (ADM), and DPP3.

In some embodiments, the target protein includes an immune function-related protein. The term "immune function-related protein" as used herein refers to a functional protein involved in body's immune process, e.g., an antigen-presenting molecule (e.g., a MHC class I molecule, a MHC class II molecule, a HLA, etc.), an antigen recognition molecule (e.g., TCR, CD123, NKG2D, etc.), an immune checkpoint molecule (e.g., PD-1, PD-L1, CTLA4, TIM3, TIGIT, LAG3, A2AR, BTLA, IDO1, IDO2, TDO, KIR, NOX2, VISTA , SIGLEC7, PVR, etc.), an immune stimulatory/co-stimulatory molecule (e.g., CD3, CD80/86, CD28, etc.), and other molecules involved in innate or adaptive immunity. In some embodiments, target proteins associated with immune function include, e.g., CD123, CD7, CD5, MHC class I molecules, MHC class II molecules, non-classical MHC molecules (e.g., HLA-G, HLA-E), MICA/B, ULBP1-6, IL6, IL6 receptor, IL1 receptor, RANKL, TGF-β1, PD1, PD-L1, CTLA4, Tim3, LAG3, Siglec-15, TIGIT, CD47, IL4RA, CD94/NKG2A, CXCR1/2, CXCL8, CCR2/CCR5, CCR4, CXCR4, c-Rel, CCL2, CCL5, CCL20, CCL22, CSF-1, CCL2, CCL2, CCL5 indolamine-2,3-dioxygenase (IDO), or arginase 1 (ARG1).

In some embodiments, the target protein includes a neurological disease-related target protein. As used herein, the term " nervous system diseases " refers to a protein involved in neurological diseases, especially a protein in neurodegenerative diseases. In some embodiments, the neurological disease-related target protein includes, for example, Tau, amyloid-β (Aβ), α Synuclein, mutant huntingtin (mHTT), α-synuclein, TAR RNA binding protein (TARDBP) and FUS RNA binding protein (FUS).

In some embodiments, the target protein includes a target protein associated with viral infection. The target protein associated with viral infection includes, for example, HBV encoded X protein (HBx), HBV DNA polymerase, HBV capsid glycoprotein, HIV-1 reverse transcriptase, HIV gp120, HCV NS3-4A protease, HCV RNA polymerase, HCV envelope protein, EBV DNA polymerase, EBV EBNA1 , coronavirus RNA synthetase, coronavirus spike protein, coronavirus envelope protein, coronavirus membrane protein, coronavirus nucleocapsid protein, RNA-dependent RNA polymerase (RdRp), such as novel coronavirus RNA Dependent RNA polymerase, Herpesviruses DNA and RNA polymerase, Herpesvirus capsid glycoprotein, CMV DNA polymerase, CMV capsid glycoprotein, RSV envelope protein, RSV capsid protein, RSV RNA polymerase, Influenza virus RNA polymerase, influenza virus envelope protein, HPV DNA polymerase, or HPV capsid protein. In another embodiment, the target protein is a viral protein, e.g., a HBV surface antigen, HBeAg, HBV polymerase protein, HIV Gag protein, HIV Env protein, etc., of which function is considered important for viral replication amplification and function and the progression of viral diseases.

In some embodiments, the target protein includes an autoantigen-associated protein. A variety of autoantigens associated with autoimmune diseases are known in the art. For instance, there are: an autoantigen associated with type 1 diabete, e.g., an islet cell antigen (ICA), an insulin (IAA), a glutamine Acid decarboxylase 65 (GAD65), an insulinoma antigen-2 (IA-2); an autoantigen associated with rheumatoid arthritis (RA), e.g., a citrullinated protein/peptide antibody, a heteroribonucleoprotein (heterogeneous nuclear ribonucleoprotein A2/B1), an aldolase, an alpha-enolase, a calreticulin, a heat-activated protein (HSP60), BiP, PGK1, a stress-induced phosphoprotein 1, FUSE-BP1/2; a systemic lupus erythematosus (SLE)-associated autoantigen, e.g., a deoxyribonucleoprotein, SmD1, SmD3, Clq, a sore anticoagulant (LA), cardiolipin (CL), β2 glycoprotein I (β2 GPI), a prothrombin (PT) and phosphatidylserine (PS); an autoantigen associated with Systemic Sclerosis (SSc)/scleroderma (SD), e.g., Scl-70, SSA, Ro52; an antigen associated with autoimmune liver diseases, e.g., mitochondrial antigen, Sp100, PML, gp210, p62; an autoantigen associated with myasthenia gravis, e.g., an acetylcholine receptor; an autoantigen associated with central nervous system autoimmune disease (limbic encephalitis, encephalomyelitis, cerebellar ataxia), e.g., voltage-gated potassium channels (VGKC) complex, voltage-gated calcium channel receptor, a-amino-3-hydroxy-5-methylisoxazole-4-propionic acid (AMPA) receptor, γ-aminobutyric acid-B (GABAB ) receptors, glycine receptors; an autoantigen associated with multiple sclerosis, e.g., a myelin basic protein (MBP), a myelin oligodendrocyte glycoprotein (MOG); an autoantigen associated with polymyositis (PM) and Myositis (DM), e.g., Jo-1, Mi-2, PM-Scl, Ro-52; an autoantigen associated with gluten-sensitive enteropathy, e.g., endomysial antibody (EMA), tissue transglutaminase (tTG); an autoantigen associated with anti-NMDAR antibody encephalitis, e.g., N-methyl-D-aspartate receptor; an autoantigen associated with neuromyelitis optica (NMO), e.g., aquaporin 4 (AQP4); and, an autoantigen related to reproduction, e.g., ovarian antigen, sperm antigen.

In some embodiments, the pathogenic protein is a target protein associated with metabolic diseases. The target protein associated with metabolic diseases includes, for example: a target protein for atherosclerosis, AS, including, but not limited to, CD36, low-density lipoprotein receptor (LDLR), ChemR23 (CMKLR1), mitochondrial dehydrogenase (mitochondrial dehydrogenase) ALDH4A1 (target protein); target proteins for type 2 diabetes, including but not limited to, RalGAPα1, dipeptidyl peptidase IV (DPP4); Target proteins for nonalcoholic fatty liver disease, including but not limited to, TMEM16A, VAMP3; target proteins for tumor glucose metabolism, including but not limited to, hexokinase (HK), glucosetransporter 1 (GLUT1), glucose transporter 4 (GLUT4), phosphoglycerate dehydrogenase, lactate dehydrogenase; target proteins for tumor lipid metabolism, including but not limited to, ATP citrate lyase (ACLY), fatty acid synthase (FASN), and acetyl-CoA acetyltransferase 1 (ACAT1).

In some embodiments, the target binding domain specifically targets other target proteins in addition to the following target proteins: TCR, HLA-I, MICA and MICB; and the viral endoplasmic reticulum resident protein is any viral endoplasmic reticulum resident protein described in the present disclosure.

In some embodiments, the target binding domain specifically targets at least two of the following target proteins: TCR, HLA-I, MICA, MICB; and the viral endoplasmic reticulum resident protein is any viral endoplasmic reticulum resident protein described in the present disclosure.

In some embodiments, the target binding domain specifically targets TCR, HLA-I, MICA or MICB, and the viral endoplasmic reticulum resident protein is not adenovirus E3-K19.

In some embodiments, the target binding domain of the chimeric protein construct is connected to the ERAD mechanism protein binding domain via a hinge or linker. In some embodiments, the hinge includes an amino acid sequence as indicated by SEQ ID NO. 123 (IgG4 hinge) or SEQ ID NO.133 (((Gly4Ser)2).

In some preferred embodiments, the HLA binding domain of the HCMV glycoprotein US2 includes an amino acid sequence having 80% or more identity to the amino acid sequence shown in SEQ ID NO. 47, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the HCMV glycoprotein US2 is shown as in SEQ ID NO. 47.

In some preferred embodiments, the HLA binding domain of the US2 includes an amino acid sequence having 80% or more identity to the amino acid sequence shown in SEQ ID NO. 75, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the US2 is shown as in SEQ ID NO. 75.

In some preferred embodiments, the MHC binding domain of the HCMV glycoprotein US 11 includes an amino acid sequence having 80% or more identity to the amino acid sequence shown in SEQ ID NO. 53, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the HCMV glycoprotein US11 is shown as in SEQ ID NO. 53.

In some preferred embodiments, the HLA binding domain of the US11 includes an amino acid sequence having 80% or more identity to the amino acid sequence shown in SEQ ID NO. 81, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the US11 is shown as in SEQ ID NO. 81.

In some preferred embodiments, the HLA binding domain of the HCMV glycoprotein US3 includes an amino acid sequence having 80% or more identity to the amino acid sequence shown in SEQ ID NO. 50, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the HCMV glycoprotein US3 is shown as in SEQ ID NO. 50.

In some preferred embodiments, the HLA binding domain of the US3 includes an amino acid sequence having 80% or more identity to the amino acid sequence shown in SEQ ID NO. 78, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the US3 is shown as in SEQ ID NO. 78.

In some preferred embodiments, the HLA binding domain of the HCMV glycoprotein US 10 includes an amino acid sequence having 80% or more identity to the amino acid sequence shown in SEQ ID NO. 56, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the HCMV glycoprotein US 10 is shown as in SEQ ID NO. 56.

In some preferred embodiments, the binding protein molecular domain targeting MHC I and/or MHC II or its functional variants are derived from viral glycoproteins that degrade MHC and/or MHC II molecules; preferably, viral glycoproteins are selected from HCMV glycoprotein US2, US3, US 11 or US 10, adenovirus E3-19K, or HHV-7 US21.

In some preferred embodiments, the binding protein molecular domain targeting MHC I and/or MHC II or functional variants thereof further includes a viral protein that directively inhibits or degrades the NK target protein of MICA, MICB, ULBP1, ULBP2, ULBP3, ULBP4, ULBP5 or ULBP6; preferably, the viral protein is selected from HCMV UL 16, UL141, UL142, or adenovirus E3-19K.

In some preferred embodiments, the binding protein molecular domain targeting MHC I and/or MHC II or its functional variants further includes a viral protein that transports MHC I molecules from the Golgi apparatus to lysosomes for degradation; preferably, the viral protein is selected from HIV Nef, HIV Vpu, HHV-7 U21, HHV-8 KK3, HHV-8 KK5, MHV-68 MK3, and HTLV-1 p12.

In some preferred embodiments, the binding protein molecular domain targeting MHC I and/or MHC II or functional variants thereof further includes a viral protein that mediates the return of MHC- polypeptide molecules from the Golgi apparatus to the endoplasmic reticulum and promotes their degradation; preferably, the viral protein comprises an MHC binding structure and a KDEL receptor binding domain; preferably, the viral protein is Cowpox virus protein CPXV203.

In some preferred embodiments, the MHC binding domain of the HHV-7 US21 includes an amino acid sequence having 80% or more identity to the amino acid sequence shown in SEQ ID NO. 62, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the HHV-7 US21 is shown as in SEQ ID NO. 62.

In some preferred embodiments, the NK target protein binding domain of the HCMV UL16 includes an amino acid sequence having 80% or more identity to the amino acid sequence shown in SEQ ID NO. 84, preferably an amino acid sequence having an identity of 85%, 90%, 95%, 96%, 97%, 98%, or 99% or more, and more preferably an amino acid sequence having an identity of 98%, or 99% or more; and the NK target protein binding domain of the HCMV UL16 is shown as in SEQ ID NO. 84.

In some preferred embodiments, the NK target protein binding domain of the HCMV UL141 includes an amino acid sequence having 80% or more identity to the amino acid sequence shown in SEQ ID NO. 87, preferably an amino acid sequence having an identity of 85%, 90%, 95%, 96%, 97%, 98%, or 99% or more, and more preferably an amino acid sequence having an identity of 98%, or 99% or more; and the NK target protein binding domain of the HCMV UL141 is shown as in SEQ ID NO. 87.

In some preferred embodiments, the MICA and ULBP3 binding domain of HCMV UL142 includes an amino acid sequence having 80% or more identity to the amino acid sequence shown in SEQ ID NO. 90, preferably an amino acid sequence having an identity of 85%, 90%, 95%, 96%, 97%, 98%, or 99% or more, and more preferably an amino acid sequence having an identity of 98%, or 99% or more; and the MICA and ULBP3 binding domains of HCMV UL142 is shown as in SEQ ID NO. 90.

In some preferred embodiments, the MHC binding domain of the vaccinia virus protein CPXV203 includes an amino acid sequence having 80% or more identity to the amino acid sequence shown in SEQ ID NO. 99, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the vaccinia virus protein CPXV203 is shown as in SEQ ID NO. 99.

In some preferred embodiments, the MHC binding domain of the vaccinia virus protein CPXV203 includes an amino acid sequence having 80% or more identity to the amino acid sequence shown in SEQ ID NO. 102, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the vaccinia virus protein CPXV203 is shown as in SEQ ID NO. 102.

In some preferred embodiments, the MHC binding domain of the adenovirus E3-19K includes an amino acid sequence having 80% or more identity to the amino acid sequence shown in SEQ ID NO. 59, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the adenovirus E3-19K is shown as in SEQ ID NO. 59.

### 3. Protein degradation pathway member binding domain

The chimeric protein construct provided in the present disclosure may also contain a protein degradation pathway member binding domain. As described herein, the "protein degradation pathway member binding domain" refers to any portion that may bind directly or indirectly to a member of the protein degradation pathway.

The protein degradation pathway may be any pathway that mediates the degradation of proteins within a cell. Known protein degradation pathways include, but are not limited to, ubiquitination-proteasome pathway, endosome-lysosomal pathway, and autophagy degradation pathway.

Ubiquitinylation-proteasome pathway members include, for example, E1 ubiquitin-activating enzyme, E2 ubiquitin-binding enzyme, E3 ubiquitin ligase, and proteasome.

Examples of E1 ubiquitin-activating enzymes include, for example, UBA1, UBA2, UBA3, UBA5, UBA6, UBA7, ATG7, NAE1, and SAE1.

Examples of E2 ubiquitin-binding enzymes include, for example, hCdc34, Ubc-Uev1A, UBE2A, UBE2B, UBE2C, UBE2D1, UBE2D2, UBE2D3, UBE2D4, UBE2E1, UBE2E2, UBE2E3, UBE2F, UBE2G1, UBE2G2, UBE2H, UBE2I, UBE2J1, UBE2J2, UBE2K, UBE2L3, UBE2L6, UBE2M, UBE2N, UBE2O, UBE2Q1, UBE2Q2, UBE2R1 (CDC34), UBE2R2, UBE2S, UBE2T, UBE2U, UBE2V1, UBE2V2, UBE2W, UBE2Z, ATG3, BIRC6, and UFC1.

Examples of E3 ubiquitin ligases include, for example, von Hippel-Lindau (VHL), Cereblon (CRBN), inhibitor of apoptosis protein (IAP), Kelch-like ECH-associated protein 1 (Keap1), RNF4, RNF114, MDM2, LUBAC, FBW7, Met30, HECT, SKP2, beta TRCP1, HUWEI, TRAF6, SMURF1, and E6AP. In certain embodiments, examples of E3 ubiquitin ligase include, e.g., E3A, mdm2, Anaphase-promoting complex (APC), UBR5 (EDD1), SOCS/BC-box/eloBC/CUL5/RING, LNXp80, CBX4, CBLL1, HACE1, HECTD1, HECTD2, HECTD3, HECTD4, HECW1, HECW2, HERC1, HERC2, HERC3, HERC4, HERC5, HERC6, HUWE1, ITCH, NEDD4, NEDD4L, PPIL2, PRPF19, PIAS1, PIAS2, PIAS3, PIAS4, RANBP2, RNF4, RBX1, SMURF1, SMURF2, STUB1, TOPORS, TRIP12, UBE3A, UBE3B, UBE3C, UBE3D, UBE4A, UBE4B, UBOX5, UBR5, VHL, WWP1, WWP2, Parkin, and MKRN1.

Members of the endosome-lysosomal pathway include, for example, AP-1, AP-2, AP-3, endosomes, lysosomes, HOPS, ESCRT, GASP, BLOC-1, ESCRT, Retromer, ESCRT, sortingnexin, Dapper2, SNX4, Pincher, Rap1-PDZ-GEF1, clathrin, C3G/CrkL/Shp2/Gab2, etc.

Autophagy degradation pathway members include, for example, chaperonemediated autophagy (CMA), USP10, G3BP1, ULK1, ATG16L1, TRIM16, FBXO27VDAC, RHOT1, MFN1/2, BNIP3L, FUNDC1, BNIP3, AMBRA1, BCL2LI3, FKBP8, CHDH, DISC1, PHB2, Cardiolipin, SEC62, RTN3, PEX5, PEX14, ABCD3, NUFIP1, etc.

In some embodiments, the protein degradation pathway member binding domain is a VHL binding domain. In some embodiments, the VHL binding domain includes an amino acid sequence such as SEQ ID NO.119 (DRHDSGLDSM) or such as SEQ ID NO.120 (ALAPYIP).

In some embodiments, the protein degradation pathway member binding domain is a Keap1 binding domain. In some embodiments, the Keap1 binding domain includes an amino acid sequence such as SEQ ID NO. 121 (LDPETGEYL).

In some embodiments, the protein degradation pathway member binding domain is the E3 ubiquitin ligase binding domain, which includes an amino acid sequence such as SEQ ID NO. 119 (DRHDSGLDSM).

In some embodiments, the protein degradation pathway member binding domain is a CMA binding domain. In some embodiments, the CMA binding domain includes an amino acid sequence such as SEQ ID NO. 122 (KFERQ). In some embodiments, the CMA binding domain includes an amino acid sequence such as SEQ ID NO. 123 (KFERQKII,DQRFFE).

In some embodiments, the protein degradation pathway member binding domain is a proteasome binding domain. In some embodiments, the proteasome binding domain is selected from: yeast Rad23 (e.g., S. cerevisiae Rad23), human Rad23b (hHR23b) ubiquitin-like (UbL) domain, HPV E7, and proteasome binding domain of ankyrin protein. In some embodiments, the proteasome binding domain contains amino acids 1-77 of the yeast Rad23. In some embodiments, the proteasome binding domain contains amino acids 1-83 of the human Rad23.

The inventors of the present application found that compared with the basic TPD design (Target protein binding domain-transmembrane domain-ER retention domain (TBD-TMD-ERD)), a chimeric protein construct containing a protein degradation pathway member binding domain (ligand for E3 Ligase (E3L), ligand for E2 Ubiquitin-conjugating enzyme (E2L) or ligand for lysosome (LL)) (e.g., those described above) has significantly improved degradation of target proteins. The binding ability between the functional structure of viral ER-resident protein and ERAD reverse transporter complex is different, and the interaction between the natural structure and ER-resident E3ligase is also different, and it also competes with the normal degradation pathway of misfolded protein ERAD mechanism in the endoplasmic reticulum, such that the natural TMD-ERD domain has differences and limitations in the ubiquitination and degradation ability of the target protein. In order to further improve the degradation efficiency of the target protein, the introduction of the ubiquitin-proteasome system (UPS) ligand domain and/or autophagy-lysosomal pathway (ALP) ligand domain can further promote the ubiquitination of target proteins transported into the cytoplasm and proteasome degradation or transported to lysosomes, achieving efficient hydrolysis of target proteins.

In some embodiments, the target binding domain or ERAD mechanism protein binding domain of the chimeric protein construct is connected to the protein degradation pathway member binding domain via a hinge or linker. The hinge or linker can be one or more molecular sequence structures (Glyx Sery) n, wherein n can be numbers from 1 to 10, while x, y can be numbers from 0 to 10, respectively, but x, y cannot be 0 at the same time. In some embodiments, the hinge includes an amino acid sequence as indicated by SEQ ID NO. 172 (IgG4 hinge) or SEQ ID NO. 173 (((Gly4Ser)2).

### 4. TPD chimeric protein construct

In some embodiments, the chimeric protein construct provided by the present disclosure includes a target protein binding domain targeting TCR and adenovirus E3-19K, as well as one or more of the protein degradation pathway member binding domains described above. In some embodiments, the chimeric protein construct provided by the present disclosure includes a target protein binding domain targeting TCR and adenovirus E3-19K, as well as an E3 ubiquitin ligase binding domain. In some embodiments, the E3 ubiquitin ligase binding domain includes the amino acid sequence as shown in SEQ ID NO. 147 (DRHDSGLDSMGSGSGALAPYIP).

In some embodiments, the chimeric protein construct provided by the present disclosure includes a target protein binding domain targeting TCR, and a transmembrane domain or functional variants thereof and endoplasmic reticulum resident domains or functional variants thereof of a viral endoplasmic reticulum resident protein other than adenovirus E3-19K. The viral endoplasmic reticulum resident protein may be selected from at least one of the following group consisting of: HCMV glycoprotein US2, US11, US3, US10, US6, HSV ICP47, CPXV12, BHV UL49.5, EBV BNFL2a, HCMV UL16, UL141, UL142, HIV Nef, HIV Vpu, HHV-7 U21, HHV-8 KK3, HHV-8 KK5, MHV-68 MK3, HTLV-1 p12, and Cowpox Virus protein CPXV203.

In some embodiments, the chimeric protein construct provided by the present disclosure includes a target protein binding domain targeting the above target protein (e.g., TCR), and a transmembrane domain or functional variants thereof and endoplasmic reticulum resident domains or functional variants thereof of HCMV US2 and/or US11.

In some embodiments, the chimeric protein construct provided by the present disclosure includes a target protein binding domain targeting the above target protein (e.g., TCR), and a transmembrane domain or functional variants thereof and endoplasmic reticulum resident domains or functional variants thereof of HCMV US3.

In some embodiments, the chimeric protein construct provided by the present disclosure includes a target protein binding domain targeting the above target protein (e.g., TCR), and a transmembrane domain or functional variants thereof and endoplasmic reticulum resident domains or functional variants thereof of HCMV US10.

In some embodiments, the chimeric protein construct provided by the present disclosure includes a target protein binding domain targeting the above target protein (e.g., TCR), and a transmembrane domain or functional variants thereof and endoplasmic reticulum resident domains or functional variants thereof of HCMV US6, HSV ICP47, CPXV012, EBV BNFL2a, and/or BHV UL49.5.

In some embodiments, the chimeric protein construct provided by the present disclosure includes a target protein binding domain targeting the above target protein (e.g., TCR), and a transmembrane domain or functional variants thereof and endoplasmic reticulum resident domains or functional variants thereof of HHV-7 US21.

In some embodiments, the chimeric protein construct provided by the present disclosure includes a target protein binding domain targeting the above target protein (e.g., TCR), and a transmembrane domain or functional variants thereof and endoplasmic reticulum resident domains or functional variants thereof of HCMV US16, UL 141, and/or UL142.

In some embodiments, the chimeric protein construct provided by the present disclosure includes a target protein binding domain targeting the above target protein (e.g., TCR), and a transmembrane domain or functional variants thereof and endoplasmic reticulum resident domains or functional variants thereof of HIV Nef, HIV Vpu, HHV-7 U21, HHV-8 KK3, HHV-8 KK5, MHV-68 MK3, and/or HTLV-1 p12.

In some embodiments, the chimeric protein construct provided by the present disclosure includes a target protein binding domain targeting the above target protein (e.g., TCR), and a transmembrane domain or functional variants thereof and endoplasmic reticulum resident domains or functional variants thereof of Cowpox Virus protein CPXV203.

In some embodiments, the chimeric protein construct provided by the present disclosure includes a target protein binding domain not targeting TCR and adenovirus E3-19K. In some embodiments, the chimeric protein construct provided by the present disclosure includes a target protein binding domain targeting CD123 and adenovirus E3-19K.

In some embodiments, the above chimeric protein construct further includes one or more of the protein degradation pathway member binding domains described above.

### 5. Co-expression moiety

In some embodiments, the chimeric protein construct provided by the present disclosure and at least one co-expression moiety are co-expressed. Any way that applies to common expression may be used. For example, the chimeric protein construct (or a portion thereof) and at least one co-expression moiety may be connected by a cleavable linker, and in a case of the linker being cleaved, the chimeric protein construct and the co-expression moiety may be co-expressed.

In some embodiments, the chimeric protein construct provided by the present disclosure is linked, for example, by a self-cleavable linkage, with at least one co-expression moiety. In some embodiments, the self-cleavable linkage is a cleavable peptide, for example, a T2A peptide, GSG-T2A peptide, E2A peptide, GSG-E2A peptide, F2A peptide, GSG-F2A peptide, P2A peptide, or GSG-P2A peptide.

In some preferred embodiments, the T2A includes an amino acid sequence having 80% or more identity to the amino acid sequence shown in SEQ ID NO. 108, preferably an amino acid sequence having an identity of 85%, 90%, 95%, 96%, 97%, 98%, or 99% or more, and more preferably an amino acid sequence having an identity of 98%, or 99% or more; and the amino acid sequence of the T2A is shown as in SEQ ID NO. 108.

In some preferred embodiments, the amino acid sequence of the GSG-T2A peptide is shown in SEQ ID NO. 109.

In some preferred embodiments, the P2A includes an amino acid sequence having 80% or more identity to the amino acid sequence shown in SEQ ID NO. 110, preferably an amino acid sequence having an identity of 85%, 90%, 95%, 96%, 97%, 98%, or 99% or more, and more preferably an amino acid sequence having an identity of 98%, or 99% or more; and the amino acid sequence of the P2Ais shown in SEQ ID NO. 110.

In some preferred embodiments, the amino acid sequence of the GSG-P2A peptide is shown in SEQ ID NO. 111.

In some preferred embodiments, the E2A includes an amino acid sequence having 80% or more identity to the amino acid sequence shown in SEQ ID NO. 112, preferably an amino acid sequence having an identity of 85%, 90%, 95%, 96%, 97%, 98%, or 99% or more, and more preferably an amino acid sequence having an identity of 98%, or 99% or more; and the amino acid sequence of the E2Ais shown in SEQ ID NO. 112.

In some preferred embodiments, the amino acid sequence of the GSG-E2A peptide is shown in SEQ ID NO. 113.

In some preferred embodiments, the F2A includes an amino acid sequence having 80% or more identity to the amino acid sequence shown in SEQ ID NO. 114, preferably an amino acid sequence having an identity of 85%, 90%, 95%, 96%, 97%, 98%, or 99% or more, and more preferably an amino acid sequence having an identity of 98%, or 99% or more; and the amino acid sequence of the F2A is shown as in SEQ ID NO. 114.

In some preferred embodiments, the amino acid sequence of the GSG-F2A peptide is shown in SEQ ID NO. 115.

The co-expression moiety provided by the present application may be any protein or polypeptide having a biological function. Depending on a desired biological function, an appropriate co-expression moiety may be selected. For example, to reduce the immunogenicity or antigen presentation of cells, a protein capable of degrading or reducing MHC class I or II molecules may be selected as a co-expression moiety. As another example, in order for cells to recognize a target protein, a binding domain that recognizes or binds to the target protein (e.g., chemokine receptor, or chimeric antigen receptor CAR) may be selected as a co-expression moiety. As another example, in order to increase immunostimulating activity, an immunostimulatory molecule may be selected as a co-expression moiety.

In some preferred embodiments, examples of the co-expression moieties include, but are not limited to, intact viral ER-resident glycoproteins (e.g., HCMV US2, US3, US11, US10, adenovirus E3-Kl 9, HCMV US6, HSV ICP47), chimeric antigen receptor (CAR), functional T cell receptor (TCR), chemokine receptors (e.g., CCR4, CCR5, CCR6, CCR7, CCR9, CCR2b, CXCR1, CXCR2, and CXCR4), NK-cell-activating receptors (e.g., NKG2D, NKG2C, NKG2E, NKG2F, NKG2H, CD94, KIR2DL4, KIR2DS1, KIR2DS2, KIR2DS4, KIR3DS1, native cytotoxic receptors, TRAIL, DNAM-1, CD16a, 2B4, NTB-A, CRACC, and NKp80), CNK signal transduction components, cytokines, CD7, immunostimulatory molecules (e.g., TNF-a, IFN-β, IFN-γ, IL-1, IL-2, IL-4, IL-6, IL-8, IL-10, IL-12, IL-18, and one or more of granulocyte macrophage colony-stimulating factors), etc.

### i) Intact viral ER-resident glycoprotein

In some embodiments, the co-expression moiety is an intact viral ER-resident glycoprotein, including, but not limited to, HCMV US2, US3, US11, US10, adenovirus E3-Kl 9, HCMV US6, and HSV ICP47.

In some preferred embodiments, the full-length sequence of the HCMV glycoprotein US2 includes an amino acid sequence having 80% or more identity to the amino acid sequence shown in SEQ ID NO. 46, preferably an amino acid sequence having an identity of 85%, 90%, 95%, 96%, 97%, 98%, or 99% or more, and more preferably an amino acid sequence having an identity of 98%, or 99% or more, preferably, having activity of degrading MHC class I or MHC class II molecules; and the amino acid sequence of the full-length sequence of the HCMV glycoprotein US2 is shown as in SEQ ID NO. 46.

In some preferred embodiments, the full-length sequence of the US2 includes an amino acid sequence having 80% or more identity to the amino acid sequence shown in SEQ ID NO. 74, preferably an amino acid sequence having an identity of 85%, 90%, 95%, 96%, 97%, 98%, or 99% or more, and more preferably an amino acid sequence having an identity of 98%, or 99% or more, preferably, having activity of degrading MHC class I or MHC class II molecules; and the amino acid sequence of the full-length sequence of the US2 is shown as in SEQ ID NO. 74.

In some preferred embodiments, the full-length sequence of the HCMV glycoprotein US3 includes an amino acid sequence having 80% or more identity to the amino acid sequence shown in SEQ ID NO. 49, preferably an amino acid sequence having an identity of 85%, 90%, 95%, 96%, 97%, 98%, or 99% or more, and more preferably an amino acid sequence having an identity of 98%, or 99% or more, preferably, having activity of degrading MHC class I or MHC class II molecules; and the amino acid sequence of the full-length sequence of the HCMV glycoprotein US3 is shown as in SEQ ID NO. 49.

In some preferred embodiments, the full-length sequence of the US3 includes an amino acid sequence having 80% or more identity to the amino acid sequence shown in SEQ ID NO. 77, preferably an amino acid sequence having an identity of 85%, 90%, 95%, 96%, 97%, 98%, or 99% or more, and more preferably an amino acid sequence having an identity of 98%, or 99% or more, preferably, having activity of degrading MHC class I or MHC class II molecules; and the amino acid sequence of the full-length sequence of the US3 is shown as in SEQ ID NO. 77.

In some preferred embodiments, the full-length sequence of the HCMV glycoprotein US 11 includes an amino acid sequence having 80% or more identity to the amino acid sequence shown in SEQ ID NO. 52, preferably an amino acid sequence having an identity of 85%, 90%, 95%, 96%, 97%, 98%, or 99% or more, and more preferably an amino acid sequence having an identity of 98%, or 99% or more, preferably, having activity of degrading MHC class I or MHC class II molecules; and the amino acid sequence of the full-length sequence of the HCMV glycoprotein US11 is shown as in SEQ ID NO. 52.

In some preferred embodiments, the full-length sequence of the US 11 includes an amino acid sequence having 80% or more identity to the amino acid sequence shown in SEQ ID NO. 80, preferably an amino acid sequence having an identity of 85%, 90%, 95%, 96%, 97%, 98%, or 99% or more, and more preferably an amino acid sequence having an identity of 98%, or 99% or more, preferably, having activity of degrading MHC class I or MHC class II molecules; and the amino acid sequence of the full-length sequence of the US11 is shown as in SEQ ID NO. 80.

In some preferred embodiments, the full-length sequence of the HCMV glycoprotein US 10 includes an amino acid sequence having 80% or more identity to the amino acid sequence shown in SEQ ID NO. 55, preferably an amino acid sequence having an identity of 85%, 90%, 95%, 96%, 97%, 98%, or 99% or more, and more preferably an amino acid sequence having an identity of 98%, or 99% or more, preferably, having activity of degrading MHC class I or MHC class II molecules; and the amino acid sequence of the full-length sequence of the HCMV glycoprotein US10 is shown as in SEQ ID NO. 55.

In some preferred embodiments, the full-length sequence of the adenovirus E3-19K includes an amino acid sequence having 80% or more identity to the amino acid sequence shown in SEQ ID NO. 58, preferably an amino acid sequence having an identity of 85%, 90%, 95%, 96%, 97%, 98%, or 99% or more, and more preferably an amino acid sequence having an identity of 98%, or 99% or more, preferably, having activity of degrading MHC class I or MHC class II molecules; and the amino acid sequence of the full-length sequence of the adenovirus E3-19K is shown as in SEQ ID NO. 58.

In some preferred embodiments, the full-length sequence of the HCMV US6 includes an amino acid sequence having 80% or more identity to the amino acid sequence shown in SEQ ID NO. 64, preferably an amino acid sequence having an identity of 85%, 90%, 95%, 96%, 97%, 98%, or 99% or more, and more preferably an amino acid sequence having an identity of 98%, or 99% or more, preferable, having activity of degrading MHC class I or MHC class II molecules; and the full-length sequence of the HCMV US6 is shown as in SEQ ID NO. 64.

In some preferred embodiments, the full-length sequence of the HSV ICP47 includes an amino acid sequence having 80% or more identity to the amino acid sequence shown in SEQ ID NO. 66, preferably an amino acid sequence having an identity of 85%, 90%, 95%, 96%, 97%, 98%, or 99% or more, and more preferably an amino acid sequence having an identity of 98%, or 99% or more, preferable, having activity of degrading MHC class I or MHC class II molecules in; and the full-length sequence of the HSV ICP47 is shown as in SEQ ID NO. 66.

### ii) Chimeric antigen receptor (CAR)

In certain embodiments, the co-expression moiety is a chimeric antigen receptor (CAR). The CAR includes a target protein-binding domain (e.g., an extracellular recognition domain targeting a tumor), a transmembrane domain, and an immune receptor activation signal transduction domain (ITAM) (also referred to as an "intracellular signal transduction domain"). In certain embodiments, the CAR may further include a co-stimulatory domain. In certain embodiments, a hinge or linker is included among the extracellular recognition domain targeting a tumor, transmembrane domain, and/or intracellular signal transduction domain.

In some embodiments, the extracellular recognition domain targeting a tumor is selected from a tumor antigen-specific binding domain, a tumor microenvironment target antigen binding domain, and/or a chemokine receptor targeting tumor microenvironment.

In some preferred embodiments, the extracellular recognition domain targeting a tumor is selected from an antibody capable of targeted recognizing a tumor-associated antigen or its functional fragment, TCR or combination thereof. The functional fragments of the antibody are selected from the group consisting of Fd, Fv, Fab, Fab', F(ab') 2, Fv (scFv), singlechain antibody (scFv) or nanobody, diabody, triabody and terabody.

In some preferred embodiments, the transmembrane domain of the CAR described herein may be derived from any membrane-binding protein or transmembrane protein, including but not limited to BAFFR, BLAME (SLAMF8), CD2, CD3ε, CD4, CD5, CD8, CD9, CD11a (CD18, ITGAL, LFA-l), CD11b, CD11c, CD11d, CD16, CD19, CD22, CD27, CD28, CD29, CD33, CD37, CD40, CD45, CD49a, CD49d, CD49f, CD64, CD80, CD84, CD86, CD96(Tactile), CD100(SEMA4D), CD103, CD134, CD137(4-1BB), CD150(IPO-3, SLAMF1, SLAM), CD154, CD160(BY55), CD162(SELPLG), CD226(DNAM1), CD229(Ly9), CD244(2B4, SLAMF4), CD278(ICOS), CEACAM1, CRT AM, GITR, HYEM(LIGHTR), IA4, IL2Rβ, IL2Rγ, IL7R a, ITGA1, ITGA4, ITGA6, ITGAD, ITGAE, ITGAM, ITGAX, ITGB1, ITGB2, ITGB7, KIR, LTBR, OX40, NKG2C, NKG2D, NKp30, NKp44, NKp46, NKp80 (KLRF1), PAG/Cbp, PSGL1, SLAMF6 (NTB-A, Ly108), SLAMF7, α, β or ζ chains of T cell receptors, TNFR2, VLA1, and VLA-6.

In some preferred embodiments, the transmembrane domain of the CAR is selected from the NK-cell-activating receptor transmembrane domain, DAP10 transmembrane domain, DAP12 transmembrane domain, CD8 transmembrane domain, CD28 transmembrane domain, CD4 transmembrane domain, 4-1BB transmembrane domain, OX40 transmembrane domain, ICOS transmembrane domain, CTLA-4 transmembrane domain, PD-1 transmembrane domain, LAG-3 transmembrane domain, 2B4 transmembrane domains, and BTLA transmembrane domain, as well as combinations thereof.

In some preferred embodiments, the transmembrane domain of the CAR is selected from CD8 transmembrane domain, α and/or β chain transmembrane domain of T cell receptor, CD28 transmembrane domain, CD3ε transmembrane domain, CD45 transmembrane domain, CD4 transmembrane domain, CD5 transmembrane domain, CD8 transmembrane domain, CD9 transmembrane domain, CD16 transmembrane domain, CD22 transmembrane domain, CD33 transmembrane domain, CD37 transmembrane domain, CD64 transmembrane domain, CD80 transmembrane domain, CD86 transmembrane domain, CD134 transmembrane domain, CD137 transmembrane domain, CD154 transmembrane domain, GITR transmembrane domain, and combinations thereof.

In some preferred embodiments, the immune receptor activation signal transduction domain (ITAM) derives from the intracellular activation signal transduction domain of the immune receptor; preferably, the immune receptor is selected from TCRζ, CD2, CD3γ, CD3δ, CD3ε, CD3ζ, CD5, CD22, FcRy, CD66d, FcαRI, FcyRI, FcyRII, FcyRIII, Dectin-1, CLEC-1, CD72, CD79A, CD79B; preferably, the immune receptor activation signal transdution domain (ITAM) is fused with the NK cell signaling adaptor or its functional variant; and preferably, the immune receptor is CD3ζ.

In some preferred embodiments, the immune receptor activation signal transduction domain (ITAM) is derived from CD3ζ, common FcRγ (FCER1G), FcyRIIa, FcRβ, CD3γ, CD3δ, CD3ε, CD5, CD22, CD79a, CD79b, CD278 ("ICOS"), FcεRI CD66d, DAP10 and DAP12 and other intracellular signal transduction domains.

In some preferred embodiments, the intracellular signal transduction domain of the CAR includes an intracellular signal transduction domain of the NK-cell-activating receptor and/or a co-stimulation signal transduction domain.

In some preferred embodiments, the T cell co-stimulation signal transduction domain is derived from the intracellular signal transduction domain of the co-stimulatory molecules; preferably, the co-stimulatory molecules are selected from the group consisting of MHC class I molecules, TNF receptor proteins, immunoglobulin-like proteins, cytokine receptors, integrin proteins, lymphocyte activation signaling molecules (SLAM proteins), activated NK cell receptors, BTLA, Toll ligand receptors, OX40, CD2, CD7, CD7, CD16, CD27, CD28, CD30, CD40, CD38, CD35, CD79A, CD79B, CDS, ICAM-1, LFA-1, (CD11a/CD18), 4-1BB(CD137), B7-H3, CDS, ICAM-1, ICOS(CD278), GITR, BAFFR, LIGHT, HVEM(LIGHTR), KIRDS2, SLAMF7, NKp80(KLRF1), NKp44, NKp30, NKp46, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, NCR, DAP10, DAP12, TNFR2, TRANCE/RANKL, DNAM1(CD226), SLAMF4(CD244, 2B4), CD84, CD96(Tactile), CEACAM1, CRTAM, Ly9(CD229), CD160(BY55), PSGL1, CD100SEMA4D), CD69, SLAMF6(NTB-A, Ly108), SLAM (SLAMF1, CD150, IPO-3), BLAME (SLAMF8), SELPLG (CD162), LTBR, LAT, GADS, SLP-76, PAG/Cbp, CD19a, CD83-specific binding ligands, CARD11, FcRa, FcRp, FcRy, Fyn, HVEM, ICOS, Lck, LAG3, LAT, LRP, NOTCH1, Wnt, OX40, ROR2, Ryk, SLAMF1, Slp76, pTa, TCRa, TCRp, TRIM, ZAP70, and PTCH2. More preferably, the co-stimulation signal transduction domain may be selected from the NKG2D intracellular signal transduction domain, DAP10 intracellular signal transduction domain, DAP12 intracellular signal transduction domain, NCR intracellular signal transduction domain, CD28 intracellular signal transduction domain, 4-1BB intracellular signal transduction domain, OX40 intracellular signal transduction domain, and ICOS intracellular signal transduction domain.

In some embodiments, the hinge and/or transmembrane domain of CAR described herein provides cell surface presentation of the extracellular domain of CAR. The hinge of the CAR described herein may be derived from any membrane-binding protein or transmembrane protein, including but not limited to BAFFR, BLAME (SLAMF8), CD2, CD3ε, CD4, CD5, CD8, CD9, CD11a (CD18, ITGAL, LFA-l), CD11b, CD11c, CD11d, CD16, CD19, CD22, CD27, CD28, CD29, CD33, CD37, CD40, CD45, CD49a, CD49d, CD49f, CD64, CD80, CD84, CD86, CD96(Tactile), CD100(SEMA4D), CD103, CD134, CD137(4-1BB), CD150(IPO-3, SLAMF1, SLAM), CD154, CD160(BY55), CD162(SELPLG), CD226(DNAM1), CD229(Ly9), CD244(2B4, SLAMF4), CD278(ICOS), CEACAM1, CRT AM, GITR, HYEM(LIGHTR), IA4, IL2Rβ, IL2Rγ, II,7Ra a, ITGA1, ITGA4, ITGA6, ITGAD, ITGAE, ITGAM, ITGAX, ITGB1, ITGB2, ITGB7, KIR, LTBR, OX40, NKG2C, NKG2D, NKp30, NKp44, NKp46, NKp80 (KLRF1), PAG/Cbp, PSGL1, SLAMF6 (NTB-A, Ly108), SLAMF7, α, β or ζ chains of T cell receptors, TNFR2, VLA1, and VLA-6. In some embodiments, the hinge of the CAR described herein includes a hinge region of CD8α, a hinge region of human immunoglobulin (Ig), or a glycine-serine-rich sequence.

In some preferred embodiments, the linker is a flexible linker; preferably, the flexible linker includes the amino acid sequence indicated as (Gly(x)Ser(y)n, wherein n is an integer from 1 to 10, and x and y are independent integers from 0 to 10, provided that x and y are not both 0; and more preferably, the linker includes an amino acid sequence indicated in SEQ ID NO. 104 or an amino acid sequence indicated in SEQ ID NO. 105.

In some preferred embodiments, the hinge is an IgG1 hinge or an IgG4 hinge.

In some preferred embodiments, the IgG1 hinge includes an amino acid sequence having 80% or more identity to the amino acid sequence shown in SEQ ID NO. 106, preferably an amino acid sequence having an identity of 85%, 90%, 95%, 96%, 97%, 98%, or 99% or more, and more preferably an amino acid sequence having an identity of 98%, or 99% or more; and the amino acid sequence of the IgG1 hinge is shown as in SEQ ID NO. 106.

In some preferred embodiments, the IgG4 hinge includes an amino acid sequence having 80% or more identity to the amino acid sequence shown in SEQ ID NO. 107, preferably an amino acid sequence having an identity of 85%, 90%, 95%, 96%, 97%, 98%, or 99% or more, and more preferably an amino acid sequence having an identity of 98%, or 99% or more; and the amino acid sequence of the IgG4 hinge is shown as in SEQ ID NO. 107.

In some preferred embodiments, a cleavage peptide is comprised among the NK activation receptor component, CNK signal transduction component, and/or UT component, for example, T2A peptide, GSG-T2A peptide, E2A peptide, GSG-E2A peptide, F2A peptide, GSG-F2A peptide, P2A peptide, or GSG-P2A peptide.

In some preferred embodiments, the T2A includes an amino acid sequence having 80% or more identity to the amino acid sequence shown in SEQ ID NO. 108, preferably an amino acid sequence having an identity of 85%, 90%, 95%, 96%, 97%, 98%, or 99% or more, and more preferably an amino acid sequence having an identity of 98%, or 99% or more; and the amino acid sequence of the T2A is shown as in SEQ ID NO. 108.

In some preferred embodiments, the amino acid sequence of the GSG-T2A peptide is shown in SEQ ID NO. 109.

In some preferred embodiments, the P2A includes an amino acid sequence having 80% or more identity to the amino acid sequence shown in SEQ ID NO. 110, preferably an amino acid sequence having an identity of 85%, 90%, 95%, 96%, 97%, 98%, or 99% or more, and more preferably an amino acid sequence having an identity of 98%, or 99% or more; and the amino acid sequence of the P2Ais shown as in SEQ ID NO. 110.

In some preferred embodiments, the amino acid sequence of the GSG-P2A peptide is shown in SEQ ID NO. 111.

In some preferred embodiments, the E2A includes an amino acid sequence having 80% or more identity to the amino acid sequence shown in SEQ ID NO. 112, preferably an amino acid sequence having an identity of 85%, 90%, 95%, 96%, 97%, 98%, or 99% or more, and more preferably an amino acid sequence having an identity of 98%, or 99% or more; and the amino acid sequence of the E2A is shown as in SEQ ID NO. 112.

In some preferred embodiments, the amino acid sequence of the GSG-E2A peptide is shown in SEQ ID NO. 113.

In some preferred embodiments, the F2A includes an amino acid sequence having 80% or more identity to the amino acid sequence shown in SEQ ID NO. 114, preferably an amino acid sequence having an identity of 85%, 90%, 95%, 96%, 97%, 98%, or 99% or more, and more preferably an amino acid sequence having an identity of 98%, or 99% or more; and the amino acid sequence of the F2A is shown as in SEQ ID NO. 114.

In some preferred embodiments, the amino acid sequence of the GSG-F2A peptide is shown in SEQ ID NO. 115.

### iii) CNK receptor

In certain embodiments, the co-expression moiety is a CNK receptor. The CNK receptor includes a chimeric NK-activating receptor component and a chimeric NK signal transduction component (also referred to as a "CNK signal transduction component"). The chimeric NK-activating receptor component may at least include a NK cell activation receptor or a functional variant thereof.

### NK-cell-activating receptors

In some embodiments, the NK-cell-activating receptor includes: (a) an extracellular domain (ED) or a functional variant of the NK-cell-activation receptor, (b) a transmembrane domain (TMD) or a functional variant of the NK cell activation receptor, and (c) an intracellular domain (ICD) or a functional variant of the NK cell activation receptor; and wherein, optionally, a hinge or linker is included among the extracellular domain or functional variant of the NK-cell-activating receptor, the transmembrane domain or functional variant of NK-cell-activating receptor, and/or the intracellular domain or functional variant of the NK-cell-activating receptor. A detailed description of NK-cell-activating receptors and CNK signal transduction components can be found at, e.g. US 2020/0308248A1, the entirety of which is incorporated herein by reference.

The NK-cell-activating receptor is selected from NKG2D, NKG2C, NKG2E, NKG2F, NKG2H, CD94, KIR2DL4, KIR2DS1, KIR2DS2, KIR2DS4, KIR3DS1, natural cytotoxicity receptor (NCR), TRAIL, DNAM-1, signaling lymphocytic activation molecule (SLAM) family molecules 2B4 (also known as CD244), DNAX attachment molecule 1 (DNAM-1, also known as CD226), CD16a, 2B4, NTB-A, CRACC (CS1) and NKp80, wherein the natural cytotoxic receptor includes NKp46 (a.k.a. NCR1 or CD335), NKp44 (also known as NCR2 or CD336) and NKp30 (also known as NCR3 or CD337). In some embodiments, the natural cytotoxic receptor is selected from NKp46, NKp44 and NKp30.

In some preferred embodiments, the NK-cell-activating receptor is a mammalian derived NK-cell-activating receptor; preferably, the mammal is selected from human, primate, rat, horse, cattle, sheep, goat, cat, pig, dog, llama, alpacas, elephant, squirrel, guinea pig.

In some preferred embodiments, the NK-cell-activating receptor is a recombinant NK-cell-activating receptor comprising different original NK-cell-activating domains.

In some preferred embodiments, the NK-cell-activating receptor is a human derived NK-cell-activating receptor; and preferably, the NK-cell-activating receptor is a recombinant NK-cell-activating receptor comprising different and human derived NK-cell-activating domains.

In some preferred embodiments, the NK-cell-activating receptor is a mouse or rat derived NK-cell-activating receptor; and preferably, the NK-cell-activating receptor is a recombinant NK-cell-activating receptor comprising different and mouse or rat derived NK-cell-activating domains.

In some preferred embodiments, the NK-cell-activating receptor is a recombinant NK-cell-activating receptor comprising human derived, and mouse or rat derived NK-cell-activating domains.

In some preferred embodiments, the extracellular domain of the NK-cell-activating receptor is the extracellular domain of the human or mouse or rat NK-cell-activating receptor.

In some preferred embodiments, the transmembrane domain of the NK-cell-activating receptor is the transmembrane domain of the human or mouse or rat NK-cell-activating receptor.

In some preferred embodiments, the intracellular domain of the NK-cell-activating receptor is the intracellular domain of the human or mouse or rat NK-cell-activating receptor.

In some preferred embodiments, the NK-cell-activating receptor includes the extracellular domain of human NK-cell-activating receptor, transmembrane domain of mouse or rat NK-cell-activating receptor, and intracellular domain of human NK-cell-activating receptor.

In some preferred embodiments, the NK-cell-activating functional variant is selected from the NK-cell-activating receptor mutant, wild-type fusion protein, or wild-type and mutant fusion protein.

In some preferred embodiments, the extracellular domain of human NKG2D includes an amino acid sequence having 80% or more identity to the amino acid sequence shown in SEQ ID NO. 1, preferably an amino acid sequence having an identity of 85%, 90%, 95%, 96%, 97%, 98%, or 99% or more, and more preferably an amino acid sequence having an identity of 98%, or 99% or more; and the amino acid sequence of the extracellular domain of human NKG2D is shown as in SEQ ID NO. 1.

In some preferred embodiments, the full-length sequence of the human NKG2D includes an amino acid sequence having 80% or more identity to the amino acid sequence shown in SEQ ID NO. 2, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKG2D is shown as in SEQ ID NO. 2.

In some preferred embodiments, the extracellular domain of mouse NKG2D includes an amino acid sequence having 80% or more identity to the amino acid sequence shown in SEQ ID NO. 3, preferably an amino acid sequence having an identity of 85%, 90%, 95%, 96%, 97%, 98%, or 99% or more, and more preferably an amino acid sequence having an identity of 98%, or 99% or more; and the amino acid sequence of the extracellular domain of mouse NKG2D is shown as in SEQ ID NO. 3.

In some preferred embodiments, the full-length sequence of the mouse NKG2D includes an amino acid sequence having 80% or more identity to the amino acid sequence shown in SEQ ID NO. 4, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the mouse NKG2D is shown as in SEQ ID NO. 4.

In some preferred embodiments, the full-length sequence of the human-mouse recombinant NKG2D includes an amino acid sequence having 80% or more identity to the amino acid sequence shown in SEQ ID NO. 5, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human-mouse recombinant NKG2D is shown as in SEQ ID NO. 5.

In some preferred embodiments, the full-length sequence of the human NKG2C includes an amino acid sequence having 80% or more identity to the amino acid sequence shown in SEQ ID NO. 6, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKG2C is shown as in SEQ ID NO. 6.

In some preferred embodiments, the full-length sequence of the human NKG2E includes an amino acid sequence having 80% or more identity to the amino acid sequence shown in SEQ ID NO. 7, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKG2E is shown as in SEQ ID NO. 7.

In some preferred embodiments, the full-length sequence of the human NKG2F includes an amino acid sequence having 80% or more identity to the amino acid sequence shown in SEQ ID NO. 8, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKG2F is shown as in SEQ ID NO. 8.

In some preferred embodiments, the full-length sequence of the human CD94 includes an amino acid sequence having 80% or more identity to the amino acid sequence shown in SEQ ID NO. 9, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human CD94 is shown as in SEQ ID NO. 9.

In some preferred embodiments, the full-length sequence of the human KIR2DL4 includes an amino acid sequence having 80% or more identity to the amino acid sequence shown in SEQ ID NO. 10, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human KIR2DL4 is shown as in SEQ ID NO. 10.

In some preferred embodiments, the full-length sequence of the human KIR2DS1 includes an amino acid sequence having 80% or more identity to the amino acid sequence shown in SEQ ID NO. 11, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human KIR2DS1 is shown as in SEQ ID NO. 11.

In some preferred embodiments, the full-length sequence of the human KIR2DS2 includes an amino acid sequence having 80% or more identity to the amino acid sequence shown in SEQ ID NO. 12, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human KIR2DS2 is shown as in SEQ ID NO. 12.

In some preferred embodiments, the full-length sequence of the human KIR2DS4 includes an amino acid sequence having 80% or more identity to the amino acid sequence shown in SEQ ID NO. 13, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human KIR2DS4 is shown as in SEQ ID NO. 13.

In some preferred embodiments, the full-length sequence of the human KIR3DS1 includes an amino acid sequence having 80% or more identity to the amino acid sequence shown in SEQ ID NO. 14, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human KIR3DS1 is shown as in SEQ ID NO. 14.

In some preferred embodiments, the full-length sequence of the human NKp46 includes an amino acid sequence having 80% or more identity to the amino acid sequence shown in SEQ ID NO. 15, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKp46 is shown as in SEQ ID NO. 15.

In some preferred embodiments, the full-length sequence of the human NKp44 includes an amino acid sequence having 80% or more identity to the amino acid sequence shown in SEQ ID NO. 16, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKp44 is shown as in SEQ ID NO. 16.

In some preferred embodiments, the full-length sequence of the human NKp30 includes an amino acid sequence having 80% or more identity to the amino acid sequence shown in SEQ ID NO. 17, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKp30 is shown as in SEQ ID NO. 17.

In some preferred embodiments, the full-length sequence of the human DNAM1 includes an amino acid sequence having 80% or more identity to the amino acid sequence shown in SEQ ID NO. 18, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DNAM1 is shown as in SEQ ID NO. 18.

In some preferred embodiments, the full-length sequence of the human TRAIL includes an amino acid sequence having 80% or more identity to the amino acid sequence shown in SEQ ID NO. 19, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human TRAIL is shown as in SEQ ID NO. 19.

In some preferred embodiments, the full-length sequence of the human CD16a includes an amino acid sequence having 80% or more identity to the amino acid sequence shown in SEQ ID NO. 20, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human CD16a is shown as in SEQ ID NO. 20.

In some preferred embodiments, the full-length sequence of the human 2B4 includes an amino acid sequence having 80% or more identity to the amino acid sequence shown in SEQ ID NO. 21, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human 2B4 is shown as in SEQ ID NO. 21.

In some preferred embodiments, the full-length sequence of the human NTB-A includes an amino acid sequence having 80% or more identity to the amino acid sequence shown in SEQ ID NO. 22, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NTB-A is shown as in SEQ ID NO. 22.

In some preferred embodiments, the full-length sequence of the human CRACC includes an amino acid sequence having 80% or more identity to the amino acid sequence shown in SEQ ID NO. 23, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human CRACC is shown as in SEQ ID NO. 23.

In some preferred embodiments, the full-length sequence of the human NKp80 includes an amino acid sequence having 80% or more identity to the amino acid sequence shown in SEQ ID NO. 24, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKp80 is shown as in SEQ ID NO. 24.

### CNK signal transduction component

In some embodiments, the CNK signal transduction component includes at least (i) NK cell signal adaptor (e.g., DAP10 or DAP12) or a functional variant thereof. In some embodiments, the NK cell signal adaptor includes: (a) an extracellular domain (ED) or a functional variant of the NK cell signal adaptor, (b) a transmembrane domain (TMD) or a functional variant of the NK cell signal adaptor, and (c) an intracellular domain (ICD) or a functional variant of the NK cell signal adaptor; and wherein, optionally, a hinge or linker is included among the extracellular domain or a functional variant of the NK cell signal adaptor, the transmembrane domain or a functional variant of NK cell signal adaptor, and/or the intracellular domain or a functional variant of the NK cell signal adaptor.

In some preferred embodiments, the NK cell signal adaptor is a mammalian derived NK cell signal adaptor; preferably, the mammal is selected from human, primate, rat, horse, cattle, sheep, goat, cat, pig, dog, llama, alpacas, elephant, squirrel, guinea pig.

In some preferred embodiments, the NK cell signal adaptor is a recombinant NK cell signal converter comprising different original NK cell signal adaptor domains.

In some preferred embodiments, the NK cell signal adaptor is a human derived NK cell signal adaptor; preferably, the NK cell signal adaptor is a recombinant NK cell signal adaptor comprising different and human derived NK cell signal adaptor domains.

In some preferred embodiments, the NK cell signal adaptor is a mouse or rat derived NK cell signal adaptor; preferably, the NK cell signal adaptor is a recombinant NK cell signal adaptor comprising different and mouse or rat derived NK cell signal adaptor domains.

In some preferred embodiments, the NK cell signal adaptor is a recombinant NK cell signal adaptor comprising human-derived, and mouse/rat-derived NK cell signal adaptor domains.

In some preferred embodiments, the extracellular domain of the NK cell signal adaptor is the extracellular domain of the human or mouse or rat NK cell signal adaptor.

In some preferred embodiments, the transmembrane domain of the NK cell signal adaptor is the transmembrane domain of the human or mouse or rat NK cell signal adaptor.

In some preferred embodiments, the intracellular domain of the NK cell signal adaptor is the intracellular domain of the human or mouse or rat NK cell signal adaptor.

The NK cell signal adaptor in the CNK signal transduction component is DAP10 or DAP12.

In some preferred embodiments, the functional variant of CNK cell signal adaptor is selected from a mutant of DAP10 or DAP12, or a fusion protein of DAP10 and DAP 12, or a wild-type DAP10 or DAP12 fusion protein with a mutant type DAP10 or DAP12.

In some preferred embodiments, the CNK signal transduction component further includes (ii) immune receptor activation signal transduction domain (ITAM) and/or (iii) T cell co-stimulation signal transduction domain.

In some preferred embodiments, a hinge or linker is included among the NK cell signal adaptor or a functional variant thereof, immune receptor activation signal transduction domain (ITAM), and/or T cell co-stimulation signal transduction domain; preferably, the NK cell signal adaptor or a functional variant thereof is fused with the immune receptor activation signal transduction domain (ITAM).

In some preferred embodiments, the immune receptor activation signal transduction domain (ITAM) derives from the intracellular activation signal transduction domain of the immune receptor; preferably, the immune receptor is selected from TCRζ, CD2, CD3γ, CD3δ, CD3ε, CD3ζ, CD5, CD22, FcRy, CD66d, FcαRI, FcyRI, FcyRII, FcyRIII, Dectin-1, CLEC-1, CD72, CD79A, CD79B; preferably, the immune receptor activation signal transduction domain (ITAM) is fused with the NK cell signaling adaptor or its functional variant; and preferably, the immune receptor is CD3ζ.

In some preferred embodiments, the T cell co-stimulation signal transduction domain is derived from an intracellular signal transduction domain of a co-simulation molecule.

In some preferred embodiments, the full-length sequence of the human DAP10 includes an amino acid sequence having 80% or more identity to the amino acid sequence shown in SEQ ID NO. 25, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DAP10 is shown as in SEQ ID NO. 25.

In some preferred embodiments, the full-length sequence of the human DAP10 includes an amino acid sequence having 80% or more identity to the amino acid sequence shown in SEQ ID NO. 26, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DAP10 is shown as in SEQ ID NO. 26.

In some preferred embodiments, the transmembrane domain of human DAP10 includes an amino acid sequence having 80% or more identity to the amino acid sequence shown in SEQ ID NO. 27, preferably an amino acid sequence having an identity of 85%, 90%, 95%, 96%, 97%, 98%, or 99% or more, and more preferably an amino acid sequence having an identity of 98%, or 99% or more; and the amino acid sequence of the transmembrane domain of human DAP10 is shown as in SEQ ID NO. 27.

In some preferred embodiments, the full-length sequence of the human DAP12 includes an amino acid sequence having 80% or more identity to the amino acid sequence shown in SEQ ID NO. 28, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DAP12 is shown as in SEQ ID NO. 28.

In some preferred embodiments, the transmembrane domain of human DAP12 includes an amino acid sequence having 80% or more identity to the amino acid sequence shown in SEQ ID NO. 29, preferably an amino acid sequence having an identity of 85%, 90%, 95%, 96%, 97%, 98%, or 99% or more, and more preferably an amino acid sequence having an identity of 98%, or 99% or more; and the amino acid sequence of the transmembrane domain of human DAP12 is shown as in SEQ ID NO. 29.

In some preferred embodiments, the fusion protein of the transmembrane domains of human DAP12 and human DAP10 includes an amino acid sequence having 80% or more identity to the amino acid sequence shown in SEQ ID NO. 30, preferably an amino acid sequence having an identity of 85%, 90%, 95%, 96%, 97%, 98%, or 99% or more, and more preferably an amino acid sequence having an identity of 98%, or 99% or more; and the amino acid sequence of the transmembrane domains of human DAP10 and human DAP12 is shown as in SEQ ID NO. 30.

In some preferred embodiments, the sequence of fusion protein of human DAP10-DAP12 includes an amino acid sequence having 80% or more identity to the amino acid sequence shown in SEQ ID NO. 31, preferably an amino acid sequence having an identity of 85%, 90%, 95%, 96%, 97%, 98%, or 99% or more, and more preferably an amino acid sequence having an identity of 98%, or 99% or more; and amino acid sequence of the fusion protein of human DAP10-DAP12 is shown as in SEQ ID NO. 31.

In some preferred embodiments, the sequence of an intracellular signal transduction domain of human CD3zeta includes an amino acid sequence having 80% or more identity to the amino acid sequence shown in SEQ ID NO. 32, preferably an amino acid sequence having an identity of 85%, 90%, 95%, 96%, 97%, 98%, or 99% or more, and more preferably an amino acid sequence having an identity of 98%, or 99% or more; and the amino acid sequence of the intracellular signal transduction domain of human CD3zeta is shown as in SEQ ID NO. 32.

In some preferred embodiments, the sequence of human DAP10-CD3zeta includes an amino acid sequence having 80% or more identity to the amino acid sequence shown in SEQ ID NO. 33, preferably an amino acid sequence having an identity of 85%, 90%, 95%, 96%, 97%, 98%, or 99% or more, and more preferably an amino acid sequence having an identity of 98%, or 99% or more; and amino acid sequence of the DAP10-CD3zeta is shown as in SEQ ID NO. 33.

In some preferred embodiments, the sequence of human DAP12-CD3zeta includes an amino acid sequence having 80% or more identity to the amino acid sequence shown in SEQ ID NO. 34, preferably an amino acid sequence having an identity of 85%, 90%, 95%, 96%, 97%, 98%, or 99% or more, and more preferably an amino acid sequence having an identity of 98%, or 99% or more; and amino acid sequence of the DAP12-CD3zeta is shown as in SEQ ID NO. 34.

In some preferred embodiments, the sequence of human DAP10-DAP12-CD3zeta includes an amino acid sequence having 80% or more identity to the amino acid sequence shown in SEQ ID NO. 35, preferably an amino acid sequence having an identity of 85%, 90%, 95%, 96%, 97%, 98%, or 99% or more, and more preferably an amino acid sequence having an identity of 98%, or 99% or more; and amino acid sequence of the DAP10-DAP12-CD3zeta is shown as in SEQ ID NO. 35.

In some preferred embodiments, the sequence of an intracellular signal transduction domain of human 41BB includes an amino acid sequence having 80% or more identity to the amino acid sequence shown in SEQ ID NO. 36, preferably an amino acid sequence having an identity of 85%, 90%, 95%, 96%, 97%, 98%, or 99% or more, and more preferably an amino acid sequence having an identity of 98%, or 99% or more; and the amino acid sequence of the intracellular signal transduction domain of human 41BB is shown as in SEQ ID NO. 36.

In some preferred embodiments, the full-length sequence of the human DAP 10-41BB includes an amino acid sequence having 80% or more identity to the amino acid sequence shown in SEQ ID NO. 37, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DAP10-41BB is shown as in SEQ ID NO. 37.

In some preferred embodiments, the sequence of human DAP10-41BB-CD3zeta includes an amino acid sequence having 80% or more identity to the amino acid sequence shown in SEQ ID NO. 38, preferably an amino acid sequence having an identity of 85%, 90%, 95%, 96%, 97%, 98%, or 99% or more, and more preferably an amino acid sequence having an identity of 98%, or 99% or more; and amino acid sequence of the DAP10-41BB-CD3zeta is shown as in SEQ ID NO. 38.

In some preferred embodiments, the sequence of an intracellular signal transduction domain of human CD28 includes an amino acid sequence having 80% or more identity to the amino acid sequence shown in SEQ ID NO. 39, preferably an amino acid sequence having an identity of 85%, 90%, 95%, 96%, 97%, 98%, or 99% or more, and more preferably an amino acid sequence having an identity of 98%, or 99% or more; and the amino acid sequence of the intracellular signal transduction domain of human CD28 is shown as in SEQ ID NO. 39.

In some preferred embodiments, the sequence of human DAP10-CD28 includes an amino acid sequence having 80% or more identity to the amino acid sequence shown in SEQ ID NO. 40, preferably an amino acid sequence having an identity of 85%, 90%, 95%, 96%, 97%, 98%, or 99% or more, and more preferably an amino acid sequence having an identity of 98%, or 99% or more; and amino acid sequence of the DAP10-CD28 is shown as in SEQ ID NO. 40.

In some preferred embodiments, the sequence of human DAP10-CD28-CD3zeta includes an amino acid sequence having 80% or more identity to the amino acid sequence shown in SEQ ID NO. 41, preferably an amino acid sequence having an identity of 85%, 90%, 95%, 96%, 97%, 98%, or 99% or more, and more preferably an amino acid sequence having an identity of 98%, or 99% or more; and amino acid sequence of the DAP10-CD28-CD3zeta is shown as in SEQ ID NO. 41.

In some preferred embodiments, the sequence of the human DAP12-41BB includes an amino acid sequence having 80% or more identity to the amino acid sequence shown in SEQ ID NO. 42, preferably an amino acid sequence having an identity of 85%, 90%, 95%, 96%, 97%, 98%, or 99% or more, and more preferably an amino acid sequence having an identity of 98%, or 99% or more; and the amino acid sequence of the sequence of the human DAP12-41BB is shown as in SEQ ID NO. 42.

In some preferred embodiments, the sequence of human DAP12-41BB-CD3zeta includes an amino acid sequence having 80% or more identity to the amino acid sequence shown in SEQ ID NO. 43, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP12-41BB-CD3zeta is shown as in SEQ ID NO. 43.

In some preferred embodiments, the sequence of human DAP12-CD28 includes an amino acid sequence having 80% or more identity to the amino acid sequence shown in SEQ ID NO. 44, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP12-CD28 is shown as in SEQ ID NO. 44.

In some preferred embodiments, the sequence of human DAP12-CD28-CD3zeta includes an amino acid sequence having 80% or more identity to the amino acid sequence shown in SEQ ID NO. 45, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP12-CD28-CD3zeta is shown as in SEQ ID NO. 45.

In some preferred embodiments, the CNK signal transduction component includes an amino acid sequence selected from SEQ ID NOs. 25~45.

In some preferred embodiments, the chimeric protein construct and NK-cell-activating receptor and CNK signal transduction components form a multifunctional complex, wherein the multifunctional complex includes an amino acid sequence as shown in SEQ ID NO. 117. In some preferred embodiments, the multifunctional complex includes an amino acid sequence having 80% or more identity to the amino acid sequence shown in SEQ ID NO. 117, preferably an amino acid sequence having an identity of 85%, 90%, 95%, 96%, 97%, 98%, or 99% or more, and more preferably an amino acid sequence having an identity of 98%, or 99% or more; and the amino acid sequence of the multifunctional complex includes an amino acid sequence shown as in SEQ ID NO. 117.

### 6. Nucleic Acid Molecules

In another respect, the present disclosure also provides a chimeric nucleic acid construct, which encodes any of the chimeric protein constructs described in the present application.

The nucleic acid molecule may include deoxyribonucleic acid (DNA), ribonucleic acid (RNA), threose nucleic acid (TNA), glycol nucleic acid ( GNA), peptide nucleic acid (PNA), locked nucleic acid (LNA, including LNA with β-D-ribose configuration, α-LNA with α-L-ribose configuration (diastereoisomers of LNA), with 2'-amino-functionalized 2'-amino-LNA and 2'-amino-α-LNA with 2'-amino-functionalization), ethylene nucleic acid (ENA), cyclohexenyl nucleic acid (CeNA) and/or chimeras and/or combinations thereof. Exemplary DNA includes, but is not limited to, plasmid DNA (pDNA), etc. Examplary RNA includes, but are not limited to, mRNA, circular RNA, and ccRNA.

In some preferred embodiments, the nucleic acid molecule is mRNA. mRNA refers to any RNA that encodes at least one protein and is capable of producing the encoded protein in vitro, in vivo, in situ, or ex vivo. Those skilled in the art may understand that, unless otherwise indicated, the nucleic acid sequence described in the present application may enumerate "T" in a representative DNA sequence, but in the case of the sequence representing RNA (e.g., mRNA), "T" will be replaced by "U". Thus, any DNA disclosed and identified herein by a particular sequence identification number also discloses the corresponding RNA (e.g., mRNA) sequence complementary to the DNA, wherein each "T" of the DNA sequence is replaced by a "U".

In some preferred embodiments, the nucleic acid molecule includes a nucleotide sequence having 80% or more identity to the nucleotide sequence shown in SEQ ID NO. 118, preferably a nucleotide sequence having an identity of 85%, 90%, 95%, 96%, 97%, 98%, or 99% or more, and more preferably a nucleotide sequence having an identity of 98%, or 99% or more.

In some preferred embodiments, the nucleic acid molecule includes a nucleotide sequence having 80% or more identity to the nucleotide sequence shown in SEQ ID NO. 160, preferably a nucleotide sequence having an identity of 85%, 90%, 95%, 96%, 97%, 98%, or 99% or more, and more preferably a nucleotide sequence having an identity of 98%, or 99% or more. In some preferred embodiments, the nucleic acid molecule includes a nucleotide sequence having 80% or more identity to the nucleotide sequence shown in SEQ ID NO. 162, preferably a nucleotide sequence having an identity of 85%, 90%, 95%, 96%, 97%, 98%, or 99% or more, and more preferably a nucleotide sequence having an identity of 98%, or 99% or more. In some preferred embodiments, the nucleic acid molecule includes a nucleotide sequence having 80% or more identity to the nucleotide sequence shown in SEQ ID NO. 165, preferably a nucleotide sequence having an identity of 85%, 90%, 95%, 96%, 97%, 98%, or 99% or more, and more preferably a nucleotide sequence having an identity of 98%, or 99% or more.

In some preferred embodiments, the nucleic acid molecule is an in vitro synthesized mRNA. In some preferred embodiments, the in vitro synthesized mRNA has modifications, optionally, including one or more modifications selected from the following group: 5'UTR, 5'UTR, polyA tail, 5'-capped, and one or more modified nucleotides in the coding region.

### i) Modification of nucleic acid molecules (e.g., mRNA).

Natural mRNA molecules in eukaryotic cell may contain stable elements, including, but not limited to, untranslated regions (UTRs) at their 5'-terminus (5'UTR) and/or UTRs at their 3'-terminus (3'UTR), as well as other structural features, e.g., a 5'-cap structure and a 3'-polyA tail. Both 5' UTR and 3' UTR are usually transcribed from genomic DNA and are parts of premature mRNA. Characteristic structures of mature mRNAs (e.g., 5'-cap structure and 3'-polyA tail) are often added to a transcribed (preterm) mRNA during mRNA processing.

"5' UTR" refers to a region of an mRNA at upstream (i.e., 5') of the initiation codon (i.e., the first codon of the ribosome-translated mRNA transcript), which does not encode a polypeptide. When generating RNA transcripts, 5' UTR may contain a promoter sequence. Such promoter sequences are known in the art. It may be understood that such promoter sequences will not be present in nucleic acid molecules of the present disclosure.

"3 UTR" refers to the region of the mRNA at downstream (i.e., 3') of the stop codon (i.e., the codon of mRNA transcript indicating the translation termination), which does not encode the polypeptide.

A "polyA-tail" is the downstream region of an mRNA, e.g., a direct downstream of the 3' UTR (i.e., 3'), containing multiple consecutive adenosine monophosphates. ApolyA-tail may contain 10 to 300 adenosine monophosphates. For example, a polyA-tail may contain 10, 20, 30, 40, 50, 60, 70, 80, 90, 100, 110, 120, 130, 140, 150, 160, 170, 180, 190, 200, 210, 220, 230, 240, 250, 260, 270, 280, 290, or 300 adenosine monophosphates. In some embodiments, a polyA-tail contains 50 to 250 adenosine monophosphates. In relevant biological environments (e.g., in cells or in vivo), the role of a polyA-tail is to protect mRNA from enzymatic degradation (e.g., enzymatic degradation in the cytoplasm) and to aid transcription termination and/or mRNA output and translation from nucleus. The 3'-polyA-tail is usually a segment of adenine nucleotides added to the 3'-end of a transcribed mRNA. In some cases, it may contain up to about 400 adenine nucleotides. The length of the 3'-polyA-tail may affect the stability of the mRNA molecule itself.

The 5'-capping of polynucleotides can be done simultaneously during in vitro transcription reaction, and, depending on manufacturer's protocols, a 5'-guanosine cap structure may be generated by the following chemical RNA cap analogues: 3'-O-Me-m7G(5') ppp(5')G[ARCA cap], G(5')ppp(5')A, G(5') ppp(5') G, m7G(5') ppp(5') A, m7G(5') ppp(5') G (New England Biolabs, Ipswich, Massachusetts). The 5'-capping of a modified RNA can be done after transcription using vaccinia virus capping enzyme to produce a "Cap 0" structure: m7G(5')ppp(5')G (New England Biolab, Ipswich, MA). The Cap1 structure can be produced using vaccinia virus capping enzyme and 2'-O methyltransferase: m7G(5')ppp(5')G-2'-O-methyl. The Cap2 structure can be generated from the Cap1 structure, followed by 2'-O-methylation of the antepenultimate (the 5'-antepenultimate) nucleotide using 2'-O methyltransferase. The Cap3 structure can be generated from the Cap2 structure, followed by 2'-O-methylation of the last fourth (the 5'-preantepenultimate) nucleotide using 2'-O methyltransferase. Enzymes can be derived from recombinant sources.

An mRNA molecule comprising at least one of the above stable elements can significantly increase the expression of its encoded protein, and when two or more of the above stable elements are used, its protein expression shows a synergistic effect compared to an mRNA of a single stable element used.

In some embodiments, one or more AU-rich sequences are removed from the mRNA described herein. AU-rich sequences, also known as AURES, are de-stable sequences found in 3' UTR.

In some embodiments, the mRNA described in the present disclosure further comprises an open reading frame (ORF) encoding a signal peptide. The signal peptide may contain the 15th-60th amino acids at the N-terminus of the protein, which is normally required for membrane transport across secretory pathway, thus, generally controlling the secretory pathways of most proteins into eukaryotes and prokaryotes. The length of a signal peptide may be 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, or 59 amino acids.

An "open reading frame" is a continuous piece of DNA that begins with a start codon (e.g., methionine (ATG)) and ends with a stop codon (e.g., TAA, TAG, or TGA), and encodes a polypeptide.

Signal peptides derived from heterologous genes are known in the art and may be tested for desired properties and then incorporated into nucleic acids of the present disclosure. In some embodiments, the signal peptide may include one of the following sequences: MDSKGSSQKGSRLLLVVSNLLLPQGVVG (SEQ ID NO. 148), MDWTWILFLVAAATRVHS (SEQ ID NO. 149), METPAQLLFLLLLWLPDTTG (SEQ ID NO. 150), MLGSNSGQRVVFTILLVAPAYS (SEQ ID NO. 151), MKCLLYLAFLFIGVNCA (SEQ ID NO.152), MWLVSLAIVTACAGA (SEQ ID NO.153), and MFVFLVLLPLVSSQC (SEQ ID NO.154).

The nucleic acid molecules provided by the present application can be synthesized by solid-phase technology, liquid phase chemical synthesis, enzymatic ligation and combinations thereof. Synthesized nucleic acid molecules can be purified and quantified. Details may be seen in WO2021222304, the full content of which is incorporated herein by reference.

### 7. Vector

In another aspect, the present disclosure further provides a vector comprising the nucleic acid molecule provided by the present disclosure, wherein the nucleic acid molecule is operably linked to at least one polynucleotide regulatory element (e.g., a promoter) to express a chimeric protein construct encoded by the nucleic acid molecule.

In some embodiments, the vector is selected from: plasmid, nanoplasmid, cosmid, viral vector, mini circle, RNA vector, or linear or circular DNA (e.g., transposon DNA) or RNA molecules.

In some embodiments, the viral vector is selected from: retrovirus, lentiviral vector, adenovirus, parvoviruse (e.g., adeno-associated virus), adeno-associated virus (AAV) vector, coronavirus, negative-strand RNA virus such as orthomyxovirus (e.g., influenza virus), rhabdovirus (e.g., rabies and vesicular stomatitis viruses), paramyxovirus (e.g., Machi and Sendai), positive-strand RNA Virus such as picornavirus and alphavirus, and wherein the double-stranded DNA virus, comprises adenovirus, herpesvirus (e.g., herpes simplex virus types 1 and 2, Epstein-Barr virus, cytomegalovirus) and pox Virus (e.g., vaccinia, fowlpox, and canarypox), Norwalk, togavirus, flavivirus, reovirus, papovavirus, hepadnavirus, baculovirus, and hepatitis virus , Virus-like particle (VLP).

In some embodiments, the viral vector is a retroviral vector.

In some embodiments, the retroviral vector is selected from: avian leukoproliferative-sarcoma, mammalian C-type, B-type virus, D-type virus, HTLV-BLV collection, Lentivirus, bubble virus.

In some embodiments, the viral vector is a lentiviral vector. In some embodiments, the viral vector is an oncolytic virus vector. An oncolytic virus vector refers to an oncolytic virus-based viral vector in which exogenous nucleic acid sequences can be inserted.

In some embodiments, the lentiviral vector is selected from HIV-1, HIV-2, SIV, FIV, BIV, EIAV, CAEV or ovine demyelinating leukoencephalitis lentivirus.

In some embodiments, the vector is a transposon-based expression vector. A transposon is a DNA sequence that can change its position within genome. In the transposon subsystem, the nucleic acid molecule encoding the chimeric protein construct presented herein is flanked by terminal repeats recognized by transposases that mediate transposon movement. Transposases may be co-delivered as proteins, encoded on the same vector as the chimeric protein construct, or encoded on separate carriers. Non-limiting examples of transposable subsystems include Sleeping Beauty, Piggyback, Frog Prince, and Prince Charming.

In some embodiments, the vector provided by the present disclosure may be combined with other vectors, the other vectors comprising other nucleic acid molecules encoding at least one co-expression moiety.

In some preferred embodiments, the vector further includes a promoter; and preferably, the promoter is an EF1α promoter or CMV promoter.

In some preferred embodiments, the chimeric protein construct provided by the present disclosure may be expressed in the same vector with the co-expression moiety under regulation by the same or different promoters, or be expressed in a plurality of vectors.

### 8. Cell

In another respect, the present disclosure also provides an engineered cell, which expresses a chimeric protein construct as in the present disclosure, or comprises a chimeric nucleic acid construct or vector as provided by the present disclosure.

In some embodiments, the engineered cell simultaneously express the chimeric protein construct provided by the present disclosure and the co-expression moiety described above. The co-expression moiety and the target protein binding domain of the chimeric protein construct may be combined to achieve a specific purpose. For example, when the target protein binding domain specifically recognizes a protein expressed on immune cells that can cause immune rejection, the co-expression moiety may be a protein that further inhibits immune rejection (e.g., ER-resident glycoprotein of the virus, etc.), a protein that targets the tumor microenvironment (e.g., chemokine receptors, etc.), or a protein that stimulates immune activity (e.g., cytokines, immune cell germline proteins CD7, CD5, etc.), and the resulting cells will have reduced immunogenicity ( or immune rejection)when transplanted into the human body and/or have enhanced immune activity. This is useful for cell immunotherapy as well as other therapeutic cell transplantation. Thus, the cells may be immune cells (e.g., T cells), stem cells, kidney cells, islet cells, and cardiomyocytes, etc.

In some embodiments, the cell is an immune cell selected from: T cell, natural killer (NK) cell, B cell, macrophage, monocyte, dendrites cell, neutrophil, or γδT cell. The T cell may be selected from the group consisting of: CD8+ T cell, CD4+ T cell, cytotoxic T cell, terminal effector T cell, memory T cell, naive T cell, cell group consisting of regulatory T cell, natural killer T cell, gamma-delta T cell, cytokine-induced killer (CIK) T cell, and tumor infiltrating lymphocyte.

In some embodiments, the engineered cell is a T cell. In the case of expressing the chimeric protein construct and co-expression moiety described above to obtain a T cell with reduced immunogenicity and/or enhanced immune activity, the T cell may further express receptors that can specifically target tumor-related markers, including but not limited to CAR, TCR, NK-cell-activating receptor components, etc.

In some embodiments, the engineered cells are T cells, which simultaneously express a chimeric protein construct as provided by the present disclosure and selected from one or more of the following: adenovirus E3-K19, CAR, NK-cell-activating receptor and CNK signal transduction components.

In some embodiments, the engineered cells are T cells, which simultaneously express a chimeric protein construct, NK-cell-activating receptor, and optionally CNK signal transduction component, as provided by the present disclosure.

In some embodiments, the engineered cells are T cells, which simultaneously express a chimeric protein construct, adenovirus EK-K19, and CAR as provided by the present disclosure.

On the other hand, the present disclosure also provides a method for generating an engineered cell (e.g., T cell) described herein, which comprises introducing a vector described herein, under a condition suitable for expressing a nucleic acid molecule described herein, into an initiating cell.

Many methods known in the art to generate CAR-T cells may also be applied to generate engineered cells as described herein. For example, Zhang et al., Engineering CAR-T cells, Biomarker Research (2017) 5:22 describes a method of generating CAR-T cells. The method provided herein may include one or more of the following steps selected from: obtaining an initiating cell, culturing (including expansion, optionally including activation) the initiating cell, and genetically modifying the cell. The initiating cell may be a stem cell, wherein the stem cell may be a hematopoietic progenitor cell (e.g. T cell progenitor, NK cell progenitor, macrophage progenitor), a hematopoietic stem cell (HSC), a CD34+ cell, an embryonic cell line, a mesenchymal stem cell or an iPSC cell. The initiating cell may also be a cell that has differentiated from a stem cell, e.g., an immune cell described above.

The initiating cell may be obtained from any source, e.g., immune cell (e.g., T cell) isolated from a subject (e.g., human subject). In some embodiments, an immune cell is obtained from a subject of interest, e.g., a subject suspected of having a particular disease or condition, a subject suspected of having a susceptibility to a particular disease or condition, a subject who will experience, is currently experiencing, or has been treated for a specific disease or condition, and a subject that may also be a healthy volunteer or a healthy donor; and an immune cell can also come from blood banks. The immune cell may be autologous or allogeneic to a subject of interest. The immune cell can be collected from anywhere they are present in the subject, including but not limited to blood, umbilical cord blood, spleen, thymus, lymph nodes, pleural effusion, spleen tissue, tumors, and bone marrow. Isolated immune cells can be used directly or stored for a period of time, such as frozen.

Activation and/or proliferation of immune cells is one of the main steps in immune cell function. In some embodiments, immune cells are activated and proliferated at the same time, before, and/and after genetic modification. In some embodiments, immune cells are activated and/or proliferated in vitro, ex vivo or in vivo. Methods for activating and proliferating immune cells have been described in the art and may be used in the methods described herein. For example, T cells can be activated and proliferated through surface contact with agents attached to signals associated with stimulating the CD3/TCR complex and ligands stimulating co-stimulatory molecules on the surface of T cells. Particularly, the T cell population may be stimulated, for example by contacting with anti-CD3 antibody or its antigenbinding fragment or anti-CD2 antibody immobilized on the surface, or contacting with protein kinase C activator (e.g., phystinoid), as well as calcium ionophore. To co-stimulate helper molecules on the surface of T cells, ligands bound to helper molecules are used. For example, under conditions suitable for stimulating T cell proliferation, T cell populations can come into contact with anti-CD3 antibodies and anti-CD28 antibodies. To stimulate the proliferation of CD4+ T cells or CD8+ T cells, anti-CD3 antibodies and anti-CD28 antibodies can be used. In certain embodiments, the primary stimulation signal and co-stimulation signal of the T cell may be provided by different protocols.

Genetically modified cells may be achieved by transducing essentially homogeneous initiating cells with nucleic acid molecules encoding the chimeric protein constructs provided herein. In certain embodiments, a retroviral vector (e.g., lentiviral vector) is used to introduce the nucleic acid molecules provided herein into the initiating cells. For example, the nucleic acid molecules provided herein can be cloned into lentiviral vectors and expressed from their endogenous promoters, lentiviral long-terminal repeats, or promoters specific to the target cell type of interest. Conventional delivery methods used to deliver viral vectors include, but are not limited to, electroporation, microinjection, gene guns, and magnetic transfection.

Genetically modified cells can also be achieved by delivering nucleic acid molecules with LNP, as described in the "6. Nucleic Acid Molecules" subsection above. Other non-viral methods for nucleic acid delivery include in vitro transfection using calcium phosphate, DEAE dextran, electroporation, and protoplast fusion. Transposases or targeted nucleases (e.g., zinc finger nucleases, meganucleases, TALE nucleases, or CRISPR) may also be used to achieve genetic modification of the starting cell, thereby obtaining engineered cells as described in the present disclosure.

In certain embodiments, the engineered cells provided herein are prepared by transfecting nucleic acid molecules encoding the chimeric protein construct provided herein into initiating cells prior to administration. In certain embodiments, the engineered cells provided herein may be prepared by transfecting immune cells with nucleic acid molecules encoding the chimeric protein construct provided herein by, for example, viral vectors. The engineered cells provided herein exhibit reduced expression of immunogenic molecules (e.g., TCR, HLA, etc.) and/or immunosuppressive molecules (e.g., PD-1, etc.) and/or enhanced expression of tumor-targeted receptors (e.g., CAR, engineered TCR, CNK receptors, etc.) on cell surface.

In another respect, the present disclosure also provides a population of cells generated ex vivo by the above methods. In certain embodiments, at least 70%, at least 75%, at least 80%, at least 85%, at least 90%, or at least 95% of the cell population expresses the expression marker peptide provided herein (e.g., truncated EGFR (EGFRt) cleavably linked with the chimeric protein construct described herein). The expression level of truncated EGFR can represent the expression levels of chimeric protein constructs described here in the cell population.

### 9. Pharmaceutical Composition, and Kit

In another respect, the present disclosure also provides a pharmaceutical composition or a kit, which comprises (i) a chimeric protein construct, nucleic acid molecule, vector or cell population as described herein, and (ii) pharmaceutically acceptable media. The term "pharmaceutically acceptable media" refers to any and all solvents, dispersion media, coatings, antimicrobial agents and antifungals, isotonic agents and absorption delaying agents, etc., that facilitate the storage and administration of the chimeric protein construct, nucleic acid molecule, expression vector, and/or cell population provided herein, and pharmaceutically acceptable media may include any suitable component, for example, but not limited to, saline, liposomes, polymerization excipients, colloids, or carrier particles.

In certain embodiments, the pharmaceutically acceptable media are salines that can dissolve or disperse the chimeric protein construct, nucleic acid molecule, expression vector, and/or cell population provided by the present disclosure. Illustrative examples of salines include, but are not limited to, buffered saline, sucrose solution, normal saline, acetate buffer, phosphate buffer, citrate buffer, bicarbonate buffer, saline solution, and polysorbate solution.

### i) Nucleic acid pharmaceutical composition

In certain embodiments, the pharmaceutically acceptable media are liposomes. Liposomes are monolamellar or multilayer vesicles with membranes formed by lipophilic materials and an internal aqueous fraction. The nucleic acid molecules and/or vectors provided in the present disclosure may be encapsulated in the aqueous fraction of the liposomes. Exemplary liposomes include, but are not limited to, liposomes based on 3[N-(N', N'-dimethylaminoethane) carbamoyl] cholesterol (DC-Chlo), iposomes based on N-(2,3-dioleoyloxy) propyl-N, N, N-trimethylammonium chloride (DOTMA), and liposomes based on 1,2-dioleoyloxy-3-trimethylpropane (DOTAP). Methods for preparing liposomes and encapsulating nucleic acid molecules and/or vectors in liposomes are well known in the art (seeing, e.g., D.D. Lasic et al., Liposomes in gene delivery, published by CRC Press, 1997).

In certain embodiments, pharmaceutically acceptable media are polymeric excipients, including but not limited to microspheres, microcapsules, polymeric micelles, and dendritic polymers. The nucleic acid molecules and/or vectors provided by the present disclosure may be encapsulated, adhered to, or coated on polymer-based components by methods known in the art (seeing, e.g., W. Heiser, Nonviral gene transfer techniques, published by Humana Press, 2004; U.S. patent 6025337; Advanced Drug Delivery Reviews, 57(15):2177-2202(2005)).

In certain embodiments, pharmaceutically acceptable media are colloidal or carrier particles, e.g., gold colloids, gold nanoparticles, silica nanoparticles, and multi-segment nanorods. Nucleic acid molecules and/or vectors provided in the present disclosure may be coated, adhered to, or bound to the carrier in any suitable manner known in the art (seeing, e.g., M. Sullivan et al., Gene Therapy, 10:1882-1890 (2003), C. Mclntosh et al., J.Am. Chem.Soc., 123(31): 7626-7629 (2001), D. Luo et al., Nature Biotechnology,18:893-895(2000),and A. Salem et al.,Nature Materials,2:668-671(2003)).

In certain embodiments, the pharmaceutical composition may further comprise additives, including but not limited to stabilizers, preservatives and transfection accelerators that contribute to the cellular uptake of the drug. Suitable stabilizers may include, but are not limited to, sodium glutamate, glycine, EDTA, and albumin. Suitable preservatives may include, but are not limited to, 2-phenoxyethanol, sodium benzoate, potassium sorbate, methyl hydroxybenzoate, phenols, thimerosal, and antibiotics. Suitable transfection accelerators may include, but are not limited to, calcium.

The pharmaceutical composition provided herein may be administered by any suitable route known in the art, including, but not limited to, parenteral, oral, enteral, buccal, nasal, topical, rectal, vaginal, intramuscular, intranasal, transmucosal, epidermis, transdermal, dermal, eye, lung, and subcutaneous routes of administration. The pharmaceutical composition provided herein may be administered to a subject in the form of a formulation or preparation suitable for each route of administration. Formulations suitable for administration of pharmaceutical compositions may include, but are not limited to, solutions, dispersions, emulsions, powders, suspensions, aerosols, sprays, nasal drops, liposome-based formulations, patches, implants, and suppositories.

Preparations can conveniently exist in unit dosage forms and can be prepared by any method that is well known in the field of pharmacy. The method of preparing these preparations or pharmaceutical compositions comprises a step of providing the nucleic acid molecules described by the present disclosure to one or more pharmaceutically acceptable media and optionally one or more additives. Methods for the preparation of such preparations can be found in, for example, Remington's Pharmaceutical Sciences (Remington: The Science and Practice of Pharmacy, 19th ed., A.R. Gennaro(ed), Mack Publishing Co., N.J., 1995; R. Stribling et al., Proc.Natl.Acad.Sci.USA,89:11277-11281(1992); A.Barnes et al.,Current Opinion in Molecular Therapeutics 2000 2:87-93(2000); T.W.Kim et al.,The Journal of Gene Medicine,7(6):749-758(2005); and S.F. Jia et al., Clinical Cancer Research, 9:3462 (2003); A.Shahiwala et al., Recent patents on drug delivery and formulation,1:1-9(2007); of which references are incorporated herein by reference in entirety).

In some embodiments, the nucleic acid molecule (e.g., mRNA) may be delivered physically, biologically, or chemically (seeing, e.g., S. Guan, J. Rosenecker, Gene Ther. 2017, 24, 133.).

Physical methods include, but are not limited to, delivery by gene guns (e.g., with Au-particles), electroporation, acoustic perforation, etc. (seeing, e.g., Kutzler et al., (2008) DNA vaccines: Ready for prime time? Nat Rev Genet 9:776-788; Geall et al., Nonviral delivery of self-amplifying RNA vaccines. Proc Natl Acad Sci US A.Sep.4,2012; 109(36): 14604-9.).

Biological methods include, but are not limited to, delivery by viral vectors (e.g., retroviral vectors, adenoviral vectors, adeno-associated viral vectors).

Chemical methods include, but are not limited to, delivery via native proteins/glycans, polymers, lipids. Exemplary natural proteins/glycans include protamine and chitosan (seeing, e.g., A.E. et al., Cancer Immunol. Immunother.2015, 64, 1461; U.S. Kumar et al. ACS Nano 2021,11,17582). Exemplary polymers include polyethyleneimine (PEI) (e.g., linear PEI, branched PEI, and dendritic PEI), poly(β-aminoester) (PBAE) (see, e.g., K. Singha et al., Nucleic Acid Ther. 2011, 21, 133; A. A. Eltoukhy et al., Biomaterials 2012, 33, 3594).

Exemplary lipids include cationic lipids such as 1,2-diocta-decenyl-3-trimethy lammonium-propane (DOTMA) and 1,2-dioleoyl-3-trimethylammo niumpropane (DOTAP) (see, e.g., X. Hou et al., Nat.Rev.Mater.2021, 10, 1078.), Liposomes formed from DOTMA, DOTAP, and DOPE (1,2-dioleoyl-sn-glycero-3-phosphoethanolamine, auxiliary lipids), which can form colloidally stable nanoparticles with mRNA after self-assembly (see, e.g., L.M. Kranz et al., Nature 2016, 534, 396.).

Exemplary lipids can also be ionizable lipids. Ionizable lipids (pKa 6.5-6.9) are alternative lipid materials that are neutral at physiological pH but positively charged by protonation of free amines in an acidic environment (seeing, e.g., S.C. Semple et al., Nat. Biotechnol. 2010, 28, 172). After cell internalization, nanoparticles formed by ionizable lipids are encapsulated in endosomes. Subsequently, due to the continuous decrease in endosomal and lysosomal pH, ionized lipids can obtain protons for ionization, thereby promoting the fusion of lipid nanoparticles (LNPs) with endosomal membranes, ultimately enabling the release of mRNA loaded on lipid nanoparticles into the cytoplasm (seeing, e.g., L. Miao et al., Mol.Cancer 2021, 20, 41.).

Ionizable lipids can form lipid nanoparticle formulations with cholesterol, auxiliary lipids, and pegylated lipids (i.e., PEGylated lipids). Cholesterol is a naturally rigid and hydrophobic lipid that maintains the structure and stability of lipid nanoparticles. It can also facilitate fusion of mRNA-loaded lipid nanoparticles (i.e., mRNA nanoparticles) with endosomal membranes. Auxiliary lipids such as the zwitterionic lipid DOPE, 1,2-distearoyl-snglycero-3-phosphocholine (DSPC) and 1,2-Dioleoyl-sn-glycero-3-phosphocholine (DOPC) are widely used to facilitate cell membrane penetration and endosomal membrane escape (seeing, e.g., N. Chaudhary et al., Nat. Rev. Drug Discovery 2021,20,817.). PEGylated lipids are consisted of PEG and anchorage lipids. Hydrophilic PEGs are mainly distributed on the surface of mRNA complexes, while hydrophobic regions are embedded in lipid bilayers. The introduction of PEGylated lipids not only increases the half-life of lipid nanoparticles, but also adjusts the particle size by changing the molecular weight of the PEG chain. Typically, molecular weight and lipid tail length can range from 350 to 3000 Da and 10 to 18 carbons, respectively (seeing, e.g., N. Chaudhary et al., Nat. Rev. Drug Discovery 2021, 20, 817.).

Over the past few decades, researchers have developed large libraries of ionizable lipids for mRNA delivery, including DLin-MC3-DMA, SM-102, TT3, C12-200, 306O i10, and ALC-0315 (seeing, e.g., M. Yanez Arteta et al., Proc.Natl.Acad.Sci.USA 2018, 115, E3351; R. Verbeke et al., Controlled Release 2021,333,511; B. Li et al., Nano Lett.2015,15,8099; K.A. Hajj et al., Nano Lett.2020,20,5167; K.A. Hajj et al., Small 2019,15,1805097; A.B. Vogel et al., Nature 2021,592,283). Some of them have achieved remarkable results in clinical applications. A typical example is DLin-MC3-DMA, a key component of Onpattro approved by the U.S. Food and Drug Administration (FDA) for siRNA delivery (see, e.g., A. Akinc et al., Nat. Nanotechnol. 2019, 14, 1084). DLin-MC3-DMAis also widely used for mRNA delivery, including protein and peptide replacement, gene editing, and antiviral infection (see, e.g., R.S. Riley et al., Sci.Adv.2021, 7, eaba1028.). SM-102 and ALC-0315, two "star molecules," have been approved by the FDA as key components in BNT162b and mRNA-1273 vaccines to prevent COVID-19, respectively (seeing, e.g., X. Hou et al., Nat. Rev. Mater. 2021, 10, 1078.).

An ideal lipid-based mRNA vector must meet the following conditions: 1) the bare mRNA can form stable complexes that protect the mRNA from degradation; 2) four key components (ionizable lipids, cholesterol, auxiliary lipids, and pegylated lipids) should be added to stabilize the mRNA complex; 3) the components of lipid nanoparticles should be protonated to trigger membrane instability and promote endosomal escape of mRNA complexes; and 4) all lipid materials are biodegradable and do not cause any harm to patients.

The following points should be considered when optimizing lipid-based delivery platforms: 1) the degradation ability of ionizable lipids: the skeleton structure of lipids promotes lipid clearance and reduces toxicity by introducing alkyne and ester groups into the lipid tail; 2) immunogenicity of lipid nanoparticles: heterocyclic lipids within lipid nanoparticles can improve the efficiency of mRNA vaccines by activating the interferon gene stimulator (STING) pathway of dendritic cells (DCs) (seeing, e.g., L. Miao et al., Nat. Biotechnol. 2019, 37, 1174.); 3) stability of lipid nanoparticles: several promising strategies are expected to improve the stability of mRNA vaccines, including pKa optimization, excipient introduction, and mRNA modification, etc.

Lipid nanoparticles can be produced using components, compositions, and methods known in the art, see, for example, PCT/US2016/052352, PCT/US2016/068300, PCT/US2017/037551, PCT/US2015/027400, PCT/US2016/047406, PCT/US2016/000129, PCT/US2016/014280, PCT/ US2016/014280, PCT/US2017/038426, PCT/US2014/027077, PCT/US2014/055394, PCT/US2016/052117, PCT/US2012/069610, PCT/US2017/027492, CT/US2016/059575, and PCT/US2016/ 069491, all content of which are incorporated herein by reference.

### ii) Oncolytic virus and pharmaceutical composition thereof

In some embodiments, the present application provides an oncolytic virus capable of expressing the chimeric protein construct of the present application. In the present application, "oncolytic virus" means any virus capable of infecting tumor cells, replicating in tumor cells, and lyzing tumor cells. In certain embodiments, the oncolytic virus is further able to spread to other tumor cells in a continuous replication cycle.

Oncolytic viruses can be derived from a variety of viruses, and non-limited examples include vaccinia virus, adenovirus, herpes simplex virus 1 (HSV1), herpes simplex virus 2 (HSV2), myxovirus, reovirus, poliovirus, vesicular stomatitis virus (VSV), measles virus (MV), Lassa virus (LASV), and Newcastle disease virus (NDV), as well as variants of these viruses.

In some embodiments, the present application provides an oncolytic virus, comprising the nucleic acid molecule provided by the present disclosure, wherein the nucleic acid molecule is operably linked to at least one polynucleotide regulatory element (e.g., a promoter) to express a chimeric protein construct encoded by the nucleic acid molecule.

In some embodiments, the oncolytic virus described in the present application may infect substantially any cell type. In some embodiments, the oncolytic virus may be replication-selective. Replication-selective oncolytic virus replicate more in tumor cells than in non-tumor cells.

It may be understood that oncolytic virus can be replicately selective if viral replication is under the control of gene expression regulators, for example enhancer/promoter regions derived from the 5' side of the albumin gene (seeing, e.g., Miyatake et al., 1997, J. Virol.71: 5124-5132).

In some embodiments, the oncolytic virus may have tumor cell-specific promoters in their genomes, or tumor cell-specific transcriptional regulatory sequences. "Tumor cell specific" for promoter or transcriptional term sequences means that they are usually present in the target tumor cell at higher levels than normal cells. In this way, tumor cell specificity is conferred with oncolytic virus enhancement levels.

For example, by operatively attaching the HSV gene to the TGF-β promoter, the major transcriptional units of HSV can be placed under the transcriptional control of the tumor growth factor-β (TGF-β) promoter. Certain tumor cells are known to overexpress TGF-β relative to the same type of non-tumor cells. Thus, the oncolytic virus of which replication is controlled by transcription of TGF-β promoter is replication-selective because it replicates more in some tumor cells than in the same type of non-tumor cells. Similar replication-selective oncolytic virus can be prepared using any gene expression modifier known to selectively cause overexpression in affected cells. Replication-selective oncolytic virus can be, for example, HSV-1 mutant, in which the gene encoding ICP34.5 is mutated or missing. Oncolytic virus can also contain other modifications further in their genomes. For example, oncolytic virus can contain extra DNA inserted into the UL44 gene. This insertion can produce functional inactivation of the UL44 gene and the resulting cleavage phenotype, or it can be inserted into an already inactivated gene, or replace a missing gene.

In some embodiments, the oncolytic virus is engineered to place the nucleic acid sequence encoding the chimeric protein construct described in the present application under the control of a tumor cell-specific promoter.

In some embodiments, the oncolytic virus is engineered to place genes encoding at least one protein required for viral replication under the control of tumor cell-specific promoters.

In some embodiments, the oncolytic virus vector may express the chimeric protein construct described in the present application, and the targeting domain of the chimeric protein construct is capable of recognizing and binding to tumor proteins. Tumor proteins can be proteins that are highly expressed in tumor cells (e.g., c-Myc, Bcl-2, Bcl-xL, Bcl-w, KRAS), proteins expressed in tumor cells that promote tumor cell growth (e.g., estrogen receptors, androgen receptors, Her2, VEGF, VEGFR, PDGFRβ, EGFR, EGFR mutants), or proteins that promote tumor cells to escape from normal immune responses (e.g., PD-L1, TGF-β1).

In some embodiments, the targeting domain of the chimeric protein construct may bind to Bcl-2. Any targeted domain capable of target binding BCL-2 can be used. For example, but not limited to, BH1, which is highly conserved and homologous against Bcl-2 anti-apoptosis family member proteins, is developed, in which the nanobody sequence of the BH2 motif can be used as a targeted domain to construct ER-TPD elements targeting Bcl-2 family proteins; this element is cloned and mounted on an oncolytic viral strong promoter and specifically infects tumor cells; oncolytic viruses amplify in large quantities and express ER-TPD elements of Bcl-2 family proteins, thereby realizing the degradation of Bcl-2 antiapoptotic member proteins in cells, directly leading to apoptosis of tumor cells.

In some embodiments, the targeted domain of the chimeric protein construct may bind to VEGFR. Any targeted domain capable of target binding VEGFR can be used. For example, but not limited to, by connecting the heavy and light chain recombinant scFv of the VEGFR antibody (anti-VEGF-A antibody Bevacizumab and anti-VEGFR2 antibody Ramucizumab) in series with the ER-TPD element, the targeted VEGFR ER-TPD element was constructed; the element is cloned and loaded on the strong promoter of the oncolytic virus, and after specific infection of tumor cells, the oncolytic virus amplifies in large quantities and expresses the VEGFR ER-TPD element, thereby directly inhibiting the expression of VEGFR in tumor cells, resulting in growth inhibition and apoptosis of tumor cells due to lack of sufficient nutrients.

In some embodiments, the targeted domain of the chimeric protein construct may bind to TGF-β1 and PD-L1. Any targeted domain capable of target binding BCL-2 can be used. For example, but not limited to, ER-TPD elements targeting TGF-β1 and PD-L1 are constructed by connecting heavy and light chain recombinant scFv using TGF-β1 and PD-L1 antibodies in series with ER-TPD elements; the element was cloned and loaded after the oncolytic virus strong promoter, and after specifically infecting tumor cells, the oncolytic virus amplifies in large quantities and expresses TGF-β1 and PD-L1ER-TPD elements, thereby directly inhibiting and degrading the expression of TGF-β1 and PD-L1 in tumor cells, breaking the tumor immunosuppressive microenvironment, thereby achieving the effect of treating tumors.

It is not limited by theory, but it is considered that the chimeric protein construct of the present application has a unique advantage in expressing it in tumor cells by oncolytic viral vectors. On one hand, the chimeric protein construct of the present application targets the degradation of the protein in the tumor cell to help the tumor cell growth or immune escape or anti-apoptosis protein, promoting the apoptosis or death of the tumor cell. On the other hand, oncolytic virus vectors can specifically replicate in tumor cells, promoting tumor cell lysis through viral replication and lysis, integrating two different tumor killing methods in oncolytic virus vectors, and achieving synergistic killing in the same drug form.

In another respect, the present disclosure also provides a pharmaceutical composition comprising oncolytic viruses and pharmaceutically acceptable media provided by the present disclosure. In certain embodiments, pharmaceutically acceptable media include any and all transport media, solvents, diluents, excipients, adjuvants, dispersion media, coatings, antibacterial and antifungal agents, absorbents, etc. which are compatible with administration in mammals and particularly human objects.

In certain embodiments, the oncolytic virus or pharmaceutical composition thereof described in the present application is formulated for intravenous or intratumoral administration. In certain embodiments, oncolytic viruses or pharmaceutical compositions thereof may be placed in solvents or diluents suitable for human or animal use. The solvents or diluents are preferably isotonic, hypotonic or weakly hypotonic and has a relatively low ionic strength. Typical examples include sterile water, saline (e.g., sodium chloride), Ringer's solution, glucose, trehalose or deer sugar solutions, Hank's solution, and other aqueous physiologically balanced salt solutions.

In one embodiment, the oncolytic virus or pharmaceutical composition thereof described in the present application may have a buffer agent. Suitable buffer agent includes, but are not limited to, phosphate buffers (e.g., PBS), bicarbonate buffers, and/or Tris buffers capable of maintaining physiologically or slightly alkaline pH (e.g., from approximately pH 7 to approximately pH9).

In one embodiment, the oncolytic viral composition of the present invention may be prepared to improve its stability, especially its stability under production conditions and for long-term (i.e., at least 6 months, preferably at least 2 years) frozen (e.g., -70 °C, or - 20 °C) storage, refrigerated storage (e.g., 4 °C), or room temperature environment storage. A variety of viral agents existing in the art are either frozen liquid forms or lyophilized forms (e.g., WO98/02522 and WO2008/114021, etc.). Solid (e.g., dry powder or lyophilized) composition may be obtained by a step involving vacuum drying and lyophilization. For illustrative purposes, buffered preparations with NaCl and sugar are particularly suitable for preserving viruses.

### iii) Cytopharmaceutical composition

In another respect, the present disclosure also provides a cytopharmaceutical composition comprising engineered cells or cell population and pharmaceutically acceptable media provided by the present disclosure. Exemplary media include: buffer agents, such as neutral buffer saline, phosphate buffer saline, etc.; carbohydrates, such as glucose, mannose, sucrose or dextran, mannitol; protein; peptides or amino acids such as glycine; Antioxidant; chelating agents such as EDTA or glutathione; adjuvants (e.g. aluminum hydroxide); and preservatives. In one respect, the cytopharmaceutical composition of the present invention is formulated for intravenous administration.

### III. TPD Application

In one aspect, the present disclosure also provides a use of chimeric protein constructs, nucleic acid molecules or vectors described in the present application in the preparation of cells for treatment. In one aspect, the present disclosure also provides a use of chimeric protein constructs, nucleic acid molecules or vectors described in the present application in treating a disease. In one aspect, the present disclosure also provides a use of cells expressing chimeric protein constructs, nucleic acid molecules or vectors described in the present application in treating a disease.

### 1. Cell transplantation

In one aspect, the present disclosure also provides a use of chimeric protein constructs, nucleic acid molecules or vectors described in the present application in the preparation of cells to be transplanted (e.g., allogeneic cell transplantation).

In certain embodiments, the cells to be transplanted are immune cells (e.g., T cells), stem cells (and cells derived from their differentiation), kidney cells, islet cells, cardiomyocytes, etc. In certain embodiments, the cells to be transplanted may be autologous cells or allogeneic cells.

In one aspect, the present application also provides a cell to be transplanted expressing the chimeric protein construct, nucleic acid molecule or vector described above, expressing the chimeric protein construct of the present application, wherein the chimeric protein construct comprises an ERAD mechanism protein binding domain and a targeted domain, the targeted domain comprises a domain that specifically targets or binds to a target protein, and the target protein is a transplant rejection-related protein. In some embodiments, the chimeric protein constructs further comprise protein degradation pathway member (e.g., E3 ubiquitin ligase, proteasome, lysosome) binding domain described in the present application. Any protein degradation pathway member binding domain described in the present application may be used.

In certain embodiments, the transplant rejection-related proteins include: an antigen-presenting molecule (e.g., a MHC class I molecule, a MHC class II molecule, a MICA/B molecule, etc.), an antigen recognition molecule (e.g., TCR, CD123, NKG2D, etc.), an immune checkpoint molecule (e.g., PD-1, PD-L1, CTLA4, TIM3, TIGIT, LAG3, A2AR, BTLA, IDO1, IDO2, TDO, KIR, NOX2, VISTA, SIGLEC7, PVR, etc.), etc.

In certain embodiments, the transplant rejection-related protein comprises an immunogenic protein (e.g., HLA (HLA α/β), TCR (αβTCR), NKG2D (natural killer cell group 2-member D) ligand, etc.).

he present disclosure also provides the above-mentioned cells to be transplanted and their treatment methods, which can overcome the problems of the source of personalized transplanted cells and therapeutic cells, the inability to standardized and scale production, and the low treatment efficiency faced in existing cell transplantation and cell therapy.

The immune system is highly plastic and has a near-limitless ability to detect invading viruses, bacteria, xenologous cells, and diseased cells. This remarkable ability for immunologic surveillance is primarily achieved by humoral immunity and cellular immunity, wherein comprised are two important molecule structures, immunoglobulins and T cell receptors (TCR). The TCR, a defining structure of T cells, is a transmembrane heterodimer consisting of either an alpha and beta chain or delta and gamma chain linked by a disulfide bond. Within these chains, complementary determining regions (CDRs) determine the antigen to which the TCR will bind. In the TCR, TCRα and TCRβ subunits (or TCRγ and TCRδ in γδ T cells) are responsible for the recognition of the Major histocompatibility complex(MHC)/antigen ligand.

uman MHC class I gene region comprises allels of HLA-A, B, C sites, which encode classic class I antigen (molecule) e.g., HLA-A antigen, B antigen and C antigen, etc., known as HLA-A, HLA-B, and HLA-C. These antigen molecules are present on the surface of all somatic cells and bind to intracellular protein epitope peptides for recognition by the immune system. If cells produce mutant proteins, or foreign bacteria or viruses invade, the cells will present these mutant proteins or heterologous protein epitopes, immune cells will recognize them and carry out immune attack and kill, thereby removing diseased cells, germs and viruses invading cells.

Alpha/beta T lymphocytes recognize peptide-MHC ligands by means of a multimeric protein ensemble termed the αβ T cell antigen receptor (TCR).CD3 complex. This structure is composed of a variable αβ TCR dimer which binds antigens and three invariant dimers (CD3γε, δε and ζζ) which are involved in TCR.CD3 surface transport, stabilization and signal transduction. The alpha beta T cell receptor (αβTCR) is expressed on the majority (approx. 95%) of T cells and has a critical role in T cell activation via recognition of major histocompatibility complex (MHC)-anchored antigen. Therefore, TCR-mediated activation of T cells is the key step of the pathogenesis of graft versus host disease (GVHD) during the Allogeneic hematopoietic cell transplantation (allo-HCT) and allogeneic CAR-T cell therapy.

The human leukocyte antigen (HLA) system or complex is a group of related proteins that are encoded by the major histocompatibility complex (MHC) gene complex in humans. These cell-surface proteins are responsible for the regulation of the immune system. In therapeutic transplant setting, "HLA mismatch" happens when the donor HLA on the allograft differs from the recipient. HLAmismatch leads to the activation of alloreactive T cells, which can cause acute cellular rejection (ACR) within six months of transplantation. Mismatched donor HLA antigens are also targets for the development of de nova donor-specific HLA antibodies (dnDSA) which play augmented roles in both acute and chronic transplant cells (e.g., T cell) rejection. Therefore, to generate universal transplant cells (e.g., T cell) for safe allogenic infusion and therapeutic purpose, it is advisable to effectively block graft-versus-host disease (GVHD) by genetically disrupting the TCR. In addition, it is necessary to inhibit HLA expression on the allogeneic cell (e.g., T cell) to reduce rejection of the recipient immune system on allogeneic T cell TCRαβ and/or HLA class I of the allogeneic T cells.

In certain embodiments, the chimeric protein constructs, nucleic acid molecules, or vectors described above have reduced immunogenicity.

In some embodiments, the cells to be transplanted having reduced immunogenicity (or having a high degree of compatibility) described in the present application are T cells, which expresse TPD chimeric protein construct comprising a transmembrane domain or functional variant thereof specifically recognizing TCR and/or HLA and HCMV glycoprotein US2 and/or US 11, and an endoplasmic reticulum resident domain or its functional variant.

The ERAD mechanism protein binding domain comprises an amino acid sequence as indicated in SEQ ID NO. 76 or SEQ ID NO.82 or an amino acid sequence that has at least 80% (e.g., at least 85%, at least 90%, at least 95% or at least 99%) identity to it.

In some embodiments, the cells to be transplanted with reduced immunogenicity are T cells expressing ER-TPD chimeric protein constructs comprising transmembrane domains of scFv specifically recognizing TCR and adenovirus E3-K19 or their functional variants and endoplasmic reticulum resident domains or their functional variants. In some embodiments, the cell to be transplanted having reduced immunogenicity is a T cell expressing an ER-TPD chimeric protein construct comprising a target protein binding domain and an ERAD mechanism protein binding domain, wherein the target protein binding domain includes 6 CDRs contained an amino acid sequence as shown in SEQ ID NO: 116 or an amino acid sequence that has at least 80% (e.g., at least 85%, at least 90%, at least 95% or at least 99%) identity. The ERAD mechanism protein binding domain comprises an amino acid sequence as indicated in SEQ ID NO. 60 or an amino acid sequence that has at least 80% (e.g., at least 85%, at least 90%, at least 95% or at least 99%) identity.

In some embodiments, the ER-TPD chimeric protein construct with reduced immunogenicity expressed by the T cell, further comprises a protein degradation pathway member (e.g. E3 ubiquitin ligase, proteasome) binding domain, and optionally, the protein degradation pathway member binding domain being linked to the ERAD mechanism protein binding domain. In some embodiments, the ER-TPD chimeric protein construct with reduced immunogenicity expressed by the T cell further comprises an E3 ubiquitin ligase binding domain having an amino acid sequence as shown in SEQ ID NO.147.

In some embodiments, the above-described cells to be transplanted with reduced immunogenicity (e.g., T cells) also express a co-expression moiety as described above.

For example, in order to reduce the immunogenicity or antigen presentation of cells, proteins capable of degrading or reducing MHC class I or II molecules may be expressed in the cells to be transplanted (e.g., T cells) with reduced immunogenicity as co-expression moieties (e.g., intact viral ER-resident glycoproteins, including, but not limited to, HCMV US2, US3, US11, US10, adenovirus E3-K19, HCMV US6, and HSV ICP47). Effective downregulation of TCR may greatly inhibit TCR-mediated immune attack and reduce GVHD during allogeneic transfusion of T cells. Glycoproteins including native viral ER residency can further inhibit MHC molecules, thereby preventing peptide presentation to recipient CD8+ T cells and inhibiting immune recognition by allogeneic T cells. Therefore, highly compatible T cells, also known as "Universal T cells (UT)" as described above, can achieve allogeneic transfusion therapy purposes, and can improve the compatibility of allogeneic T cells and long-term durability after infusion.

In another aspect, the present disclosure provides a method of reducing a subject's immune response to cell transplantation, including administering a therapeutically-effective amount of cells to be transplanted as described above having a reduced immunogenicity to the subject, thereby reducing the subject's immune response to the graft or to the modified cell. In certain embodiments, the cells to be transplanted express within the cell a TPD chimeric protein construct described herein, wherein the TPD chimeric protein construct comprises a domain targeting immunogenic proteins (e.g., HLA (HLAα/β), TCR (αβTCR), NKG2D (natural killer cell group 2-member D) ligands, etc.) and the ERAD mechanism protein binding domain described above, and optionally, the protein degradation pathway member binding domain.

### 2. Cell therapy

Cell therapy, through genetic engineering technology to modify cells (such as immune cells) so that they can present tumor-related antigens or express receptors that specifically recognize disease cells, and then infuse them into patients after exponential expansion in vitro, activating the immune system in vivo to attack tumor cells or directly specifically recognize and kill disease cells. In 2012, CD19-targeted CAR-T cells achieved the first targeted removal of tumor cells from B-cell leukemia patients in the history of human medicine, becoming another treatment technology that can truly cure leukemia after bone marrow stem cell transplantation treatment technology, opening a new era of precision medicine cell therapy. This technology is expected to be applied to the treatment of various hematological tumors and solid tumors. However, the current clinical efficacy of conventional CAR-T in the treatment of solid tumors is not good, and the reasons are: (1) the killing function of CAR-T is highly dependent on the recognition of tumor-associated antigen (TAA) by CAR structure, but due to the heterogeneity of solid tumors, the expression of target proteins on the surface of tumor cells is very different; at present, a single targeted CAR-T cannot completely recognize and kill all tumor cells, resulting in immune evasion and recurrent metastasis of tumors; (2) there is direct immunosuppressive effect of solid tumors, expressing PD-L 1, B3H7, etc., directly inhibiting the activation of T cells; (3) in the immunosuppressive microenvironment of solid tumors, there are a large number of immunosuppressive cells, e.g., TAM, Treg, MDSC, and the design of conventional CAR-T is difficult to break through the limitations of immunosuppressive cells inside the tumor; (4) conventional CAR-T is personalized cell therapy, that is, T cells source the patient itself, and if the patient has received a large number of radiotherapy and chemotherapy, etc., immune system function is impaired, and therefore it is difficult to isolate a sufficient number of T cells from the patient's peripheral blood, even if isolated and modified; the proliferation and killing function of T cells is still very weak, so it is difficult to exert the therapeutic effect.

The cells to be transplanted (e.g., T cells) provided in the present application are able to overcome the problems of tumor target variability, tumor immunosuppressive environment, personalized treatment cell source and other problems faced in existing cell therapy.

For the purpose of cell therapy, the cells to be transplanted with reduced immunogenicity (e.g., T cells, further, universal T cells with defects in TCRαβ and MHC molecular surface expression) may be further genetically engineered, for example, to express the co-expression moiety described above, to achieve therapeutic purposes.

For example, in order to enable the cell to be transplanted (e.g., T cell) to act specifically on the disease-related target protein or the cell expressing the disease-related target protein, the binding domain or molecule that recognizes or binds to the disease-related target protein may be expressed in the cell to be transplanted (e.g., T cell) having reduced immunogenicity, e.g., a chimeric antigen receptor CAR, engineered TCR, or CNK receptor provided by the present application, or antiviral protein binding domain, etc.

In some embodiments, molecules capable of mediating the migration of the disease to be transplanted to the disease microenvironment (e.g., chemokine receptors or cytokine receptors) may also be expressed in the above-described cells to be transplanted (e.g., T cells) having reduced immunogenicity.

For another example, in order to enable the cells to be transplanted (e.g., T cells) to have increased immunostimulating activity, immunostimulatory molecules may be expressed in the above-mentioned cells to be transplanted having reduced immunogenicity (e.g., T cells).

n another aspect, the present application also provides that NK elements, especially optimized recombinant NK elements are introduced into the cells to be transplanted (e.g., UT cells) provided by the present application, such that T cells can be as efficient as NK cells to recognize all virus-infected cells and tumor cells, optionally, further enable CNK-T cells be capable of efficiently activating and killing tumor cells by optimizing the transduction element. Since that NK targets including family member proteins such as MICA, MICB and ULBP1-6, etc., can be widely expressed in various tumor cells and cover different stages of tumor progression, CNK technology can effectively solve the off-target effect of a single CAR-T and eliminate the probability of tumor immune escape. Besides, the Chimeric Adaptor introduced by CNK in the design can effectively transduce and amplify NK signals, break the limitations of immune checkpoints e.g., PD1 signals, efficiently activate T cells, and achieve the killing of tumor cells. The chimeric Adaptor amplifies CNK-T cell signals, and is capable of resisting immunosuppression in the tumor environment, realize the activation of T cells, and realize the killing of tumor cells. Then CNK-T cells recognize targets through NK, and can also clear immunosuppressive cells e.g., MDSC. After the virus infects cells and expresses specific functional proteins, the assembly or transport of MHCI or directly promotes the directional degradation of MHCI molecules, thereby inhibiting the presentation of viral antigen epitopes and producing immune escape.

In one aspect, the present disclosure provides a UT cell that also expresses a CNK receptor and optionally CAR, such UT cells may also be referred to as "CNKT-UT". In this application, it is verified that CNK-UT has broad-spectrum tumor identification and killing capabilities. This application further designs chimeric specific target CAR/CNK-UT products, demonstrating that CAR/CNK-UT products have more powerful killing and activation functions on tumor cells than conventional CAR-T. In animal experiments, CAR/CNK-UT products also have more efficient tumor clearance capabilities. UT technology realizes the allogeneic universal transformation, T cells source healthy donors, so as to realize the standardization and large-scale production of CNK-UT products, which can be prepared in advance, and ensure the function of T cells to kill and activate tumor cells.

In some preferred embodiments, the CNKT-UT includes a nucleic acid molecule encoding an amino acid sequence having 80% or more identity to the amino acid sequence shown in SEQ ID NO. 117, preferably an amino acid sequence having an identity of 85%, 90%, 95%, 96%, 97%, 98%, or 99% or more. In some preferred embodiments, the CNKT-UT includes a nucleic acid molecule encoding the amino acid sequence shown in SEQ ID NO. 117. In another aspect, the present disclosure also provides a use of CNKT-UT in the preparation of drugs for the treatment of diseases.

In one aspect, the present disclosure also provides an immune cell comprising or expressing the chimeric protein construct, nucleic acid molecule encoding it or vector described in the present application, wherein the chimeric protein construct comprises an ERAD mechanism protein binding domain and a targeting domain, the targeting domain comprises a domain that specifically targets or binds to a target protein, and the target protein is expressed both on the immune cell and the target cell. In certain embodiments, the immune cell further comprises or expresses a binding domain or molecule (e.g., CAR) targeting the target protein, a nucleic acid molecule or vector encoding it. In certain embodiments, examples of such target proteins include, CD123, CD5, CD7, CD38, or CD4.

Not limited by theory, certain target proteins are expressed both on target cells, e.g., cancer cells or virus-infected cells, and on immune cells used for treatment. Such targets are often difficult to treat by cell therapy, and therefore, when the immune cells used for treatment express CAR targeting such targets, CAR may also lead to the recognition of the same target expressed by the immune cells themselves, resulting in autophagy of immune cells, which cannot be expanded normally, or has limited killing effect on target cells. By the chimeric protein construct provided in the present application, the target protein expressed by the immune cell itself can be degraded, thereby avoiding autophagy while retaining the specific killing effect on the target cell. Another advantage of using the chimeric protein construct of the present application is that the chimeric protein construct targeting the target and the binding domain targeting the target (e.g., CAR, TCR, etc.) can be expressed simultaneously by co-expression, which is simpler and more effective than traditional gene knockout methods.

n some embodiments, the chimeric protein constructs further comprise protein degradation pathway member (e.g., E3 ubiquitin ligase, proteasome, lysosome) binding domain described in the present application. Any protein degradation pathway member binding domain described in the present application may be used.

The method of cell administration for adoptive cell therapy is known and can be combined with the methods and compositions provided. For example, the methods of adoptive T cell therapy are described in U.S. Patent Application No. 2003/0170238 (Gruenberg et al.) for example; U.S. Patent No. 4,690,915 (Rosenberg); and Rosenberg (2011) Nat Rev Clin Oncol.8(10):577-85). Details may be seen in e.g., Themeli et al. (2013), Nat Biotechnol.31(10): 928-933; Tsukahara et al. (2013), Biochem Biophys Res Commun 438(1): 84-9; Davila et al. (2013) PLoS ONE 8(4): e61338.

In certain embodiments, the individual population or subtype of cells or cells is administered to a subject in the range of about one million to about one hundred billion cells, e.g., 1 million to about 50 billion cells (e.g., about 5 million cells, about 25 million cells, about 500 million cells, about 1 billion cells, about 5 billion cells, about 20 billion cells, about 30 billion cells, about 40 billion cells, or the range defined by any of the above two values), such as about 10 million to about 100 billion cells(e.g., about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or the range defined by any of the above two values), and in some cases, about 100 million cells to about 50 billion cells (e.g., About 120 million cells, about 250 million cells, about 350 million cells, about 450 million cells, about 650 million cells, about 800 million cells, about 900 million cells, about 3 billion cells, about 30 billion cells, about 45 billion cells) or any value in between.

In some embodiments, the dose of total cells and/or individual subsets of cells is in the range from being at or about 10⁴ cells/kg body weight to being at or about 10⁹ cells/kg body weight, such as between 10⁵ and 10⁶ cells/kg body weight, for example, at least or at least about or being at or about 1×10⁵ cells/kg, 1.5×10⁵ cells/kg, 2×10⁵ cells/kg or 1×10⁶ cells/kg body weight. For example, in some embodiments, the cells are administered being at or about 10⁴ and being at or about 10⁹ T cells/ kg body weight or within a certain margin of error, such as between 10⁵ and 10⁶ T cells/kg body weight, such as at least or at least about or being at or about 1×10⁵ T cells/kg, 1.5×10⁵ T cells/kg, 2×10⁵ T cells/kg, or 1×10⁶ T cells/kg body weight.

Cells can be administered in any suitable way, e.g., by bolus, by injection, such as intravenous or subcutaneous injection, intraocular injection, periocular injection, subretinal injection, intravitreal injection, transseptal injection, subscleral injection, intrachoroidal injection, anterior intrachamber injection, subperineal injection, subconjunctival injection, sub-tenon injection, retrobulbar injection, peribulbar injection, or posterior juxtascleral. In some embodiments, they are administered parenteral, intrapulmonary and intranasal, and if local treatment is desired, by intralesional administration. Parenteral infusions include intramuscular, intravenous, intraarterial, intraperitoneal, or subcutaneous administration. In some embodiments, a given dose is administered by administering cells in a single bolus. In some embodiments, it is administered by multiple boluses of administered cells, for example, over a period of not more than 3 days, or by continuous infusion of administered cells.

In some embodiments, a repeated-dose method is provided, wherein a first dose of cells is administered, followed by one or more second consecutive doses. When administered to subjects with adoptive therapy, the timing and size of multiple doses of cells are usually designed to increase the efficacy and/or activity and/or function of antigen-expressing T cells (e.g., CAR-expressing T cells). In some embodiments, repeated administration reduces the down-regulation or inhibitory activity that can occur when inhibitory immune molecules such as PD-1 and/or PD-L1 are upregulated on antigen-expressing T cells, e.g., CAR-expressing T cells. The method includes administering the first dose, usually followed by one or more consecutive doses, and having a specific time frame between different doses.

In the context of adoptive cell therapy, the administration of a given "dose" includes administration of a given amount or number of cells as a single composition and/or a single uninterrupted administration (e.g., as a single injection or continuous infusion), and also includes the administration of a given amount or number of cells provided by a plurality of individual compositions or infusions as fractional doses for a specified period of time (not exceeding 3 days). Thus, in some cases, the first or continuous dose is a single or continuous administration of a specified number of cells given or initiated at a single time point. However, in some cases, the first or consecutive dose is administered in multiple injections or infusions over a period of no more than three days, such as once every three or two days, once a day, or multiple infusions over the course of a day.

### 3. Targeted degradation of pathogenic protein

In another aspect, the present disclosure also provides a method for degrading a target protein in vivo or in vitro, which includes delivering a nucleic acid molecule and/or vector as described herein to a cell expressing the target protein or a subject, wherein the nucleic acid molecule and/or the vector expressed in the cell or subject would produce a chimeric protein construct as described herein. The targeted protein binding domain of the chimeric protein construct is able to bind to the target protein, and the ERAD mechanism protein binding domain of the chimeric protein construct can use the ERAD pathway to induce the spatial proximity of the ubiquitin-proteasome system (UPS), thereby using the proteasome in the UPS to degrade the target protein. When the chimeric protein construct further contains the protein degradation pathway member binding domain, this method will induce additional degradation systems to get close to the target protein, resulting in a significant improvement in degradation effect.

In another aspect, the present disclosure also provides a method for treating the condition or disease of the subject in need thereof and/or preventing its recurrence, including: administering a therapeutically effective amount of the pharmaceutical composition described above (including nucleic acid molecules, e.g., mRNA, viral vectors, oncolytic viruses, chimeric protein constructs, etc.) to the subject.

In certain embodiments, the method includes introducing the nucleic acid molecule (e.g., mRNA) or vector (e.g., viral vector) encoding the chimeric protein construct into the individual in need thereof so that the nucleic acid molecule or vector expresses the chimeric protein construct in the cells of the individual.

Any of the conventional administration routes are applicable in the context of the invention including parenteral, topical or mucosal routes. The parenteral route is injection or infusion and includes systemic as well as local routes. Common parenteral injection types are intravenous (into a vein), intra-arterial (into an artery), intradermal (into the dermis), subcutaneous (under the epidermis), intramuscular (into muscle) and intratumoral (into tumor or at its close proximity). Infusions typically are given by intravenous route. Mucosal administrations include but not limited to oral/alimentary, intranasal, intratracheal, intrapulmonary, intravaginal or intra-rectal route. Topical administration can also be performed using transdermal means (e.g. patch and the like). Administrations may use conventional syringes and needles or any compound or device available in the art capable of facilitating or improving delivery of the active agent(s) in the subject.

Depending on the disease that the individual needs to treat, as well as the pathogenic protein that needs to be degraded, an appropriate chimeric protein construct and its nucleic acid molecules and vectors may be selected.

In some preferred embodiments, the disease comprises various solid tumors and hematological tumors, infectious diseases (e.g., viral infections), autoimmune diseases, neurodegenerative diseases, metabolic diseases.

In some embodiments, the disease is a tumor or cancer, and the targeting domain of the chimeric protein construct comprises a specific targeting tumor-associated target or an immune function-associated target. The tumor-associated target or immune function-associated target is as described earlier in the present application.

In some preferred embodiments, the solid tumor is selected from nervous system tumors, head and neck tumors, chest tumors, digestive system tumors, genitourinary system tumors, soft tissue and skin tumors, bone tumors, etc.

In some preferred embodiments, nervous system tumors include diffuse glioma, diffuse astrocytoma and anaplastic astrocytoma, glioblastoma, oligodendroglioma, oligoastrocytoma, diffuse Gliomas in children, other astrocytomas, ependymomas, neuronal and mixed neuronal-glial tumors, medulloblastoma, other embryonal tumors, schwannomas, meningiomas, solitary fibrous tumors and hemangiopericytoma, etc.

In some preferred embodiments, head and neck tumors comprise malignant tumors of the nasal cavity and sinuses, nasopharyngeal cancer, oral cancer, laryngeal cancer, salivary gland tumors, intracranial tumors, thyroid cancer, tongue cancer, etc.

In some preferred embodiments, thoracic tumors comprise lung cancer, esophageal cancer, cardia cancer, breast cancer, mediastinal tumors, etc.

In some preferred embodiments, digestive system tumors comprise gastric cancer, colorectal cancer, sigmoid colon and rectal cancer, liver cancer, pancreatic cancer and periampullary cancer, biliary tract cancer, malignant tumors of the small intestine, etc.

In some preferred embodiments, the genitourinary system tumors comprise kidney cancer, prostate cancer, bladder cancer, testicular cancer, penile cancer, cervical cancer, endometrial cancer, ovarian cancer, etc.

In some preferred embodiments, soft tissue and skin tumors comprise malignant fibrous histiocytoma, rhabdomyosarcoma, synovial sarcoma, malignant melanoma of the skin, etc.

In some preferred embodiments, bone tumors comprise osteosarcoma, Ewing's sarcoma, etc.

In some preferred embodiments, the colon cancer is a colon adenoma.

In some preferred embodiments, the breast cancer is a triple-negative breast cancer cell.

In some preferred embodiments, the liver cancer is hepatocellular carcinoma.

In some preferred embodiments, the disease is a hematological tumor selected from leukemia, lymphoma (HL), multiple myeloma (MM), myelodysplastic syndrome (MDS), etc.

In some preferred embodiments, the leukemia is B-cell acute lymphoblastic leukemia, T-cell acute lymphoblastic leukemia, acute myeloid leukemia, etc.

In another aspect, the present disclosure also provides a method of treating tumors or cancers of subjects in need thereof, comprising: administering a therapeutical effective amount of the oncolytic virus or oncolytic virus pharmaceutical composition of the present application to the subject. Oncolytic viruses or compositions of the present invention may be administered at a single dose or multiple doses to the subject. If multiple doses are administered, they can be administered through the same or different pathways, and can be administered at the same part or in different parts. It can also be administered in a continuous cycle, repeated after the rest period. The interval between each administration can range from a few hours to a year (e.g. 24 h, 48 h, 72 h, weekly, biweekly, monthly or yearly). Intervals can also be irregular (e.g., after tumor development). The dose of each administration can vary within the above ranges. In certain embodiments, the path of administration of oncolytic virus may include intravenous and intratumoral pathways.

In the case of the present invention, the oncolytic virus may be administered one or more times at a suitable dose (e.g., 10⁷ to 5×10⁹pfu). The interval between each viral administration can vary from about 1 day to about 8 weeks. Another preferred treatment protocol involves 2 to 5 (e.g., 3) intravenous or intratumoral administration of 10⁸ or 10⁹Pfu oncolytic virus at approximately 1 or 2 week intervals, which can be chosen by the physician based on the actual situation. In some embodiments, the method comprises administering an oncolytic viral vector comprising a nucleic acid molecule as described above, wherein the subject has cancer. The pharmaceutical composition or oncolytic viral vector comprising a nucleic acid molecule as described above is capable of targeted degradation of pathogenic proteins, e.g., oncogenic proteins, viral proteins, immune-related proteins, etc.

In some embodiments, the disease is an infectious disease, and the targeting domain of the chimeric protein construct contains a specific targeting infectious disease associated targets. The infectious disease associated targets are described earlier in the present application.

In some embodiments, viral infectious diseases comprise: respiratory viral diseases, gastrointestinal viral diseases, liver viral diseases, skin and mucous membrane viral diseases, ocular viral diseases, central nervous system viral diseases, lymphocytic viral diseases, Insect-borne viral diseases, lentivirus infection diseases, etc.

In some embodiments, respiratory viral diseases comprise infections of rhinovirus, adenovirus, respiratory syncytial virus, parainfluenza virus, and coronavirus; influenza; mumps, etc.

In some embodiments, gastrointestinal viral diseases comprise polio; Cooksackie virus infection; ECHO virus infection; and viral gastroenteritis including rotavirus gastroenteritis, Norwalk virus gastroenteritis, adenovirus gastroenteritis, astrovirus gastroenteritis, coronavirus gastroenteritis and calicivirus gastroenteritis, etc.

In some embodiments, viral diseases of the liver comprise viral hepatitis A, viral hepatitis B, viral hepatitis C, viral hepatitis D, viral hepatitis E, Epstein-Barr viral hepatitis, and cytomegalovirus hepatitis.

In some embodiments, viral diseases of the skin and mucous membranes comprise measles, rubella, infantile acute rash, chickenpox and herpes zoster, smallpox, herpes simplex virus infection, rabies and foot-and-mouth disease, etc.

In some embodiments, ocular viral diseases comprise epidemic keratoconjunctivitis, follicular conjunctivitis and herpetic keratoconjunctivitis, etc.

In some embodiments, viral diseases of the central nervous system comprise Japanese encephalitis, western equine encephalitis, eastern equine encephalitis, St. Louis encephalitis, Venezuelan equine encephalitis, Murray Valley encephalitis, Californian encephalitis, forest encephalitis and lymphocytic choroid plexus meningitis, etc.

In some embodiments, lymphocytic viral diseases comprise infectious mononucleosis, cytomegalovirus infection and acquired immunodeficiency syndrome, etc.

In some embodiments, the insect-borne viral diseases comprise: viral hemorrhagic fevers, including epidemic hemorrhagic fever, yellow fever, Crimean-Congo hemorrhagic fever, Rift Valley fever, Argentine hemorrhagic fever, Bolivian hemorrhagic fever, Lassa fever, Omu Scrubs hemorrhagic fever, Marburg disease and Ebola hemorrhagic fever, etc.; dengue fever and dengue hemorrhagic fever; West Nile fever; Colorado tick heat transfer; and sandfly fever, etc.

In some embodiments, lentiviral infection diseases comprise subacute sclerosing panencephalitis, Kuru disease, progressive multifocal leukoencephalopathy and subacute spongiform encephalopathy (corticostriatal spinal cord degeneration), etc.

In some embodiments, the disease is an autoimmune disease, and the targeting domain of the chimeric protein construct contains a target specifically targeting autoantigen-related targets. The autoantigen-related targets are described earlier in the present application.

In some embodiments, autoimmune diseases comprise organ-specific autoimmune diseases and systemic autoimmune diseases.

Preferably, organ-specific autoimmune diseases comprise chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritic syndrome, vulgaris Pemphigus, pemphigoid, primary biliary cirrhosis, multiple sclerosis, acute idiopathic polyneuritis, etc.

In some embodiments, systemic autoimmune diseases comprise systemic lupus erythematosus, rheumatoid arthritis, systemic vasculitis, scleroderma, pemphigus, dermatomyositis, mixed connective tissue disease, autoimmune hemolytic anemia, thyroid Autoimmune diseases, ulcerative colitis, etc.

In some embodiments, the disease is a neurological or degenerative disease, and the targeting domain of the chimeric protein construct comprises a target specifically targeting neurological disease related targets. The targets associated with neurological disorders are described earlier in the present application.

In some embodiments, neurological diseases comprise nervous system diseases, peripheral nerve diseases such as trigeminal neuralgia, facial paralysis, hemifacial spasm, vestibular neuronitis, glossopharyngeal neuralgia, mononerve disease, brachial plexus neuralgia, multiple mononeuropathy, multiple Neuropathy, acute inflammatory demyelinating polyneuropathy, chronic inflammatory demyelinating polyneuropathy;
spinal cord diseases, e.g., myelitis, compressive myelopathy, subacute combined degeneration of the spinal cord, syringomyelia, spinal cord vascular disease, spinal cord arachnoiditis, etc.;
cerebrovascular diseases, e.g., transient ischemic attack, cerebral infarction, cerebral hemorrhage, subarachnoid hemorrhage, intracranial venous system thrombosis, etc.;
infectious diseases of the central nervous system, e.g., meningitis, encephalitis, etc. caused by infections of viruses, bacteria, fungi, or parasites, etc, and lentiviral encephalitis caused by lentiviral infections;
central nervous system demyelinating diseases, e.g., multiple sclerosis, neuromyelitis optica, acute disseminated encephalomyelitis, leukodystrophy, etc.;
movement disorders, e.g., Parkinson's disease, chorea, hepatolenticular degeneration, dystonia, essential tremor, tardive dyskinesia, etc.;
epilepsy;
headaches, e.g., migraines, tension headaches, cluster headaches, etc.;
nervous system degenerative diseases, e.g., motor neuron disease, Alzheimer's disease, Lewy body dementia, frontotemporal dementia, multiple system atrophy, etc.;
inherited nervous system diseases such as hereditary ataxia, hereditary spastic paraplegia, Charcot-Marie-Tooth disease, neurofibromatosis, tuberous sclerosis, cerebral hemangiomatosis, etc.;
neurodevelopmental disorders such as congenital hydrocephalus, cerebral palsy, basilar depression, cerebellar subtonsillar disease, etc.;
neuromuscular junction and muscle diseases, e.g., myasthenia gravis, periodic paralysis, polymyositis, progressive muscular dystrophy, myotonic myopathy (myotonic dystrophy, myotonia congenita), metabolic Myopathy (mitochondrial myopathy, lipid storage myopathy, glycogenosis), etc.;
autonomic nervous system diseases, e.g., Raynaud's disease, erythromelalgia, hemifacial atrophy, generalized autonomic insufficiency, spontaneous hyperhidrosis, progressive lipodystrophy, etc.;
nervous system tumors, e.g., glioma, lymphoma, meningioma, etc.;
pparaneoplastic syndromes of the nervous system, e.g., paraneoplastic cerebellar degeneration, paraneoplastic encephalomyelitis, subacute necrotizing myelopathy, subacute motor neuron disease, paraneoplastic sensory neuron disease, etc.;

In some preferred embodiments, neurodegenerative diseases lead to a gradual loss of neuronal structure and function, including neuronal death and glial cell balance, leading to cognitive impairment such as dementia. Examples of neurodegenerative diseases include Alzheimer's disease, Parkinson's disease (PD), Huntington's disease, early-onset AD or PD, and amyotrophic lateral sclerosis (ALS).

In some embodiments, the disease is a metabolic disease, such as diabetes, hyperlipidemia, gout, etc. The targets associated with metabolic diseases are described earlier in the present application.

### Examples

### Example 1: Design of ER-TPD chimeric protein construct

In this example, five chimeric protein constructs based on the Endoplasmic Reticulum-based Targeted Protein Degradation (ER-TPD) technology were designed. Among them, the first structure is the basic ER-TPD chimeric protein construct, and the other four structures are more powerful ER-TPD chimeric protein constructs formed by adding E3 ubiquitin ligase ligand, E2 ubiquitin-conjugating enzyme ligand or lysozyme ligand or the combination thereof to the first structure. The ER-TPD chimeric protein construct comprises an integrated multifunctional domain or functional variant thereof of a recombinant protein molecule that binds to a target protein and induces targeted degradation.

### 1.1 Basic ER-TPD chimeric protein construct

In some embodiments, the basic ER-TPD chimeric protein construct TBD-TMD-ERD is used, its linear schematic diagram and structural schematic diagram are shown in Fig. 1A, which contains three domains: (1) a target protein binding domain (TBD) or a functional variant thereof, which may be a targeted affinity ligand protein molecule or a functional variant thereof (which may be an antibody fragment, polypeptide, natural receptor, ligand, or an artificial recombinant protein having affinity to the target protein, or a binding domain thereof); (2) a transmembrane domain (TMD) of viral endoplasmic reticulum (ER) resident glycoprotein, or a functional variant thereof; and (3) a cytoplasmic domain of viral endoplasmic reticulum (ER) resident glycoprotein (ER retention domain, ERD), or a functional variant thereof. Optionally, the target protein binding domain or the functional variant thereof, the transmembrane domain of viral endoplasmic reticulum resident glycoprotein or the functional variant thereof, and the cytoplasmic domain of viral endoplasmic reticulum resident glycoprotein or the functional variant thereof are linked by a hinge or linker.

As shown in Fig. 1B, the working mechanism of ER-TPD chimeric protein construct TBD-TMD-ERD is as follow: [1] Specific binding and retention: TBD-TMD-ERD resides in the endoplasmic reticulum after being expressed and binds to the target protein through the target protein binding domain, resulting in the retention of target protein inside the endoplasmic reticulum and preventing the translocation of the synthesized target protein to the Golgi apparatus. [2] Retrotranslocation: After the chimeric protein construct binds to the target protein, the complex formed by the chimeric protein construct and the target protein is translocated from the endoplasmic reticulum to the cytoplasm through the retrotransporter in the ERAD mechanism. Classical retrotransporters include Derlin, E3 ligase Hrd1 and the chaperone Sel1L, which are able to recruit ATPase p97 from the cytoplasm. Derlin has six transmembrane domains, of which the N-terminal and C-terminal are located in the cytoplasm and form a transport channel, ATPase p97 is required for the retrotranslocation process (Byun H et al., ERAD and how viruses exploit it. Front Microbiol. 2014 Jul 3; 5:330). [3] Ubiquitination and proteasome degradation: retrotransporters can interact with the E3 ubiquitin ligase intrinsic to the endoplasmic reticulum to promote the ubiquitination of target proteins, the ubiquitinated target proteins are translocated to the cytoplasm through the retrotransporters and are recognized and degraded by the proteasome.

### 1.2 ER-TPD chimeric protein construct having E3 ubiquitin ligase ligand domain

In some embodiments, the ER-TPD chimeric protein construct TBD-TMD-ERD-E3L having an E3 ubiquitin ligase ligand domain is used, its linear schematic diagram and structural schematic diagram are shown respectively in Fig. 2A and 2B, which contains four domains: (1) a target protein binding domain (TBD) or a functional variant thereof, which may be a targeted affinity ligand protein molecule or a functional variant thereof (which may be an antibody fragment, polypeptide, natural receptor, ligand, or an artificial recombinant protein domain having affinity to the target protein); (2) a transmembrane domain of viral endoplasmic reticulum resident glycoprotein, or a functional variant thereof; (3) a cytoplasmic domain of viral endoplasmic reticulum resident glycoprotein, or a functional variant thereof; and (4) an E3 ubiquitin ligase ligand (ligand for E3 ligase, E3L), or a functional variant thereof, the E3 ubiquitin ligase ligand may be a polypeptide or a recombinant protein for linking with the large number of E3 ubiquitin ligase outside the endoplasmic reticulum. Optionally, the target protein binding domain or the functional variant thereof, the transmembrane domain of viral endoplasmic reticulum resident glycoprotein or the functional variant thereof, the cytoplasmic domain of viral endoplasmic reticulum resident glycoprotein or the functional variant thereof, and the E3 ubiquitin ligase ligand domain or the functional variant thereof are linked by a hinge or linker. Because of the limited number of E3 ubiquitin ligases intrinsic to the ER, and also because of the competition for E3 ubiquitin ligases from the ERAD pathway triggered by misfolded proteins in the ER, it is not possible to achieve the efficient degradation of target proteins if the use of ubiquitin-proteasome degradation pathway is not increased. The inventors thus found that the efficient ubiquitination and degradation of the target protein can be achieved by exogenously increasing the E3 ubiquitin ligase ligand.

As shown in Fig. 2C, the working mechanism of ER-TPD chimeric protein construct TBD-TMD-ERD-E3L is as follow: [1] Specific binding and retention: TBD-TMD-ERD-E3L resides in the endoplasmic reticulum after being expressed and binds to the target protein through the target protein binding domain, resulting in the retention of target protein inside the endoplasmic reticulum and preventing the translocation of the synthesized target protein to the Golgi apparatus. [2] Retrotranslocation: After the chimeric protein construct binds to the target protein, the complex formed by the chimeric protein construct and the target protein is translocated from the endoplasmic reticulum to the cytoplasm through the retrotransporter in the ERAD mechanism. Classical retrotransporters include Derlin, E3 ligase Hrd1 and the chaperone Sel1L, which are able to recruit ATPase p97 from the cytoplasm. Derlin has six transmembrane domains, of which the N-terminal and C-terminal are located in the cytoplasm and form a transport channel, ATPase p97 is required for the retrotranslocation process. [3] Ubiquitination and proteasome degradation: the chimeric protein construct binds to the E3 ligase complex by the E3 ligase ligand, the target protein is ubiquintinated by the E2 ubiqutin conjugating enzyme in the complex and translocated to the proteasome for degradation. Among them, the ligand targeting E3 ligase complex may be an artificially screened and constructed protein sequence, or be a naturally existed protein sequence or the recombinant version thereof, such as the phosphopeptide derived from IκBα (DRHDSGLDSM, SEQ ID NO. 119), the phosphopeptide can recruit SCFβ-TRCP in the E3 ubiquitin ligase complex, so as to mediate the ubiquitination degradation pathway of the target protein (Sakamoto KM et al., Protacs: chimeric molecules that target proteins to the Skp1-Cullin-F box complex for ubiquitination and degradation.Proc Natl Acad Sci U S A.2001; 98:8554-9). It may also comprise the E3 ligase recognition peptide (ALAPYIP, SEQ ID NO. 120) derived from HIF-1α, such recognition peptide may bind to E3 ligase VHL, therby allowing for ubiquitination degradation pathway (Ivan M et al., HIFalpha targeted for VHL-mediated destruction by proline hydroxylation: implications for O2 sensing. Science.2001; 292:464-8).

### 1.3 ER-TPD chimeric protein construct having E2 ubiquitin conjugating enzyme ligand domain

In some embodiments, the ER-TPD chimeric protein construct TBD-TMD-ERD-E2L having an E2 ubiquitin conjugating enzyme ligand domain is used, its linear schematic diagram and structural schematic diagram are shown respectively in Fig. 3A and 3B, which contains four domains: (1) a target protein binding domain or a functional variant thereof, which may be a targeted affinity ligand protein molecule or a functional variant thereof (which may be an antibody fragment, polypeptide, natural receptor, ligand, or an artificial recombinant protein domain having affinity to the target protein); (2) a transmembrane domain of viral endoplasmic reticulum resident glycoprotein, or a functional variant thereof; (3) a cytoplasmic domain of viral endoplasmic reticulum resident glycoprotein, or a functional variant thereof; and (4) an E2 ubiquitin conjugating enzyme ligand domain, or a functional variant thereof, the E2 conjugating enzyme ligase ligand may be a polypeptide or a recombinant protein. Optionally, the target protein binding domain or the functional variant thereof, the transmembrane domain of viral endoplasmic reticulum resident glycoprotein or the functional variant thereof, the cytoplasmic domain of viral endoplasmic reticulum resident glycoprotein or the functional variant thereof, and the E2 ubiquitin conjugating enzyme ligand domain or the functional variant thereof are linked by a hinge or linker.

As shown in Fig. 3C, the working mechanism of ER-TPD chimeric protein construct TBD-TMD-ERD-E2L is as follow: [1] Specific binding and retention: TBD-TMD-ERD-E2L resides in the endoplasmic reticulum after being expressed and binds to the target protein through the target protein binding domain, resulting in the retention of target protein inside the endoplasmic reticulum and preventing the translocation of the synthesized target protein to the Golgi apparatus. [2] Retrotranslocation: After the chimeric protein construct binds to the target protein, the complex formed by the chimeric protein construct and the target protein is translocated from the endoplasmic reticulum to the cytoplasm through the retrotransporter in the ERAD mechanism. Classical retrotransporters include Derlin, E3 ligase Hrd1 and the chaperone Sel1L, which are able to recruit ATPase p97 from the cytoplasm. Derlin has six transmembrane domains, of which the N-terminal and C-terminal are located in the cytoplasm and form a transport channel, ATPase p97 is required for the retrotranslocation process. [3] Ubiquitination and proteasome degradation: the chimeric protein construct directly binds to the E2 conjugating enzyme by the E2 conjugating enzyme ligand, allowing the target protein to be ubiquintinated by the E2 ubiqutin conjugating enzyme and translocated to the proteasome for degradation. The E2 ubiquitin conjugating enzyme ligand domain can bind to the UBC structure of E2 ubiquitin conjugating enzyme by way of the RING zinc finger domain derived from the IAP family protein, so as to achieve the ubiquitination and proteasome degradation of the target protein. The ligand targeting the E2 ubiquitin conjugating enzyme may be a RING zinc finger domain derived from an IAP family protein or a recombinant protein sequence with a RING zinc finger domain. IAP (inhibitor of apoptosis proteins) is a family of apoptosis protein inhibitory molecules, IAP may function as the E3 ligase for pro-apoptotic proteins (such as Caspases, SMAC, ARTS), thereby inhibiting cellular apoptosis by achieving the ubiquitination and degradation of pro-apoptotic proteins. It may also regulate NOD signaling and immune response through the ubiquitylation of RIPK2 (receptor-interacting serine/threonine-protein kinase 2). The five members of IAP family (XIAP, cIAP1, cIAP2, Livin and ILP2) all have RING zinc finger domains, which can bind to ubiquitin conjugating enzyme E2 and Apollon protein (Mikihiko Naito et al., SNIPERs-Hijacking IAP activity to induce protein degradation. Drug Discov Today Technol. 2019 Apr; 31:35-42). Therefore, more efficient ubiquitination and proteasome-mediated degradation of target proteins can be achieved by using the RING zinc finger domain derived from IAP family members for connecting the chimeric protein construct and the ubiquitin-conjugating enzyme E2. In addition, the following sequences may be used for designing the E2 ligand:
the RING domain derived from XIAP: CKICMDRNIAIVFVPCGHLVTCKQCAEAVDKCPMCY (SEQ ID NO.155);
the RING domain derived from cIAP1: CKVCMDKEVSVVFIPCGHLVVCQECAPSLRKCPICR (SEQ ID NO.156);
the RING domain derived from cIAP2: CKVCMDKEVSVVFIPCGHLVVCQECAPSLRKCPICR (SEQ ID NO.157);
the RING domain derived from Livin: CKVCLDRAVSIVFVPCGHLVCAECAPGLQLCPICR (SEQ ID NO.158);
the RING domain derived from ILP-2: CKICMDRHIAVVFIPCGHLVTCKQCAEAVDRCPMCS (EQ ID NO. 159).

### 1.4 ER-TPD chimeric protein construct having lysosome ligand domain

In some embodiments, the ER-TPD chimeric protein construct TBD-TMD-ERD-LL having a lysosome ligand domain is used, its linear schematic diagram and structural schematic diagram are shown respectively in Fig. 4Aand 4B, which contains four domains: (1) a target protein binding domain or a functional variant thereof, which may be a targeted affinity ligand protein molecule or a functional variant thereof (which may be an antibody fragment, polypeptide, natural receptor, ligand, or an artificial recombinant protein domain having affinity to the target protein); (2) a transmembrane domain of viral endoplasmic reticulum resident glycoprotein, or a functional variant thereof; (3) a cytoplasmic domain of viral endoplasmic reticulum resident glycoprotein, or a functional variant thereof; and (4) a lysosome ligand domain, or a functional variant, the lysosome ligand may be a polypeptide or a recombinant protein. Optionally, the target protein binding domain or the functional variant thereof, the transmembrane domain of viral endoplasmic reticulum resident glycoprotein or the functional variant thereof, the cytoplasmic domain of viral endoplasmic reticulum resident glycoprotein or the functional variant thereof, and the lysosome ligand domain or the functional variant thereof are linked by a hinge or linker.

As shown in Fig. 4C, the working mechanism of ER-TPD chimeric protein construct TBD-TMD-ERD-LL is as follow: [1] Specific binding and retention: TBD-TMD-ERD-LL expresses and resides in the endomembrane of endoplasmic reticulum, and binds to the target protein through the target protein binding domain, resulting in the retention of target protein inside the endoplasmic reticulum and preventing the translocation of the synthesized target protein to the Golgi apparatus. [2] Retrotranslocation: After the chimeric protein construct binds to the target protein, the complex formed by the chimeric protein construct and the target protein is translocated from the endoplasmic reticulum to the cytoplasm through the retrotransporter in the ERAD mechanism. [3] Lysosome endocytosis and hydrolysis: The chimeric protein construct directly binds to the lysosomal chaperone through the lysosome ligand domain, and is translocated to the lysosome by way of the chaperone. The target protein is degraded by various hydrolases in the lysosome.

The lysosome ligand domain may be a KFERQ-like motif or a recombinant protein sequence with a KFERQ-like motif. The KFERQ-like motif can be specifically recognized and bound by the chaperone Hsc70, allowing the target protein ER-TPD complex to be translocated to lysosome by way of the chaperone mediated autophagy (CMA) mechanism and degraded by various hydrolases therein.

Chaperone mediated autophagy (CMA) is a process of selective protein autophagy and lysosomal degradation. The chaperone Hsc70 transports the target protein to the lysosome for degradation by recognizing and targeting the KFERQ-like motif on the target protein and by recognizing the receptor protein LAMP2A on the lysosomal membrane. The KFERQ-like motif may be KFERQ derived from RNaseA (Backer J et al., Regulation of catabolism of microinjected ribonuclease A requires the amino-terminal 20 amino acids. Proc Natl Acad Sci. 1983; 80:2166-2170), QKILD (SEQ ID NO. 124) and QRDKV (SEQ ID NO. 125) derived from Hsc70 (Cuervo AM et al., Unique properties of lamp2a compared to other lamp2 isoforms. J Cell Sci.2000; 113(Pt 24):4441-4450); QRFFE (SEQ ID NO. 126) derived from hemoglobin (Slot LA et al., Intracellular protein degradation in serum-deprived human fibroblasts. Journal of Biochemistry. 1986; 237:491-498); QKKEL (SEQ ID NO.127), QFREL (SEQ ID NO.128) and IKLDQ (SEQ ID NO.129) derived from aldolase B; EFLKQ (SEQ ID NO.130) derived from Annexin I; QKVFD (SEQ ID NO.131) derived from Annexin II; QELLR (SEQ ID NO.132) derived from Annexin IV; QEFIK (SEQ ID NO.133) derived from Annexin VI; RKVEQ (SEQ ID NO.134) derived from aspartate aminotransferase; NLLKE derived from c-fos; NRVVD (SEQ ID NO.135) derived from GAPDH; NKKFE (SEQ ID NO.136) derived from glutathione transferase; VKELQ (SEQ ID NO.137) derived from IκB; VDKLN (SEQ ID NO.138) and RIKEN (SEQ ID NO.139) derived from α-2- microglobulin; DVVRQ (SEQ ID NO.140), QRIVE (SEQ ID NO.141) and QLLRE (SEQ ID NO.142) derived from Pax-2; IEKLQ (SEQ ID NO.143) derived from 26S proteasome (C8); QEKVF (SEQ ID NO.144) derived from 19S proteasome (PA28); QDLKF (SEQ ID NO.145) derived from pyruvate kinase; VKKDQ (SEQ ID NO.146) derived from α-synuclein (Massey AC.et al., Chaperone-Mediated Autophagy in Aging and Disease.Current Topics in Developmental Biology,2006; 73:205-235).

### 1.5 ER-TPD chimeric protein construct having E3 ubiquitin ligase and lysosome ligand domain

In some embodiments, the ER-TPD chimeric protein construct TBD-TMD-ERD-E3L-LL having a combination of E3 ubiquitin ligase ligand and lysosome ligand is used, its linear schematic diagram and structural schematic diagram are shown respectively in Fig. 5A and 5B, which contains five domains: (1) a target protein binding domain or a functional variant thereof, which may be a targeted affinity ligand protein molecule or a functional variant thereof (which may be an antibody fragment, polypeptide, natural receptor, ligand, or an artificial recombinant protein domain having affinity to the target protein); (2) a transmembrane domain of viral endoplasmic reticulum resident glycoprotein, or a functional variant thereof; (3) a cytoplasmic domain of viral endoplasmic reticulum resident glycoprotein, or a functional variant thereof; (4) an E3 ubiquitin ligase ligand (ligand for E3 ligase, E3L), or a functional variant thereof; and (5) a lysosome ligand (Ligand for lysosome), or a functional variant. Optionally, the target protein binding domain or the functional variant thereof, the transmembrane domain of viral endoplasmic reticulum resident glycoprotein or the functional variant thereof, the cytoplasmic domain of viral endoplasmic reticulum resident glycoprotein or the functional variant thereof, the E3 ubiquitin ligase ligand or the functional variant thereof, and the lysosome ligand domain or the functional variant thereof are linked by a hinge or linker.

As shown in Fig. 5C, the working mechanism of ER-TPD chimeric protein construct TBD-TMD-ERD-E3L-LL is as follow: [1] Specific binding and retention: TBD-TMD-ERD-E3L-LL resides in the endoplasmic reticulum after being expressed and binds to the target protein through the target protein binding domain, resulting in the retention of target protein inside the endoplasmic reticulum and preventing the translocation of the synthesized target protein to the Golgi apparatus. [2] Retrotranslocation: After the chimeric protein construct binds to the target protein, the complex formed by the chimeric protein construct and the target protein is translocated from the endoplasmic reticulum to the cytoplasm through the retrotransporter in the ERAD mechanism. [3] Lysosome endocytosis and enzymatic degradation as well as ubiquitination/proteasome degradation: The chimeric protein construct is translocated outside of the endoplasmic reticulum, and directly binds to the lysosomal chaperone through the lysosome ligand domain, and is translocated into the lysosome by way of the chaperone. The target protein is degraded by various hydrolases in the lysosome, and some of the chimeric protein is translocated outside of the endoplasmic reticulum. The chimeric protein construct can bind to the E3 ligase complex by the E3 ligase ligand, and be translocated to proteasomes for degradation since the target protein can be ubiquitinized by the E2 ubiquitin conjugating enzyme in the complex.

### Example 2: Design of ER-TPD chimeric nucleic acid construct targeting TCR

In this example, two TCR-targeted ER-TPD chimeric nucleic acid constructs were designed.

In some embodiments, two TCR-targeted ER-TPD chimeric protein constructs TCR-ER-TPD were designed, as shown in Fig. 6, by using a single lentiviral expression vector and an EF1α promoter:
(A) TCR-TPD1: anti-TCR-E3/19K TMD-ERD-P2A-EGFRt (Fig. 6A);
(B) TCR-TPD2: anti-TCR-E3/19K TMD-ERD-G4S-E3L-P2A-EGFRt (Fig. 6B).

### Example 3: Preparation of expression plasmid of TCR-targeted ER-TPD chimeric protein construct

Whole gene synthesis was carried out for the coding nucleotide sequences of the TCR-targeted ER-TPD chimeric protein construct TCR-ER-TPD (TCR-TPD1 and TCR-TPD2) designed in Example 2, wherein the nucleotide sequence of TCR-TPD1 is shown in SEQ ID NO. 160, the encoded protein thereof is shown in SEQ ID NO. 161, the nucleotide sequence of TCR-TPD2 is shown in SEQ ID NO. 162, and encoded protein thereof is shown in SEQ ID NO.163 . The nucleotide sequence of TCR-TPD1 and the nucleotide sequence of TCR-TPD2 were respectively inserted into the lentiviral vector pCDH-CMV-MCS-EF1α-Puro (purchased from Youbao Biotech) by molecular cloning to construct TCR-ER-TPD lentiviral target vector. The obtained plasmids (which were confirmed by sequencing) were used to transform DH5α competent cells (Thermo Fisher) respectively. Single colonies were picked and cultured in large-scale. Then, the plasmids were purified and produced by using the PureLink^{™} HiPure Plasmid Maxiprep Kit (Thermo Fisher), to obtain the TCR-ER-TPD lentiviral plasmids, i.e., the TCR-TPD1 lentiviral plasmid and the TCR-TPD2 lentiviral plasmid.

### Example 4: Preparation of virus of TCR-targeted ER-TPD chimeric protein construct

293T cells (provided by ATCC, Cat# CRL3216TM) were co-transfected by the TCR-ER-TPD lentiviral plasmid obtained in Example 3 as well as the packaging plasmids psPAX2 and pMD2.G (purchased from Addgene, Cat# 12259&12260) at the ratio of 1.64pmol: 1.3pmol: 0.72pmol. Polyethyleneimine (408727, Sigma) was used as the transfection reagent, at the ratio of DNA: µg PEI=1:3. Other details of the transfection process can be found in the instruction of PureLink^{™} HiPure Plasmid Maxiprep Kit (K210006, Thermo). 16 hours after transfection, culture medium was changed to the complete medium (purchased from Life Technologies, Cat# 11995-065). After culturing for 24 hours, 48 hours and 72 hours, the supernatants with lentivirus were collected, combined, and centrifuged at -80°C, 3000rpm for 10-15 minutes, then filtered through a 0.45µm filter membrane, and finally ultra-centrifuged at 25000rpm, 4°C for 2-3 hours, so as to obtain the concentrate of lentivirus. The concentrates of TCR-ER-TPD lentivirus (TCR-TPD1 lentivirus or TCR-TPD2 lentivirus) were collected and stored at -80°C.

### Example 5: Preparation of TCR ER-TPD Jurkat cells

The resuscitated Jurkat cells were cultured in RMPI1640 medium supplemented with 5% FBS, in a 37°C, 5% CO₂ incubator. The medium was changed every 2-3 days, and the cell density was maintained at 1 × 10⁶ single cells/ml. After the cell state was recovered, the recovered cells were transfected with the lentivirus loaded with the TCR-ER-TPD lentiviral plasmid prepared in Example 4. The detailed steps are as follows: Dilute the Jurkat cells to a concentration of 0.5 × 10⁶ single cells/ml, spread them in a 24-well plate, and add the TCR-TPD1 lentivirus and TCR-TPD2 lentivirus concentrates prepared in Example 4 to the cells (MOI = 1), add Polybrene 5µg/ml as well, centrifuge at a low speed (500g-1000g/min) for 30-60 minutes in a flat-angle centrifuge, and then culture the cells in an incubator at 37°C. Jurkat cells expressing TCR-ER-TPD (TCR ER-TPD Jurkat cells) were obtained 48 hours after infection, the cell phenotype was determined by flow cytometry. The phenotype measurement and the cellular function experiment can be carried out after further culturing for 96h and 6 days, respectively.

### Example 6: Phenotype measurement of ER-TPD transfected cells

Flow cytometry was performed on the TCR ER-TPD Jurkat cells prepared in Example 5. Fig. 7 shows the cellular phenotyping results of TCR ER-TPD Jurkat cells at 48h (A), 96h (B) and Day 6 (C). In the process of cell culture and flow cytometry, 5 × 10⁵ untransfected Jurkat cells and TCR-ER-TPD lentiviral transfection vector transfected Jurkat cells were cultured respectively, and treated with antibodies CD8-Pacific Blue, CD4-APC, EGFR-PE, TCR-αβ-APC-Cy7 (all antibodies were purchased from Biolegend) for staining, followed by cell phenotyping by a flow cytometer (BD FACSCanto II). The results showed that (A) After 48 hours of culture, untransfected Jurkat cells expressed TCR-αβ but not EGFR; in Jurkat cells altered by TCR-TPD1 lentivirus transfection, EGFR was highly expressed, TCR-αβ began to degrade with a decreased expression level on cell membrane; in Jurkat cells altered by TCR-TPD2 lentivirus transfection, EGFR was highly expressed, the decrease of the expression level of TCR-αβ on the cell membrane was significantly higher than that of cells transfected with TCR-TPD1 lentivirus; (B) After 96 hours of culture, in both Jurkat cells transfected with the TPD virus, the TCR on the cell surface decreased, the decrease of the TCR expression level in a case of TCR-TPD2 being significantly lower than that of cells transfected with TCR-TPD 1 lentivirus; (C) After 6 days of culture, in both Jurkat cells transfected with the TPD virus, the TCR on the cell surface decreased, the decrease of the TCR expression level in a case of TCR-TPD2 being significantly lower than that of cells transfected with TCR-TPD1 lentivirus.

### Example 7: Design of BLA-targeted ER-TPD chimeric nucleic acid construct

In this example, two HLA-targeted ER-TPD chimeric nucleic acid constructs were designed.

In some embodiments, three expression vectors were designed by using a single lentiviral expression vector and an EF1α promoter, as shown in Fig. 6:
(A) CNK: DAP10-CD3ζ-T2A-NKG2D (Fig. 8A);
(B) HLA-TPD: E3/19K-E3L (Fig. 8B);
(C) CNK-HLA-TPD: DAP10-CD3ζ-T2A-NKG2D-p2A-E3/19K-E3L (Fig. 8C).
   Wherein, HLA-TPD is the HLA-targeted ER-TPD chimeric protein construct HLA-ER-TPD; CNK-HLA-TPD is the HLA-targeted ER-TPD chimeric protein construct CNK-HLA-ER-TPD.

### Example 8: Preparation of expression vector of HLA-targeted ER-TPD chimeric protein construct

Whole gene synthesis was carried out for the coding nucleotide sequences of the HLA-targeted ER-TPD chimeric protein construct designed in Example 7 (the nucleotide sequence of E3/19K-E3L is shown in SEQ ID NO. 164, the encoded protein thereof is shown in SEQ ID NO. 165, the nucleotide sequence of DAP10-CD3ζ-T2A-NKG2D-p2A-E3/19K-E3L is shown in SEQ ID NO. 166, and encoded protein thereof is shown in SEQ ID NO. 167). Whole gene synthesis was carried out for the nucleotide sequences of the CNK designed in Example 7 (the nucleotide sequence is shown in SEQ ID NO. 168; the encoded protein is shown in SEQ ID NO. 169). The coding nucleotide sequences of the HLA-targeted ER-TPD chimeric protein construct and the nucleotide sequences of the CNK were respectively inserted into the lentiviral vector pCDH-CMV-MCS-EF1α-Puro (purchased from Youbao Biotech) by molecular cloning to construct HLA-TPD lentiviral target vector, CNK-HLA-TPD lentiviral target vector and CNK lentiviral target vector. The obtained plasmids (which were confirmed by sequencing) were used to transform DH5α competent cells (Thermo Fisher) respectively. Single colonies were picked and cultured in large-scale. Then, the plasmids were purified and produced by using the PureLinkTM HiPure Plasmid Maxiprep Kit (Thermo Fisher), to obtain the TCR-ER-TPD lentiviral plasmids (the HLA-TPD lentiviral plasmid, the CNK-HLA-TPD lentiviral plasmid) and the CNK lentiviral plasmid.

### Example 9: Preparation of virus of HLA-targeted ER-TPD chimeric protein construct

293T cells (provided by ATCC, Cat# CRL3216TM) were co-transfected by the TCR-ER-TPD lentiviral plasmid and CNK lentiviral plasmid obtained in Example 8 as well as the packaging plasmids psPAX2 and pMD2.G (purchased from Addgene, Cat# 12259& 12260) at the ratio of 1.64pmol: 1.3pmol: 0.72pmol. Polyethyleneimine (408727, Sigma) was used as the transfection reagent, at the ratio of DNA:µg PEI=1 :3. The details for the preparation of the packaging plasmids can be found in the instruction of PureLinkTM HiPure Plasmid Maxiprep Kit (K210006, Thermo), other details of the transfection process can be found in the instruction of Sigma Transfections. 16 hours after transfection, culture medium was changed to the complete medium (purchased from Life Technologies, Cat# 11995-065). After culturing for 24 hours, 48 hours and 72 hours, the supernatants with lentivirus were collected, combined, and centrifuged at -80°C, 3000rpm for 10-15 minutes, then filtered through a 0.45µm filter membrane, and finally ultra-centrifuged at 25000rpm, 4°C for 2-3 hours, so as to obtain the concentrate of lentivirus respectively. The HLA-TPD lentivirus concentrate, CNK-HLA-TPD lentivirus concentrate and CNK lentivirus concentrate were respectively collected and stored at -80°C.

### Example 10: Preparation of HLA ER-TPD 293 cells

The resuscitated 293 cells were cultured in DMEM medium supplemented with 5% FBS, in a 37°C, 5% CO₂ incubator. The medium was changed every 2-3 days, and the cell density was maintained at 80% confluent. After the cell state was recovered, the recovered cells were transfected with the lentivirus prepared in Example 9. The detailed steps are as follows: Dilute the 293T cells to a concentration of 0.5 × 10⁶ single cells/ml, spread them in a 24-well plate, add the lentivirus concentrate prepared in Example 9 to the cells (MOI = 1), add Polybrene 5µg/ml as well, centrifuge at a low speed (500g-1000g/min) for 30-60 minutes in a flat-angle centrifuge, then culture the cells in an incubator at 37°C. 293T cells expressing CNK, HLA-TPD or CNK-HLA-TPD were obtained 48 hours after infection, the cell phenotype was determined by flow cytometry.

Fig. 9 shows the 48h flow cytometry results for cellular phenotyping of 293 cells co-transfected with the HLA-abc-targeted ER-TPD chimeric nucleic acid construct and the CNK expression vector. The cultured 293T cells were transfected with the following virus respectively: (1) CNK lentivirus (DAP10-CD3ζ-T2A-NKG2D), (2) HLA-TPD lentivirus (E3/19K-E3L), and (3) CNK-HLA-TPD lentivirus (DAP10-CD3ζ-T2A-NKG2D-p2A-E3/19K-E3L); untransfected cells were used as control. 48 hours after transfection, 5 × 10⁵ untransfected 293T cells and virus transfected cells were treated with antibodies HLA-abc-FITC, NKG2D-PE-Cy7 (all antibodies were purchased from Biolegend) for staining, followed by cell phenotyping by a flow cytometer (BD FACSCanto II). The results showed that the untransfected 293 cells express HLA-abc but notNKG2D; (1) 293T cells transfected with the CNK lentivirus express both HLA-abc and NKG2D; (2) 293T cells transfected with the HLA-TPD lentivirus express neither HLA-abc norNKG2D; (3) 293T cells transfected with the CNK-HLA-TPD lentivirus express NKG2D but not HLA-abc.

### Example 11: Design of CD123-targeted ER-TPD chimeric nucleic acid construct

In this example, CD123-targeted ER-TPD chimeric nucleic acid constructs were designed.

In some embodiments, the CD123-targeted ER-TPD chimeric nucleic acid constructs expressing anti-CD123 scFv-E3L were designed by using a single lentiviral expression vector and an EF1α promoter, as shown in Fig. 10.

### Example 12: Preparation of expression vector of CD123-targeted ER-TPD chimeric protein construct

Whole gene synthesis was carried out for the coding nucleotide sequences of the CD123-targeted ER-TPD chimeric protein construct designed in Example 11 (the nucleotide sequence of anti-CD123 scFv-E3L is shown in SEQ ID NO.170, the encoded protein thereof is shown in SEQ ID NO.171). The nucleotide sequence was inserted into the lentiviral vector pCDH-CMV-MCS-EF1α-Puro (purchased from Youbao Biotech) by molecular cloning to construct CD123-ER-TPD lentiviral target vector. The obtained plasmids (which were confirmed by sequencing) were used to transform DH5α competent cells (Thermo Fisher). Single colonies were picked and cultured in large-scale. Then, the plasmids were purified and produced by using the PureLinkTM HiPure Plasmid Maxiprep Kit (Thermo Fisher), to obtain the CD123 ER-TPD lentiviral plasmid.

### Example 13: Preparation of virus of CD 123-targeted ER-TPD chimeric protein construct

293T cells (provided by ATCC, Cat# CRL3216TM) were co-transfected by the CD123 ER-TPD lentiviral plasmid obtained in Example 12 as well as the packaging plasmids psPAX2 and pMD2.G (purchased from Addgene, Cat# 12259&12260) at the ratio of 1.64pmol: 1.3pmol: 0.72pmol. Polyethyleneimine (408727, Sigma) was used as the transfection reagent, at the ratio of DNA:µg PEI=1:3. The details for the preparation of the packaging plasmids can be found in the instruction of PureLinkTM HiPure Plasmid Maxiprep Kit (K210006, Thermo), other details of the transfection process can be found in the instruction of Sigma Transfections. 16 hours after transfection, culture medium was changed to the complete medium (purchased from Life Technologies, Cat# 11995-065). After culturing for 24 hours, 48 hours and 72 hours, the supernatants with lentivirus were collected, combined, and centrifuged at -80°C, 3000rpm for 10-15 minutes, then filtered through a 0.45 µm filter membrane, and finally ultra-centrifuged at 25000rpm, 4°C for 2-3 hours, so as to obtain the concentrate of lentivirus. The CD123-ER-TPD lentivirus concentrate was collected and stored at -80°C.

### Example 14: Preparation of CD123-targeted ER-TPD THP1 cells

The resuscitated THP1 cells were cultured in RMPI1640 medium supplemented with 5% FBS, in a 37°C, 5% CO₂ incubator. The medium was changed every 2-3 days, and the cell density was maintained at 1 × 10⁶ single cells/ml. After the cell state was recovered, the recovered cells were transfected with the CD123-ER-TPD lentivirus prepared in Example 13. The detailed steps are as follows: Dilute the THP1 cells to a concentration of 0.5 × 10⁶ single cells/ml, spread them in a 24-well plate, add the lentivirus concentrate prepared in Example 13 to the cells (MOI = 0.5 or 1), add Polybrene 5µg/ml as well, centrifuge at a low speed (500g-1000g/min) for 30-60 minutes in a flat-angle centrifuge, then culture the cells in an incubator at 37°C. THP1 cells expressing CD123 ER-TPD were obtained 48 hours after infection, the cell phenotype was determined by flow cytometry.

Fig. 11 shows the 48h flow cytometry results for the cells prepared in Example 13, *i.e.,* the acute myeloid leukemia cell line THP1 cells transfected with the CD123-targeted scFv TPD lentivirus. The THP1 cells cultured in RPMI medium were transfected with the following virus respectively: (1) anti-CD123 ERD-E3L (MOI=0.5); (2) anti-CD123 ERD-E3L (MOI=1); untransfected cells were used as control. 48 hours after transfection, 5 × 105 untransfected THP1 cells and virus transfected cells were treated with antibodies CD14-FITC, CD33-APC-Cy7, CD123-PE-Cy7 (all antibodies were purchased from Biolegend) for staining, followed by cell phenotyping by a flow cytometer (BD FACSCanto II). The results showed that the untransfected THP1 cells express CD14, CD33 and CD123 at high level; (1) in THP1 cells transfected with the anti-CD123 ERD-E3L (MOI=0.5), the expression level of CD14 and CD33 was maintained, while the expression level of CD123 was significantly decreased; (2) in THP1 cells transfected with the anti-CD123 ERD-E3L (MOI=1), the expression level of CD14 and CD33 was maintained, while the expression level of CD123 was significantly decreased.

### Example 15: Application of CD123-targeted ER-TPD-CD123 CAR-T cells

CD123 is a very important target for acute myeloid leukemia AML, and CAR-T cells targeting CD123 were prepared (Fig. 12A1). However, since activated T cells also express CD123 (Fig. 12B1), the CD123-targeted CAR-T cells will demonstrate suicide effect in each process of activation, cultivation and tumor cell killing (Fig. 12C1), resulting in limited cell proliferation and poor therapeutic effect in clinic.

The CD123 CAR-T-T2A-CD123 ER-TPD chimeric protein construct (Fig. 12A2) can be designed by way of the ER-TPD technology of the present disclosure, which can efficiently degrade the endogenous CD123 (Fig. 12B2), thereby ensuring good cell proliferation and achieving more precise tumor cell killing (FIG. 12C2). In a case of CAR-T targeting T-cell antigens, the self-antigen can be degraded in a targeted manner by combining with ER-TPD technology. The CAR and the ER-TPD (that target the same antigen) can be constructed in the same vector, so as to achieve both the degradation of self-antigen and the targeting activity of T cells by one single engineering of T cell. The transfected cells degrade the self-antigen in a targeted manner by ER-TPD chimeric protein construct, while untransfected cells express the antigen, therefore the transfected CAR-T cells will specifically eliminate the untransfected cells and the cell purity will be naturally increased along with the process of cell cultivation. In that case, the subsequent purification and screening steps can be skipped, the efficiency of large-scale production will be greatly improved. In addition, compared with conventional Crispr/cas9 technology, ER-TPD technology has advantages in low cost, high efficiency, and is easy to achieve large-scale production, while eliminate the side effects of chromosome instability or off-target effects caused by gene editing. Therefore, CAR/ER-TPD-T cells are superior in both cellular function and vitality. This combined CAR/ER-TPD technology can resolve clinical challenges well, allowing CAR-T to be applicable for the targets specifically expressed on T cells (such as CD4, CD5, CD7, CD38, CD123) without causing suicide effects. For example, it may be used for T-lymphoblastic leukemia (e.g., acute T-lymphoblastic leukemia (T-ALL) or chronic T-lymphoblastic leukemia (T-CLL), T-prolymphocytic leukemia (T-PLL), large granular lymphocytic leukemia (LGLL), myeloid leukemia (such as acute myeloid leukemia (AML), chronic myeloid leukemia (CML))), HIV infection, for clearing the virus-infected CD4 T cells.

### Example 16: Application of Tau/Aβ-targeted ER-TPD mRNA for neurodegenerative disease Alzheimer's disease

The intracellular accumulation of Tau and amyloid-β (Aβ) is the pathological hallmark of Alzheimer's disease (AD) and related tauopathies, so enhancing the targeting activity against Tau can facilitate drug development. Intracellular accumulation of hyperphosphorylated Tau (p-tau) and extracellular deposition of β-amyloid (Aβ) are the pathological hallmarks of the brain with Alzheimer's disease (AD). In the past few decades, AD drug development has mainly focused on Aβ. Therefore, various Aβ-targeted therapies have been developed and tested, such as reducing Aβ production by β-/γ-secretase inhibitors, promoting Aβ degradation or removing Aβ by antibodies. Unfortunately, none of the therapies targeting Aβachieved satisfied clinical efficacy to date. Therefore, therapies targeting Tau become important strategy for the treatment of AD. At present, drugs for the targeted degradation of Tau in brain cells have been developed based on PROTAC technology, but none achieves observable therapeutic effect in clinic since the molecules often cannot effectively enter the cells. However, the ER-TPD technology of the present disclosure can be used to develop and design mRNA or eRNA (endless cyclic RNA) encoding the Tau-targeted or β-amyloid (Aβ)-targeted ER-TPD chimeric protein constructs. The Tau-targeted and/or β-amyloid (Aβ)-targeted ER-TPD chimeric protein constructs may be delivered into neurons by neuron-targeted liposomes (LNP) or nanoparticles (NP), and generate stable expression to achieve efficient degradation of Tau and β-amyloid. In that case, it is possible to effectively prevent the degeneration of neurons and to slow the progress of Alzheimer's disease.

### Example 17: Application of HBx-targeted ER-TPD DNA for treatment of HBV Chronic hepatitis

Hepatocellular carcinoma (HCC) is one of the leading causes of cancer death in the modern world, and most cases are associated with chronic hepatitis B virus (HBV) infection. HBV-encoded X protein (HBx) is known to play a key role in the pathogenesis of viral HCC. HBx is a multifunctional protein that regulates the expression of various cellular and viral genes involved in cell survival, cell cycle progression, DNA repair, invasion, and protein degradation. It also regulates multiple signaling pathways (such as Ras/Raf/MAPK, PI3K/Akt, NF-κB and JNK). The ER-TPD technology of the present disclosure can be used to design and express the transposon DNA of the HBx-targeted ER-TPD chimeric protein construct. It may be delivered and integrated into liver cells by liver cell-targeted liposomes or nanoparticles. After integration, long-term degradation of the HBV oncogenic protein HBx may be achieved by the long-term expression of HBx ER-TPD chimeric protein constructs. In that case, it is possible to effectively prevent the proliferation of HBV and to eliminate the cytopathies and cancer progression caused by HBx.

### Example 18: Application of DNA Polymerase ER-TPD DNA for treatment of HBV Chronic hepatitis

HBV DNA polymerase: Hepatitis B virus (HBV) polymerase is a multifunctional enzyme with RNA-dependent and DNA-dependent polymerase functions, as well as ribonuclease H function. It converts the HBV pregenomic RNA (pgRNA) by way of reverse transcription, to form new rcDNA molecules within the new capsid. HBV DNA polymerase is a key component of the HBV viral capsid. The ER-TPD technology of the present disclosure can be used to design and express transposon DNA of the HBV DNA Polymerase-targeted ER-TPD chimeric protein construct. It may be delivered and integrated into liver cells by liver cell-targeted liposomes or nanoparticles. After integration, efficient degradation of the HBV DNA Polymerase may be achieved by the long-term expression of HBV DNA Polymerase ER-TPD chimeric protein construct. In that case, it directly inhibits the DNA replication after HBV infection and genomic integration, blocks HBV construction and packaging, thereby completely silencing the HBV gene.

### Example 19: Application of HIV RNA Reverse transcriptase ER-TPD DNA for treatment of HIV infection

HIV is a single-stranded RNA virus. In order to infect host cells, it is necessary to reverse-transcribe the single-stranded RNA genome into a double-stranded DNA copy by reverse transcriptase and to integrate the copy into the host chromosome. Therefore, targeting HIV RNA reverse transcriptase can effectively control the packaging and integration of active HIV The ER-TPD technology of the present disclosure can be used to design and express transposon DNA of the HIV-1 reverse transcriptase-targeted ER-TPD chimeric protein construct. It may be delivered and integrated into CD4⁺ cells by CD4⁺ cell-targeted liposomes or nanoparticles. After integration, targeted degradation of the HIV-1 reverse transcriptase may be achieved by the long-term expression of HIV-1 reverse transcriptase ER-TPD chimeric protein construct. In that case, it directly inhibits the virus packaging after HIV infection and genomic integration, thereby directly achieving the silencing of HIV genes.

### Example 20: Application of Bcl-2-targeted ER-TPD DNA for treatment of tumor

Apoptosis plays an important role in the occurrence, development and drug resistance of tumors. The expression and regulation of the Bcl-2 family have a greater impact on tumor cell apoptosis, and can cooperate with the proto-oncogene ras to cause malignant transformation of cells and promote tumor growth. Bcl-2 and/or Bcl-xL are overexpressed in many human tumors such as gastrointestinal tumors, lymphoma, neuroblastoma, bladder cancer and the like. Highly expressed Bcl-2 can inhibit cell apoptosis, accelerate cell growth, and lead to the occurrence of malignant tumors.

Overexpression of Bcl-2 protein is also common in non-solid tumors. Bcl-2 is highly expressed in chronic lymphocytic leukemia (CLL), follicular lymphoma (FL), mantle cell lymphoma (MCL) and Waldenström's macroglobulinemia (WM). Therefore, the anti-apoptotic protein Bcl-2 has become an emerging drug target in the field of tumor therapy. Bcl-2 anti-apoptotic members include Bcl-2, Bcl-xL, Bcl-W, MCl-1 and the like, sharing conserved and highly homologous BH1 and BH2 motifs. Therefore, it is possible to develop nanobodies against BH1 and BH2 motifs, to screen antibody sequences that broadly bind to Bcl-2 anti-apoptotic family members, and to design and express ER-TPD chimeric nucleic acid constructs targeting Bcl-2 anti-apoptotic family members (such as Bcl-2, Bcl-xL and Bcl- The of w), based on the ER-TPD technology. The chimeric nucleic acid construct is loaded on an oncolytic virus to obtain an oncolytic virus loaded with a Bcl-2-targeted ER-TPD chimeric nucleic acid construct. The oncolytic virus specifically infects tumor cells, and initiates the expression of an ER-TPD chimeric protein construct targeting Bcl-2 anti-apoptotic protein by a strong promoter on the oncolytic virus such as CMV, to achieve the efficient trageted degradation of Bcl-2 anti-apoptotic family members and to eliminate the anti-apoptotic mechanism of tumors. In that case, it directly induces the apoptosis of tumor cells, promotes the cell lysis and diffusion of oncolytic viruses, so as to kill and eliminate tumor cells.

For the same reason, it is possible to target oncolytic virus directly to the oncogene proteins, such as the mutant Kras protein ER-TPD, in order to achieve the degradation of the oncogene proteins.

### Example 21: Application of autoantibody-targeted ER-TPD chimeric protein construct for treatment of autoimmune diseases

Markers of beta cell autoimmunity in type I diabetes include four autoantibodies, i.e., islet cell antibody (ICA), glutamic acid decarboxylase antibody (GAD-65), insulin antibody (IAA) and tyrosine phosphate Enzyme antibody (IA-2A), which are the diagnostic markers for diabete occurrence and the factors inducing disease progression. Insulin antibody is usually the earliest risk marker of type I diabetes in children. 70% of pediatric patients have insulin antibody at the time of diagnosis, 85% have antibodies against GAD65. Clinically, it is extremely difficult to intervene or eliminate these autoantibodies. Transposon DNA of ER-TPD targeting the insulin autoantibody (IAA) can be designed based on the ER-TPD technology. It may be delivered and integrated into plasma cells by plasma cell-targeted liposomes or nanoparticles. After integration, the long-term expression of IAA ER-TPD chimeric protein construct may achieve long-term degradation of the insulin antibody, increase the utilization rate of insulin in peripheral blood, thereby effectively prevent the progress of diabetes. As shown in Fig. 13A, plasma cells in type I diabetes patients express and release insulin antibodies, which bind insulin and eliminate insulin from the peripheral blood, resulting in a significant decrease of the insulin utilization rate in patients. Fig. 13B shows that, the ER-TPD transposon DNA targeting the insulin autoantibodies may be delivered into plasma cells by plasma cell-targeted liposomes to achieve integration, long-term expression of IAA ER-TPD chimeric protein construct, and target binding and degradation of islet cell antibodies in endoplasmic reticulum, thereby ensuring the normal function of insulin in peripheral blood

### Example 22: UT Elements and Derivatives Thereof

Exemplary chimeric fusion proteins containing TCRαβ or HLA molecules binding moiety, one or more transmembrane and cytoplasmic domains of viral ER-resident protein in cassettes are illustrated in FIG. 14. For example, in FIG. 14A, the chimeric fusion proteins include the anti-TCRαβ HLA antibody fragment scFv, Hinge/linker (e.g. SEQ NO. 5. IgG4 hinge; and SEQ ID NO. 15 (Gly 4 Ser)2 and fused to any transmembrane domain and cytoplasmic domain of ER-resident glycoprotein, e.g. HCMV US2 (SEQ ID NO. 19 and NO.20). For example, in FIG. 14B, the chimeric fusion proteins include the anti-TCRαβ or HLA antibody fragment scFv, Hinge/linker (e.g. SEQ NO. 5. IgG4 hinge; and SEQ ID NO.15 (Gly 4 Ser)2 and fused to two or more transmembrane domain and cytoplasmic domain of ER-resident glycoprotein, e.g. HCMV US2 (SEQ ID NO. 19 and NO.20) and HCMV US3 (SEQ ID NO.20 and NO.21). For example, in FIG. 14C, the chimeric fusion proteins include the anti-TCRαβ or HLA antibody fragment scFv, Hinge/linker (e.g. SEQ NO. 5. IgG4 hinge; and SEQ ID NO. 15 (Gly 4 Ser)2 and fused to the recombinant motif of transmembrane domain and cytoplasmic domain derived from various ER-resident glycoprotein, e.g. HCMV US2 (SEQ ID NO. 19 and NO.20) and HCMV US11 (SEQ ID NO. 19, NO.20, NO.22, NO.23).

This UT elements encoding nucleic,' acid molecule was cloned into the lentiviral vector and then transduced into Jurkat cells or human T cells.

### Example 23: Transduced UT elements efficiently block TCRαβ expression in Jurkat cells.

An exemplary lentiviral construct encoding the UT element comprising anti-TCRαβ fused to the transmembrane and cytoplasmic domain of HCMV US2 or HCMV US11 or/and adenovirus E19, T2A self-cleavage peptide, truncated EGFR was designed (named anti-TCR-US2 TM/CT-T2A-EGFRt; anti-TCR-US11 TM/CT-T2A-EGFRt; anti-TCR-E19 TM/CT-T2A-EGFRt; anti-TCR-US2-TM/CT-T2A-anti-TCR-E19 TM/CT-T2A-EGFRt). The DNA was synthesized and cloned into the lentiviral vector (such as pLenti CMV GFP-puro) and lentivirus were produced in 293T cells, using the package vectors (such as psPAX and pMD2G). Jurkat cells were culture in RPMI, 10% human serum, 2 mM L-glutamine and 1% penicillin-streptomycin (CTL medium) and then transduced with a lentiviral supernatant (as indicated) (MOI=3) supplemented with 0.8 µg/mL polybrene (Millipore, Bedford, Mass.). After 3 days' expansion, the cells were submitted to flow cytometry to examine expression of truncated EGFR and TCRαβ in Jurkat cells (FIG. 15). The following conjugated antibodies were used for flow cytometric phenotyping and analysis: CD4-APC, CD8-Pacific Blue, TCR-ab-APC-Cy7, EGFR-PE (Biolegend). Staining with propidium iodide (PI, BD Biosciences) was performed for live/dead cell discrimination as directed by the manufacturer. Flow analyses were done on a FACS Canto II, sort-purifications on a FACS Ariall (Becton Dickinson, Franklin Lakes, N.J.) and data analyzed using FlowJo software (Treestar, Ashland, Oreg.) The result indicated the transduced cells expressed the truncated EGFR and significant downregulation of on TCRαβ on both CD8+ and CD4+ T cells, while the nontransduced Jurkat cells express high level of TCRαβ in absence of truncated EGFR.

### Example 24: Forced expression of UT elements could efficiently suppress TCR expression in human T cells.

In FIG. 16A, an exemplary lentiviral construct encoding the UT element comprising anti-TCRαβ fused to the transmembrane and cytoplasmic domain of HCMV US2, T2A self-cleavage peptide and truncated EGFR was designed (named anti-TCR-US2 TM/CT-T2A-EGFRt were cloned into the lentiviral vector (such as pLenti CMV GFP-puro) and lentivirus were produced in 293T cells, using the package vectors (such as psPAX and pMD2G). Human CD8+ and CD4+ were isolated from PBMC of normal donors using CD3+ T Cell Isolation Kit (Miltenyi Biotec), activated with anti-CD3/CD28 beads (Life Technologies) according to the manufacturer's instructions, and transduced with a lentiviral supernatant (as indicated in each Example) (MOI=3) supplemented with 0.8 µg/mL polybrene (Millipore, Bedford, Mass.) on day 3 after activation by centrifugation at 2,100 rpm for 45 min at 32° C. T cells were expanded in RPMI, 10% human serum, 2 mM L-glutamine and 1% penicillin-streptomycin (CTL medium), supplemented with recombinant human (rh) IL-2 to a final concentration of 50 U/mL every 48 hours. After 12 days' expansion, an aliquot of each transduced T cell line was stained with CD8-Pacific Blue, CD4-APC, TCR-ab-APC-Cy7, EGFR-PE (Biolegend) and then submitted to flow cytometry analysis.

The result indicates that the transduced T cells (EGFR+) significantly downregulate TCRαβ expression on both CD8+ and CD4+ cell surface compared to the nontransduced T cells. In conclusion, US elements containing anti-TCR-US2 TM/CT could efficiently suppress TCRαβ expression in human T cells which may inhibit TCR-mediated GVHD in vivo after transfusion into different recipient.

In FIG. 16B, an exemplary lentiviral construct encoding the UT element comprising anti-TCRαβ fused to the transmembrane and cytoplasmic domain of adenovirus E19, T2A self-cleavage peptide and truncated EGFR was designed (named anti-TCR-E19 TM/CT-T2A-EGFRt were cloned into the lentiviral vector (such as pLenti CMV GFP-puro) and lentivirus were produced in 293T cells. Human CD8+ and CD4+ were transduced with two dose of lentiviral supernatant (1×, 2×) and expanded in CTL medium, supplemented with recombinant human (rh) IL-2. After 12 days' expansion, an aliquot of each transduced T cell line was stained with CD8-Pacific Blue, CD4-APC, TCR-ab-APC-Cy7, EGFR-PE (Biolegend) and then submitted to flow cytometry analysis.

The result indicates that the UT(E19) transduced T cells (EGFR+) could also efficiently block TCRαβ expression on both CD8+ and CD4+ cell surface compared to the nontransduced T cells. Increase virus dose of transduction, the suppression of TCRαβ expression could be significantly improved with the increased transduction efficiency and copy number of UT elements in cells. In conclusion, US elements containing anti-TCR-E19 TM/CT could efficiently suppress TCRαβ expression in human T cells which may also inhibit TCR-mediated GVHD in vivo after transfusion into different recipient.

### Example 25: Phenotype of CNK-T Cells Expressing U-T Elements

In FIG. 17, an exemplary lentiviral construct encoding the CNK sequence, T2A sequence and UT element comprising anti-TCRαβ fused to the TM and CT domain of adenovirus E19, T2A self-cleavage peptide was designed (named CNK-UT-E19 were cloned into the lentiviral vector (such as pLenti CMV GFP-puro) and lentivirus were produced in 293T cells. Human CD8+ and CD4+ were transduced with two dose of lentiviral supernatant and expanded in CTL medium, supplemented with IL-2. After 12 days' expansion, an aliquot of each transduced T cell line was stained with CD8-Pacific Blue, CD4-APC, TCR-ab-APC-Cy7, NKG2D-PE-Cy7 (Biolegend) and then submitted to flow cytometry analysis. The results indicate inclusion of UT elements in CNK-T cells could significantly suppress TCRαβ expression in CNK-T cells without interfere with the expression of NKG2D in both CD8+ and CD4+ T cells.

### Example 26: Cytotoxicity of CNK-T Cells Expressing U-T Elements

The in vitro effector function of CD3+ bulk T cells engineered to express CNK, or CNK-UT were compared to nontransduced T cells respectively-in the co-culture assay with the HCC cell line, HepG2 cells. Briefly, 0.5×10⁶ HepG2 cells were seeded on 24 well plates 24 h before the co-culture. The HepG2 cells were added with 1×10⁵ effector cells (E: T=1:5) and incubated in the 37 degree for another 24 h before analysis. After co-culture, the cells were harvested and stained with CD8-Pacific Blue, CD4-APC, CD45-PerCP-Cy5.5, CD25-APC-Cy7 and CD137-PE-Cy7 (Biolegend) and then submitted to flow cytometry analysis. The results indicate both CNK-T and CNK-UT(E19) could efficiently eliminate HepG2 in the co-culture assay compared to non-transduced T cells (Fig. 18). In addition, CNK-UT cells significantly upregulate CD25 and CD137, the T cell activation markers, as regular CNK-T. In conclusion, inclusion of UT elements will not interfere T cell function, such as cytotoxicity against tumor cells.

### Example 27: Design of CNK-UT multifunctional complex

In this example, four CNK-UT multifunctional complexes were designed. Among them, the first structure is the basic CNK-UT multifunctional complex, and the other three structures are the CNK-UT multifunctional complex having more functions formed by adding other modules to the first structure.

### 3.1 Basic CNK-UT multifunctional complex

In some embodiments, the basic CNK-UT multifunctional complex is used, its schematic diagram is shown in Fig. 19A, which contains three modules: (1) NK activating receptor module, (2) CNK signal transduction module, and (3) UT module. Optionally, hinges or linkers are included between the NK activating receptor module, the CNK signal transduction module and/or the UT module.

Wherein,
(1) the NK activating receptor module at lease comprises the NK-cell-activating receptor or a functional variant thereof, the NK-cell-activating receptor comprises: (a) an extracellular domain (ED) of the NK-cell-activating receptor or a functional variant thereof, (b) a transmembrane domain (TMD) of the NK-cell-activating receptor or a functional variant thereof, and (c) an intracellular domain (ICD) of the NK-cell-activating receptor or a functional variant thereof; and optionally, a hinge or a linker is included between the extracellular domain of the NK-cell-activating receptor or functional variant thereof, the transmembrane domain of NK-cell-activating receptor or functional variant thereof, and/or the intracellular domain of the NK-cell-activating receptor or functional variant thereof.
   In some preferred embodiments, the CNK multifunctional complex comprises an amino acid sequence selected from the group consisting of SEQ ID NOs. 1-24.
(2) the CNK signal transduction module at lease comprises (i) a NK cell signal adaptor or a functional variant thereof, the NK cell signal adaptor comprises: (a) an extracellular domain (ED) of the NK cell signal adaptor or a functional variant thereof, (b) a transmembrane domain (TMD) of the NK cell signal adaptor or a functional variant thereof, and (c) an intracellular domain (ICD) of the NK cell signal adaptor or a functional variant thereof; optionally, a hinge or a linker is included between the extracellular domain of the NK cell signal adaptor or functional variant thereof, the transmembrane domain of the NK cell signal adaptor or functional variant thereof, and/or the intracellular domain of the NK cell signal adaptor or functional variant thereof.
(3) the UT module at lease comprises (1) a recombinant protein molecule for targeted degradation of TCR or a functional variant thereof, the recombinant protein molecule for targeted degradation of TCR comprises: (a) a binding protein molecule domain targeting TCR or a functional variant thereof, (b) a transmembrane domain of the viral endoplasmic reticulum (ER) resident glycoprotein or a functional variant thereof, and (c) a cytoplasmic domain of the viral endoplasmic reticulum resident glycoprotein or a functional variant thereof; the transmembrane domain of the viral endoplasmic reticulum resident glycoprotein or a functional variant thereof, and the cytoplasmic domain of the viral endoplasmic reticulum resident glycoprotein or a functional variant thereof form the ERAD degradation domain; optionally, a hinge or a linker is included between the binding protein molecule domain targeting TCR or a functional variant thereof, the transmembrane domain of the viral endoplasmic reticulum resident glycoprotein or a functional variant thereof, and/or the cytoplasmic domain of the viral endoplasmic reticulum resident glycoprotein or a functional variant thereof.

In the recombinant protein molecule for targeted degradation of TCR that comprised in the UT module, the binding protein molecule domain targeting TCR or the functional variant thereof is derived from a TCR antibody or a functional variant thereof or the combination thereof.

In some preferred embodiments, the antibody is selected from TCRα antibody, TCRβ antibody, TCRαβ antibody, TCRγ antibody, TCR6 antibody, TCRγδ antibody, TCR Vδ2 antibody, TCR Cβ1 antibody; the functional variant of the antibody is selected from Fd, Fv, Fab, Fab', F(ab')₂, Fv(scFv), single-chain antibody (scFv) or nanobody, diabody, tribody and tetrabody; preferably, the TCR antibody is TCR single-chain antibody. In some preferred embodiments, the binding protein molecule targeting TCR is TCRαβ antibody. In some preferred embodiments, the amino acid sequence of the TCR single-chain antibody comprises the amino acid sequence set forth by SEQ ID NO. 113.

In some preferred embodiments, the ERAD degradation domain that comprised in the UT module is derived from HCMV glycoprotein US2, US3, US11, US10, adenovirus E3-Kl 9, or HHV-7 US21.

In some preferred embodiments, the ERAD degradation domain that comprised in the UT module comprises the amino acid sequence set forth by SEQ ID NOs. 46-63.

Fragments of genetic elements of the various module in the basic CNK-UT multifunctional complex can be synthesized by gene synthesis techniques. Using polycistronic expression system, the genetic element fragments of different module are linked by the elements such as self-cleaving 2A peptide (2A: T2A, p2A, E2A, F2A), to obtain the gene fragment of the basic CNK-UT multifunctional complex; the synthesized gene fragment of the basic CNK-UT multifunctional complex was cloned into a lentiviral vector by molecular cloning, and used for transfecting T cells, to simultaneously express the multiple different elements, thereby enabling T cells not only to specifically recognize and kill the NK target, but also effectively inhibit and degrade TCR with the help of the UT elements. Alternatively, the simultaneous expression of multiple CNK-UT elements can be achieved by transfecting T cells with a lentiviral vector comprising the different functional elements under different promoters. Alternatively, the simultaneous expression of multiple CNK-UT elements can also be achieved by simultaneously transfecting T cells with two different lentiviral vectors each comprising different functional elements.

### 3.2 CNK-UT multifunctional complex comprising binding protein molecule domain that targeting MHC

In some embodiments, a CNK-UT multifunctional complex that simultaneously degrades or inhibits TCR and MHC I and/or MHC II is used, its schematic diagram is shown in Fig. 19B, which also contains three modules: (1) NK activating receptor module, (2) CNK signal transduction module, and (3) UT module that comprising both a recombinant protein molecule domain for targeted degradation of TCR or a functional variant thereof and a binding protein molecule domain targeting MHC I and/or MHC II or a functional variant thereof. It differs from the basic CNK-UT multifunctional complex in that (3) the UT module further comprises a binding protein molecule domain targeting MHC I and/or MHC II or a functional variant thereof. Optionally, hinges or linkers are included between the NK activating receptor module, the CNK signal transduction module, and the UT module.

In some embodiments, the binding protein molecule domain targeting MHC I and/or MHC II or the functional variant thereof is a binding protein molecule domain targeting HLA or the functional variant thereof.

In some embodiments, the binding protein molecule domain targeting MHC I may be derived from a viral endoplasmic reticulum protein that inhibits MHC I molecules. In some embodiments, the viral endoplasmic reticulum glycoprotein that inhibits MHC I molecules is selected from human cytomegalovirus (HCMV) US6, herpes simplex virus (HSV) ICP47, cowpox virus (CPXV) CPXV12, bovine herpesvirus (BHV) UL49.5, Epstein Barr virus (EBV) BNFL2a and the like. The above-mentioned viral endoplasmic reticulum protein binds to the transporter associated with antigen processing (TAP), thereby preventing TAP-mediated polypeptide translocation to the endoplasmic reticulum and inhibiting the component of MHC molecules, thereby achieving the inhibition of MHC I expression. In some preferred embodiments, the binding protein molecule domain targeting MHC I and/or MHC II or the functional variant thereof.comprises a TAP binding domain. In some preferred embodiments, the TAP binding domain comprises an amino acid sequence selected from SEQ ID NO. 64-73.

In some embodiments, the binding protein molecule domain targeting MHC I may be derived from a viral glycoprotein that degrades MHC I molecules. In some embodiments, the viral glycoprotein is selected from HCMV glycoprotein US2, US3, US 11, US10, adenovirus E19 and the like. In some preferred embodiments, the binding protein molecule domain targeting MHC I and/or MHC II or the functional variant thereof.comprises a MHC binding domain. In some preferred embodiments, the MHC binding domain comprises an amino acid sequence selected from SEQ ID NO. 74-82.

In some embodiments, the binding protein molecule domain targeting MHC I may be derived from a viral protein that targeted inhibits or degrades NK target proteins such as MICA, MICB, ULBP1-6 and the like. In some embodiments, the viral protein is selected from HCMV UL16, UL141, UL142, adenovirus E3-19K9 and the like. In some preferred embodiments, the amino acid sequence of the viral protein is the amino acid sequence set forth by SEQ ID NO. 83-91.

In some embodiments, the binding protein molecule domain targeting MHC I may be derived from a viral protein that translocate MHC I molecule from Glogi apparatus to lysosome for degradation. In some embodiments, the viral protein is selected from HIV Nef, HIV Vpu, HHV-7U21, HHV-8 KK3, HHV-8 KK5, MHV-68 MK3, HTLV-1 p12 and the like. In some preferred embodiments, the viral protein comprises the amino acid sequence set forth by SEQ ID NO. 92-97.

In some embodiments, the binding protein molecule domain targeting MHC I and/or MHC II or the functional variant thereof may comprise a viral protein that mediates the returning of MHC-polypeptide molecule from Glogi apparatus to endoplasmic reticulum and promotes the degradation of the molecule. Preferably, the viral protein comprises a MHC binding domain and a KDEL receptor binding domain. In some embodiments, the viral protein is Cowpox Virus protein CPXV203. In some preferred embodiments, the viral protein comprises the amino acid sequence set forth by SEQ ID NO. 98-103.

The CNK-UT multifunctional complex further comprising a binding protein molecule domain for targeting MHC I was obtained in the same manner as obtaining the basic CNK-UT multifunctional complex. The synthesized gene fragment of the CNK-UT multifunctional complex was cloned into a lentiviral vector by molecular cloning, and used for transfecting T cells, so as to simultaneously express the multiple different elements, thereby enabling T cells not only to specifically recognize and kill the NK target, but also effectively inhibit and degrade TCR and MHC I with the help of the UT elements.

### 3.3 CNK-UT multifunctional complex further comprising both binding protein molecule domain targeting MHC I and chimeric adaptor

In some embodiments, a multifunctional complex that is able to achieve the simultaneous degradation of TCR and MHC I and the tumor antigen-specific recognition and activation of CNK-T cell is used, its schematic diagram is shown in Fig. 19C, which contains four modules: (1) NK activating receptor module, (2) CNK signal transduction module, (3) UT module, and (4) chimeric adaptor module. It differs from the CNK-UT multifunctional complex comprising binding protein molecule domain that targeting NHC I in that it further comprises (4) the chimeric adaptor module. Optionally, the NK activating receptor module, the CNK signal transduction module, the UT module, and/or the chimeric adaptor module are linked with a hinge or a linker.

(4) The chimeric adaptor module comprises: (i) an extracellular recognition domain targeting tumor, (ii) a transmembrane domain, and (iii) an intracellular signaling transduction domain. Optionally, hinges or linkers are included between the extracellular recognition domain targeting tumor, the transmembrane domain and/or the intracellular signaling transduction domain.

In some embodiments, the extracellular recognition domain targeting tumor of the chimeric adaptor module is selected from a tumor antigen-specific binding domain, an antigen binding domain targeting tumor microenvironment and/or a chemotactic receptor targeting tumor microenvironment.

In some embodiments, the extracellular recognition domain targeting tumor is selected from antibodies that can target and recognize a tumor associated antigen, or functional fragments thereof, TCR or a combination thereof. The functional fragment of the antibody is selected from Fd, Fv, Fab, Fab', F(ab')₂, Fv(scFv), single-chain antibody (scFv) or nanobody, diabody, tribody and tetrabody.

In some embodiments, the transmembrane domain of the chimeric adaptor module is selected from a transmembrane domain of NK-cell-activating receptor, a transmembrane domain of DAP10, a transmembrane domain of DAP12, a transmembrane domain of CD8, a transmembrane domain of CD28, a transmembrane domain of CD4, a transmembrane domain of 4-1BB, a transmembrane domain of OX40, a transmembrane domain of ICOS, a transmembrane domain of CTLA-4, a transmembrane domain of PD-1, a transmembrane domain of LAG-3, a transmembrane domain of 2B4 and a transmembrane domain of BTLA, and a combination thereof.

In some embodiments, the intracellular signaling transduction domain of the chimeric adaptor module comprises an intracellular signaling transduction domain and/or a co-stimulatory signaling transduction domain of NK-cell-activating receptor.

In some embodiments, the intracellular signaling transduction domain further comprises a co-stimulatory signaling transduction domain. Preferably, the co-stimulatory signaling transduction domain is selected from T cell co-stimulatory signaling transduction domain, including but not limited to the intracellular signaling transduction domain derived from MHC class I molecule, TNF receptor protein, immunoglobulin-like protein, cytokine receptor, integrin, signaling lymphocytic activation molecule (SLAM protein), activated NK cell receptor, BTLA, receptor of Toll ligand, OX40, CD2, CD7, CD16, CD27, CD28, CD30, CD40, CD38, CD35, CD79A, CD79B, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, NCR, DAP10, DAP12, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100SEMA4D), CD69, SLAMF6 (NTB-A, Ly108), SLAM (SLAMF1, CD150, IPO-3), BLAME (SLAMF8), SELPLG (CD 162), LTBR, LAT, GADS, SLP-76, PAG/Cbp, CD 19a and receptor specifically binding to CD83, CARD11, FcRa, FcRp, FcRy, Fyn, HVEM, ICOS, Lck, LAG3, LAT, LRP, NOTCH1, Wnt, OX40, ROR2, Ryk, SLAMF1, Slp76, pTa, TCRa, TCRp, TRIM, ZAP70, PTCH2 and the like. More preferably, the co-stimulatory signaling transduction domain is selected from an intracellular signaling transduction domain of NKG2D, an intracellular signaling transduction domain of DAP10, an intracellular signaling transduction domain of DAP12, an intracellular signaling transduction domain of NCR, an intracellular signaling transduction domain of CD28, an intracellular signaling transduction domain of 4-1BB, an intracellular signaling transduction domain of OX40, an intracellular signaling transduction domain of ICOS.

The CNK-UT multifunctional complex further comprising a binding protein molecule domain targeting MHC I and a chimeric adaptor was obtained in the same manner as obtaining the basic CNK-UT multifunctional complex. The synthesized gene fragment of the CNK-UT multifunctional complex was cloned into a lentiviral vector by molecular cloning, and used for transfecting T cells, so as to simultaneously express the four different elements, thereby enabling T cells not only to specifically recognize and kill the NK target, but also to effectively inhibit and degrade TCR and MHC I with the help of the UT elements, and also to specifically recognize tumor antigens and activate by way of the chimeric adaptor.

### 3.4 CNK-UT multifunctional complex further comprising both binding protein molecule domain targeting MHC I and receptors with targeted killing activity against tumor cells (such as CAR or TCR)

In some embodiments, a multifunctional complex that is able to achieve the simultaneous degradation of TCR and MHC I and the tumor antigen-specific recognition and activation is used, its schematic diagram is shown in Fig. 19D, which contains four modules: (1) NK activating receptor module, (2) CNK signal transduction module, (3) UT module, and (4) receptor module with targeted killing activity against tumor cells. It differs from the CNK-UT multifunctional complex comprising binding protein molecule domain that targeting MHC I in that it further comprises (4) receptor module with targeted killing activity against tumor cells. Optionally, the NK activating receptor module, the CNK signal transduction module, the UT module, and/or the receptor module with targeted killing activity against tumor cells are linked with a hinge or a linker.

(4) The receptor module with targeted killing activity against tumor cells comprises (i) an extracellular recognition domain targeting tumor antigen, (ii) a transmembrane domain, and (iii) an intracellular co-stimulatory signaling transduction domain, (iv) a T cell activation signaling transduction domain (ITAM). Optionally, hinges or linkers are included between the extracellular recognition domain targeting tumor antigen, the transmembrane domain, the intracellular co-stimulatory signaling transduction domain and/or the T cell activation signaling transduction domain (ITAM).

The transmembrane domain of the receptor module with targeted killing activity against tumor cells is selected from a transmembrane domain of CD8, a transmembrane domain of α chain and/or β chain of T cell receptor, a transmembrane domain of CD28, a transmembrane domain of CD3ε, a transmembrane domain of CD45, a transmembrane domain of CD4, a transmembrane domain of CD5, a transmembrane domain of CD8, a transmembrane domain of CD9, a transmembrane domain of CD16, a transmembrane domain of CD22, a transmembrane domain of CD33, a transmembrane domain of CD37, a transmembrane domain of CD64, a transmembrane domain of CD80, a transmembrane domain of CD86, a transmembrane domain of CD134, a transmembrane domain of CD137, a transmembrane domain of CD 154, a transmembrane domain of GITR, and a combination thereof.

The T cell activation signaling transduction domain is derived from an intracellular signaling transduction domain of CD3ζ, common FcRy (FCER1G), FcyRIIa, FcRβ, CD3γ, CD36, CD3ε, CD5, CD22, CD79a, CD79b, CD278 ("ICOS"), FcεRI CD66d, DAP10 and DAP12, and the like.

In some preferred embodiments, (4) the receptor module with targeted killing activity against tumor cells is a CAR or TCR receptor with targeted killing activity against tumor cells

The gene fragment of CNK-UT multifunctional complex further comprising a binding protein molecule domain for targeting MHC I and CAR or TCR receptor with targeted killing activity against tumor cells was obtained in the same manner as obtaining the basic CNK-UT multifunctional complex. The synthesized gene fragment of the CNK-UT multifunctional complex was cloned into a lentiviral vector by molecular cloning, and used for transfecting T cells, so as to simultaneously express the four different elements, thereby not only enabling T cells to specifically recognize and kill the NK target, to effectively inhibit and degrade TCR and MHC I with the help of the UT elements, but also achieving the specific recognition and killing effect against tumor cells by way of the CAR or TCR structure, and also enhancing the efficiency of clearing tumor cells by cooperating with the CNK element.

### Example 28: Design of CNK-UT element for expressing CNK-UT multifunctional complex

In this example, four CNK-UT element for expressing CNK-UT multifunctional complex were designed.

### 4.1 CNK-UT element structure design 1

In some embodiments, as shown in Fig. 20A, expressing multiple functional elements by one single vector is achieved by a combination of CNK-UT elements: DAP10-DAP12 ICD-T2A-NKG2D-p2A-anti-TCR-AdE3 ERAD, the combination is obtained by using a single lentiviral expression vector with EF1α promoter and elements such as the self-cleaving 2A peptide.

### 4.2 CNK-UT element structure design 2

In some embodiments, as shown in Fig. 20B, expressing multiple functional elements by one single vector is achieved by a combination of CNK-UT elements: DAP10-DAP12 ICD-T2A-NKG2D-p2A-anti-TCR-US2 ERAD, the combination is obtained by using a single lentiviral expression vector with EF1α promoter and elements such as the self-cleaving 2A peptide.

### 4.3 CNK-UT element structure design 3

In some embodiments, as shown in Fig. 20C, expressing multiple functional elements by one single vector is achieved by the polycistronic expression system with double promoters, which comprises both an EF1α promoter and a CMV promoter in a single lentiviral expression vector, the expression of multi-genes DAP10-CD3 ζ -T2A-NKG2D-p2A-anti-TAA scFv-DAP10 and anti-TCR-AdE3 ERAD-E2A-AdE3 are respectively regulated by the promoters.

### 4.4 CNK-UT element structure design 4

In some embodiments, as shown in Fig. 20D, expressing multiple functional elements in the same T cell is achieved by co-transfecting the T cell with different lentivirus, which are prepared from two different lentiviral expression vectors that respectively regulate the expression of anti-TAA scFv-CD28/4-1BB-CD3ζ-T2A-DAP10-CD3ζ-T2A-NKG2D and anti-TCR-AdE3 ERAD-T2A-AdE3.

### Example 29: Preparation of expression plasmid of specific chimeric antigen receptor targeting NKG2DL

Whole gene synthesis was carried out for the coding nucleotide sequences of CNK-UT (the nucleotide sequence is shown in SEQ ID NO. 118; the encoded protein thereof is shown in SEQ ID NO. 117). The nucleotide sequence was inserted into the lentiviral vector pCDH-CMV-MCS-EF1α-Puro (purchased from Youbao Biotech) or pLVX-EF1α-AcGFP1-C1 Vector (Takara) by molecular cloning to construct CNK-UT lentiviral target vector. The obtained plasmids (which were confirmed by sequencing) were used to transform DH5α competent cells (Thermo Fisher). Single colonies were picked and cultured in large-scale. Then, the plasmids were purified and produced by using the PureLinkTM HiPure Plasmid Maxiprep Kit (Thermo Fisher), to obtain the CNK-UT lentiviral plasmid.

### Example 30: Preparation of virus of CNK-UT

293T cells (provided by ATCC, Cat# CRL3216TM) were co-transfected by the CNK-UT lentiviral plasmid obtained in Example 29 as well as the packaging plasmids psPAX2 and pMD2.G (purchased from Addgene, Cat# 12259&12260) at the ratio of 1.64pmol: 1.3pmol: 0.72pmol. Polyethyleneimine (408727, Sigma) was used as the transfection reagent, at the ratio of DNA:µg PEI=1:3. The details for the preparation of the packaging plasmids can be found in the instruction of PureLinkTM HiPure Plasmid Maxiprep Kit (K210006, Thermo), other details of the transfection process can be found in the instruction of Sigma Transfections. 16 hours after transfection, culture medium was changed to the complete medium (purchased from Life Technologies, Cat# 11995-065). After culturing for 24 hours, 48 hours and 72 hours, the supernatants with lentivirus were collected, combined, and centrifuged at -80°C, 3000rpm for 10-15 minutes, then filtered through a 0.45µm filter membrane, and finally ultra-centrifuged at 25000rpm, 4°C for 2-3 hours, so as to obtain the concentrate of lentivirus. The lentivirus concentrate was collected and stored at -80°C.

### Example 31: Preparation of CNK-UT cells

Collect fresh peripheral blood from healthy donors, add PBS+EDTA 1: 1, and separate fresh peripheral blood mononuclear cells by Ficoll density gradient centrifugation. Then, CD3⁺ T cells were positively sorted and eluted by utilizing CD3 sorting magnetic beads, MS separation column and MiniMACS^{™} separation device (Miltenyi Biotec). The isolated CD3⁺ T cells were stimulated by culturing with anti-CD3/anti-CD28 antibody-conjugated magnetic beads (Human T-Activator CD3/CD28, Invitrogen, Cat# 11161D), according to the steps as follows: Dilute the peripheral blood mononuclear cells to a concentration of 1 × 10⁶ single cells/ml, spread them in a 24-well plate, add the magnetic beads to the cells at a ratio of 1:1 and mix well. The mixture was resuspended in culture medium (OpTmizerTM T-Cell Expansion SFM, A1048503, Life Technologies) containing IL2 50U/ml and IL15 5ng/ml, and cultured in a 37°C, 5% CO₂ incubator for 1 day. Then, the cells were transfected with the lentivirus prepared in Example 4 that loaded the CNK-UT elements, according to the steps as follows: add the lentivirus concentrate prepared in Example 4 to the cells (MOI=3-5), add Polybrene 10µg/ml as well, centrifuge at a low speed (500g-1000g/min) for 30-60 minutes in a flat-angle centrifuge, then culture the cells in an incubator at 37°C. Cells expressing CNK-UT were obtained 48 hours after infection, which may be used for cell phenotyping by flow cytometry. Cells cultured for another 8 days may be used for phenotype detection and cellular function experiments.

### Example 32: Phenotype detection of CNK-UT cells

Fig. 21 shows the flow cytometry results for the phenotyping of CNK-UT cells (DAP10-CD3ζ-T2A-NKG2D-p2A-anti-TCR scFv-AdE3) (the nucleotide sequence is shown in SEQ ID NO. 118; the encoded protein thereof is shown in SEQ ID NO. 117). Cells prepared in Example 2 were cultured for 10 days and collected for 5 × 10⁵ untransfected T cells and CNK-UT virus vector transfected cells. The cells were treated with antibodies CD8-Pacific Blue, CD4-APC, NKG2D-PE-Cy7, TCR-αβ-APC-Cy7 (all antibodies were purchased from Biolegend) for staining, followed by cell phenotyping by a flow cytometer (BD FACSCanto II). The results showed that for the untransfected T cells, the CD8 T cells express NKG2D and TCR-αβ, the CD4 T cells do not express NKG2D, but highly express TCR-αβ; while for the cells transfected with modified CNK-UT, both the CD8 and the CD4 T cells highly express NKG2D, but not express TCR-αβ.

### Example 33: Detection of broad-spectrum tumor-killing function of CNK-UT cells

In this example, the broad-spectrum tumor-killing function of CNK-UT cells prepared in Example 5 were studied. Human colon adenoma cell line HT29 was purchased from Procell (CL-0118), triple negative breast cancer cell MDA-MB453 was purchased from Procell (CL-0152), acute myeloid leukemia cell line THP1 was purchased from Procell (CL-0233), hepatocellular carcinoma (HCC) HepG2 was purchased from Procell (CL-0103), hepatocellular carcinoma (HCC) PLC was purchased from Procell (CL-0415).

### 33.1 CNK-UT cells recognize and specifically kills human colonic adenoma cell line HT29

The CNK-UT cells used in this example are the cells prepared in Example 5.

Fig. 22 shows the CNK-UT cells recognize and specifically kill human colon adenoma cell line HT29.

### A: Expression of different NK target proteins in human colonic adenoma cell line HT29

For Human colonic adenoma cell line HT29, 0.5×10⁶ cells were digested with trypsin, and respectively treated with antibodies MICA-APC, MICB-APC, ULBP1-APC, ULBP2-APC, ULBP3-APC for staining, while the IgG isotype (purchased from R&D Systems) was used as the control. Then, the cell phenotypes were determined by a flow cytometer (BD FACSCanto II). The results showed that HT29 cells highly express ULBP2, but weakly express MICA, MICB, ULBP1 and ULBP3.

### B: CNK-UT cells are activated by HT29 and have efficient killing effect on HT29

In the co-culture experiment of T cells and tumor cells, count 0.5×10⁶ cells/well of the human colon adenoma cell line HT29 and spread them in a 24-well plate, culture the cells until cell density reaches 80% confluent. Add respectively the untransfected T cells and CNK-UT cells (DAP10-CD3ζ-T2A-NKG2D-p2A-ant-TCR scFv-AdE3) at the effector-to-target ratio of 1:5 into the tumor cell. After 24 hours, all cells were digested and harvested, and treated with antibodies CD45-PerCP-Cy5.5, CD8-Pacific Blue, CD4-APC, NKG2D-PE-Cy7, CD25-APC-Cy7 (purchased from Biolegend) for staining, followed by detection by a flow cytometer (BD FACSCanto II). To analyze the results, CD45(-) tumor cells and CD45(+) T cells were distinguished by CD45, and the surface expression of NKG2D and the activation marker CD25 on CD8, CD4 T cells. The experimental data showed that 59.0% of HT29 cells have upregulated expression of MICA, 40.1% have upregulated expression of MICB, 56.1% have upregulated expression of ULBP1, 84.4% have upregulated expression of ULBP2, 23.7% have upregulated expression of ULBP3 (Fig. 22A), CNK-UT cells have good killing effect on HT29 cells. In the control group of the co-culture system, 69.5% of the cells are tumor cells, T cells do not express CD25. However, in the co-culture system of CNK-UT cells, according to the FSC/SSC results, most tumor cells have been eliminated, the proportion of T cell has significantly increased upto 84.1%, 36.345% of CD8⁺T cells have upregulated expression of CD25, and 19.08% of CD4⁺ T cells have upregulated expression of CD25 (Fig. 22B).

### 33.2 CNK-UT cells recognize and specifically kill MDA-MB453

The CNK-UT cells used in this example are the cells prepared in Example 5.

Fig. 23 shows the CNK-UT cells recognize and specifically kill MDA-MB453.

### A: Expression of different NK target proteins in triple-negative breast cancer cell line MDA-MB453

For MDA-MB453, 0.5×10⁶ cells were digested with trypsin, and respectively treated with antibodies MICA-APC, MICB-APC, ULBP1-APC, ULBP2-APC, ULBP3-APC for staining, while the IgG isotype (purchased from R&D Systems) was used as the control. Then, the cell phenotypes were determined by a flow cytometer (BD FACSCanto II). The results showed that MDA-MB453 cells highly express MICA, ULBP2, but weakly express MICB, ULBP1 and ULBP3.

### B: CNK-UT cells are activated by MDA-MB453and have efficient killing effect on MDA-MB453

In the co-culture experiment of T cells and tumor cells, count 0.5×10⁶ cells/well of the breast cancer cell line MDA-MB453 and spread them in a 24-well plate, culture the cells until cell density reaches 80% confluent. Add respectively the untransfected T cells and CNK-UT cells (DAP10-CD3ζ-T2A-NKG2D-p2A-ant-TCR scFv-AdE3) at the effector-to-target ratio of 1:5. After 24 hours, all cells were digested and harvested, and treated with antibodies CD45-PerCP-Cy5.5, CD8-Pacific Blue, CD4-APC, NKG2D-PE-Cy7, CD25-APC-Cy7 (purchased from Biolegend) for staining, followed by detection by a flow cytometer (BD FACSCanto II). The experimental data showed that 83.7% of MDA-MB453 cells have upregulated expression of MICA, 33.6% have upregulated expression of MICB, 31.2% have upregulated expression of ULBP1, 95.0% have upregulated expression of ULBP2, 6.23% have upregulated expression of ULBP3 (Fig. 23A), CNK-UT cells have good killing effect on MDA-MB453 cells. In the control group of the co-culture system, 48.9% of the cells are tumor cells, T cells do not express CD25. However, in the co-culture system of CNK-UT cells, tumor cells only account for 6.74%, 14.8% of CD8⁺T cells have upregulated expression of CD25, and 28.1% of CD4⁺ T cells have upregulated expression of CD25 (Fig. 23B). The experimental data showed that CNK-UT cells have good killing effect on MDA-MB453 cells, and can achieve specific activation.

### Example 34: Functional detection of CNK-UT cells in tumor models

In this example, immunodeficiency mice (NSG mice, purchased from Jiangsu Jicui Yaokang Biotechnology Co., Ltd., Cat# T001475) were used as the tumor-bearing model to perform in vivo pharmacodynamic studies.

### 8.1 Therapeutic effect of CNK-UT cells on the animal model of liver cancer cell line HepG2

Experimental animals were divided into three groups as follow:
Negative control group: Administer control T cells, T cells obtained from the same donor, untransfected T cells proliferated after the stimulation of magnetic beads;
Isotype control group: Administer conventional GPC3 CAR-T cells; the GPC3 CAR-T cells were prepared as follows: plasmid and lentivirus were prepared by cloning the synthesized anti-GPC3 41BB-CD3ζ gene into lentiviral vector CDH-CW-MCS-EF1α-Puro, and were used for transfecting T cells to obtain the conventional GPC3 CAR-T cells. The specific preparation method was the same as that of CNK-UT cells.
CNK-UT group: Administer GPC3 CAR/CNK-UT cells.

The GPC3 CAR/CNK-UT cells were prepared as follows: plasmid and lentivirus were prepared by cloning the synthesized anti-GPC3 41BB-Z-T2A-DAP10-CD3ζ-T2A-NKG2D-p2A-ant-TCR scFv-AdE3 gene into lentiviral vector CDH-CMV-MCS-EF1α-Puro, and were used for transfecting T cells. After proliferation and cultivation, the GPC3 CAR/CNK-UT cells were obtained. The specific preparation method was the same as that of CNK-UT cells.

The experimental animals were divided into groups, and respectively administered the control T cells, conventional GPC3 CAR-T cells and GPC3 CAR/CNK-UT cells. The animals were weighed. 3 × 10⁶ HepG2 cells were inoculated into 15 NSG mice in right armpit, the inoculation day was recorded as day 0. After 7 days, it was observed that all mice were successfully modeled. The mice were divided into 3 groups, and given primary T cells from normal donor (control T cells), conventional GPC3-CAR-T cells, and GPC3 CAR/CNK-UT cells, respectively. The clearance of in vivo tumor fluorescence by the cells was observed. Cells were administered by tail vein injection at the dosage of 2 × 10⁶ per mouse for three times (*i.e.,* on Day 7, 9 and 11, respectively). The first administration date was recorded as Day 7, and the fluorescence was observed every 7 days after Day 7.

Fig. 24 shows that GPC3 CAR/CNK-UT cells have higher tumor clearance ability in mice than GPC3 CAR-T cells.

Establish the model by hepatocarcinoma cell line HepG2-Ffluc. Then, 2 × 10⁶ untransfected T cells (left), GPC3 CAR-T (middle) and GPC3 CAR/CNK-UT (right) were administered, and the tumor growth was monitored weekly by the IVIS animal fluorescence imaging system. The experimental results showed that the fluorescence in the control T cell group continuely increased, the fluorescence in GPC3 CAR-T cell group increased slowly, while the tumor fluorescence in CNK-T001 group kept decreasing until disappeared. The results confirmed that GPC3 CAR/CNK-UT cells have more significant antitumor effect than conventional CART cells in the mouse model.

Fig. 25 shows that GPC3 CAR/CNK-UT cells have higher tumor clearance ability than GPC3 CAR-T cells.

In view of the tumor fluorescence curve plotted on the basis of quantification of the fluorescence image of Fig. 25, it can be seen that the control T cells (blue) and GPC3 CAR-T (red) are unable to completely inhibit tumor growth, however (green) can significantly inhibit tumor growth. The tumor fluorescence gradually decreased until invisible, confirming the antitumor effect of GPC3 CAR/CNK-UT cells in mouse models.

The results of the above-mentioned experiments were as follows. In the negative control mice, the tumor size gradually increased, the first death case appeared between Day 28 and Day 42, and none could survive after Day 42. In the isotype control group with administration of GPC3 CAR/CAR-T cells, tumor growth was observed to be inhibited on Day 14, but no tumor was completely eliminated; an increase of tumor size was observed on Day 21; and almost no difference compared to the negative control group could be observed after Day 28; all mice were died between Day 42 and Day 56; indicating that single-target GPC3 CAR-T could only partially inhibit the tumor, but could not completely clear it, let alone prevent recurrence. In the CNK-UT group with administration of GPC3 CAR/CNK-UT cells, the in vivo tumor fluorescent signal was significantly decreased on Day 14, and completely disappeared on Day 21; all mice survived until the cut-off day (Day 56) with no tumor recurrence. The results confirmed that GPC3CAR/CNK-UT cells have good anti-tumor activity in vivo, which is significantly superior to that of GPC3 CAR-T, and such anti-tumor effect can be maintained for at least 56 days even after the tumors were removed.

In addition, the body weight changes of mice after infusion of different cells were also studied. Compared with the body weight on Day 0, the experimental animals in the CNK-UT group showed a slight increase in body weight after the infusion of CNK-UT cells, while animals in both the isotype control group (infused with GPC3-CAR-T) and the negative control group (infused with control T cells) showed a gradual decrease in body weight as the experiment progressed.

Based on the above-mentioned experimental results, it is suggested that after different treatments, tumors in mice injected the control T cells or GPC3 CAR-T cells kept growing, while injection of GPC3 CAR/CNK-UT cells could eliminate tumor cells in 21 days without reoccurrence, since GPC3 CAR/CNK-UT cells have superior killing activity compared to GPC3 CAR-T cells. In addition, infusion of GPC3 CAR/CNK-UT causes no cytotoxicity, the animals have prolonged survival period, improved living quality and increased body weight.

## Claims

1. A chimeric protein construct comprising an endoplasmic reticulum-associated degradation (ER-associated degradation, ERAD) mechanism protein binding domain and a targeting domain.

2. The chimeric protein construct of claim 1, wherein the ERAD mechanism protein binding domain comprises: a transmembrane domain of a viral endoplasmic reticulum resident protein or a functional variant thereof, and,
an endoplasmic reticulum resident domain or a functional variant thereof.

3. The chimeric protein construct of claim 1 or 2, wherein the viral endoplasmic reticulum resident protein is adenovirus E3-19K.

4. The chimeric protein construct of claim 1 or 2, wherein the viral endoplasmic reticulum resident protein is not adenovirus E3-19K.

5. The chimeric protein construct of claim 4, wherein the viral endoplasmic reticulum resident protein is selected from at least one of the group consisting of: HCMV glycoprotein US2, US11, US3, US10, US6, HSV ICP47, CPXV12, BHV UL49.5, EBV BNFL2a, HCMV UL16, UL141, UL142, HIVNef, HIV Vpu, HHV-7 U21, HHV-8 KK3, HHV-8 KK5, MHV-68 MK3, HTLV-1 p12, Cowpox Virus protein CPXV203.

6. The chimeric protein construct of any one of the preceding claims, further comprising a protein degradation pathway member (e.g. E3 ubiquitin ligase, proteasome, lysosome) binding domain, and optionally, the protein degradation pathway member binding domain being linked to the ERAD mechanism protein binding domain.

7. The chimeric protein construct of any one of the preceding claims, wherein the targeting domain comprises an antibody specifically targeting a target protein or a functional fragment thereof (e.g., Fd, Fv, Fab, Fab', F(ab')2, Fv(scFv), single chain antibody (scFv), nanobody, diabody, triabody or tetrabody).

8. The chimeric protein construct of claim 7, wherein the target protein is a pathogenic protein, optionally, being a tumor-associated protein, a virus-associated protein, an immune function-related protein (including immunosuppressive and immune stimulatory proteins), an autoantigen protein, or a protein associated with neurodegenerative diseases.

9. The chimeric protein construct of claim 8, wherein the autoantigen protein is selected from the group consisting of:
an autoantigen associated with type 1 diabete, e.g., an islet cell antigen (ICA), an insulin (IAA), a glutamine acid decarboxylase 65 (GAD65), an insulinoma antigen-2 (IA-2);
an autoantigen associated with rheumatoid arthritis (RA), e.g., a citrullinated protein/peptide antibody, a heteroribonucleoprotein (heterogeneous nuclear ribonucleoprotein A2B 1), an aldolase, an alpha-enolase, a calreticulin, a heat-activated protein (HSP60), BiP, PGK1, a stress-induced phosphoprotein 1, FUSE-BP1/2;
an autoantigen associated with systemic lupus erythematosus (SLE), e.g., a deoxyribonucleoprotein, SmD1, SmD3, Clq, a sore anticoagulant (LA), cardiolipin (CL), β2 glycoprotein I (β2 GPI), a prothrombin (PT) and phosphatidylserine (PS);
an autoantigen associated with systemic sclerosis (SSc)/scleroderma (SD), e.g., Scl-70, SSA, Ro52;
an antigen associated with autoimmune liver diseases, e.g., mitochondrial antigen, Spl00, PML, gp210, p62;
an autoantigen associated with myasthenia gravis, e.g., an acetylcholine receptor;
an autoantigen associated with central nervous system autoimmune disease (limbic encephalitis, encephalomyelitis, cerebellar ataxia), e.g., voltage-gated potassium channels (VGKC) complex, voltage-gated calcium channel receptor, a-amino-3-hydroxy-5-methylisoxazole-4-propionic acid (AMPA) receptor, γ-aminobutyric Acid-B (GABAB) receptors, glycine receptors;
an autoantigen associated with multiple sclerosis, e.g., a myelin basic protein (MBP), a myelin oligodendrocyte glycoprotein (MOG);
an autoantigen associated with polymyositis(PM) and dermatomyositis (DM), e.g., Jo-1, Mi-2, PM-Scl, Ro-52;
an autoantigen associated with gluten-sensitive enteropathy, e.g., endomysial antibody (EMA), tissue transglutaminase (tTG);
an autoantigen associated with anti-NMDAR antibody encephalitis, e.g., N-methyl-D-aspartate receptor;
an autoantigen associated with neuromyelitis optica (NMO), e.g., aquaporin 4 (AQP4); and,
an autoantigen associated with reproduction, e.g., ovarian antigen, sperm antigen.

10. The chimeric protein construct of claim 9, wherein the tumor-associated protein is encoded by an oncogene selected from the group consisting of: BCL-2, c-MYC, Ras, HER2, BCR/ABL, ABL1/BCR, TGFB1, TLX1, P53, WNT1, WNT2, WT1, αv-β3, PKCa, ABL, BCL1, CD24, CDK4, EGFR/ERBB- 1. HSTF1, INT1/WNT1, INT2, MDM2, MET, MYB, MYC, MYCN, MYCL1, RAFI, NRAS, REL, AKT2, APC, BCL2-ALPHA, BCL2-BETA, BCL3, BCR, BRCA1, BRCA2, CBL, CCND1, CDKN1A, CDKN1C, CDKN2A, CDKN2B, CRK, CRK-II, CSF1R/FMS, DBL, DDOST, PMS-2, PRAD-1, RAF, RHOM-1, RHOM-2, SIS, TAL2, TANI, TIAM1, TSC2, TRK, TSC1, STK11, PTCH, MEN1, MEN2, P57/KIP2, PTEN, HPC1, ATM, XPA/XPG, BCL6, DEK, AKAP13, CDH1, BLM, EWSR1/FLI1, FES, FGF3, FER, FGR, FLI1/ERGB2, FOS, FPS/FES, FRA1, FRA2, FYN, HCK, HEK, HER3/ERBB-2, ERBB-3, HER4/ERBB-4, HST2, INK4A, INK4B, JUN, JUNB, JUND, KIP2, KIT, KRAS2A, KRAS2B, LCK, LYN, MAS, MAX, MCC, MLH1, MOS, MSH2, MYBA, MYBB, NF1, NF2, P53, PDGFB, PIM1, PTC, RBI, RET, ROS1, SKI, SRC1, TALI, TGFBR2, THRA1, THRB, TIAM1, TRK, VAV, VHL, WAF1, WNT2, WT1, YES1, ALK/NPM1, AMI1, AXL, FMS, GIP, GLI, GSP, HOX11, HST, IL3, INT2, KS3, K- SAM, LBC, DCC, DPC4/SMAD4, E-CAD, E2F1/RBAP, ELK1, ELK3, EPH, EPHA1, E2F1, EPHA3, ERG, ETS1, ETS2, LMO-1, LMO-2, L-MYC, LYL1, LYT-10, MDM-2, MLH1, MLL, MLM, N-MYC, OST, PAX-5, PMS-1, FGF4, FGF6, FANCA, FLI1/ERGB2, FOSL1, FOSL2, GLI, HRAS1, HRX/MLLT1, HRX/MLLT2, KRAS2, MADH4, MASI, MCF2, MLLT1/MLL, MLLT2/HRX, MTG8/RUNX1, MYCLK1, MYH11/CBFB, NFKB2, NOTCH1, NPM1/ALK, NRG/REL, NTRK1, PBX1/TCF3, PML/ RARA, PRCA1, RUNX1, RUNX1/CBFA2T1, SET, SHP2, TCF3/PBX1, TNFa, Clusterin, Survivin, TOEβ, c-fos, c-SRC, Estrogen receptor gene (estrogen receptor ER-α), androgen receptor gene (Androgen receptor, AR) and INT-1, optionally, the tumor-associated protein is selected from the group consisting of: Bcl-2 family members ( such as: Bcl-2, Bcl-xL and Bcl-w), VEGF/VEGFR, PDGFRβ, EGFR, EGFR mutants, IGF-1R, HDACs, HER2, MYC, KRAS, AFP, CEA, CA199, estrogen receptor ( estrogen receptor ER-α), androgen receptor (androgen receptor, AR), tyrosine kinases (c-ABL, BCR-ABL, BTK, FAK, PTK6, Wee1, TRK transmembrane receptors), serine/threonine kinase receptors (IRAK4, LRRK2, B-Raf, RIPK2, CDK4/6, CDK7, CDK8, CDK8/19, CDK9, TBK1), protein kinase II (CK2), epigenetic related proteins (BRD2, BRD3, BRD4, BRDT, TRIM24, BRD9, PBRM1, SMARCA2, SMARCA4, EP300, EZH2, WDR5), adrenomedullin (ADM), DPP3.

11. The chimeric protein construct of claim 8, wherein the pathogenic protein is a virus-associated protein selected from the group consisting of: HBV surface antigen, HBV capsid glycoprotein, HBeAg, HBV DNA polymerase, HBV encoded X protein (HBx), HIV Gag protein, HIVEnv protein, HIV gp120, HIV-1 reverse transcriptase, HIV gp120, HCVNS3-4A protease, HCV RNA polymerase, HCV envelope protein, EBV DNA polymerase, EBV EBNA1 , coronavirus RNA synthetase, coronavirus spike protein, coronavirus envelope protein, coronavirus membrane protein, coronavirus nucleocapsid protein, RNA-dependent RNA polymerase (RdRp), such as the novel coronavirus RNA dependent RNA polymerase, Herpesviruses DNA and RNA polymerase, Herpesvirus capsid glycoprotein, CMV DNA polymerase, CMV capsid glycoprotein, RSV envelope protein, RSV capsid protein, RSV RNA polymerase, influenza virus RNA polymerase, influenza virus envelope protein, HPV DNA polymerase, and HPV capsid protein.

12. The chimeric protein construct of claim 7, wherein the target protein is an immune function-related protein selected from the group consisting of: an antigen-presenting molecule (e.g., a MHC class I molecule, a MHC class II molecule, a MICA/B molecule, etc.), an antigen recognition molecule (e.g., TCR, CD123, NKG2D, etc.), an immune checkpoint molecule (e.g., PD-1, PD-L1, CTLA4, TIM3, TIGIT, LAG3, A2AR, BTLA, IDO1, IDO2, TDO, KIR, NOX2, VISTA, SIGLEC7, PVR, etc.), an immune stimulatory/co-stimulatory molecule (e.g., CD3, CD80/86, CD28, etc.).

13. The chimeric protein construct of claim 7, wherein the target protein is a target protein related to nervous system diseases, and is selected from the group consisting of: Tau, β-amyloid protein (amyloid-β (Aβ)), α synuclein, mutant huntingtin (mHTT), α-synuclein, TAR RNA binding protein (TARDBP) and FUS RNA binding protein (FUS).

14. The chimeric protein construct of claim 2, 4 or 5, wherein the targeting domain does not specifically binding TCR.

15. The chimeric protein construct of claim 14, wherein the targeting domain specifically binds MHC I, MHC II, MICA, MICB, ULBP1, ULBP2, ULBP3, ULBP4, ULBP5 and/or ULBP6; and wherein preferably, MHC I is HLA I; and wherein, preferably, MHC II is HLA II.

16. The chimeric protein construct of claim 4, wherein the targeting domain specifically binds a TCR.

17. The chimeric protein construct of claim 16, wherein the virus endoplasmic reticulum resident protein is selected from at least one of the group consisting of: HCMV glycoprotein US2, US11, US3, US10, US6, HSV ICP47, CPXV12, BHV UL49.5, EBV BNFL2a, HCMV UL16, UL141, UL 142, HIV Nef, HIV Vpu, HHV-7 U21, HHV-8 KK3, HHV-8 KK5, MHV-68 MK3, HTLV-1 p12, and Cowpox Virus protein CPXV203.

18. The chimeric protein construct of any one of the preceding claims, which is linked to at least one co-expression moiety.

19. The chimeric protein construct of claim 18, wherein the at least one co-expression moiety is independently selected from the group consisting of: an intact viral ER resident glycoprotein (e.g., HCMV US2, US3, US11, US10, adenovirus E3-Kl 9, HCMV US6, HSV ICP47), a chimeric antigen receptor (CAR), a functional T cell receptor (TCR), a chemokine receptor, or a NK-cell-activating receptor.

20. The chimeric protein construct of claim 19, wherein the chemokine receptor is selected from the group consisting of: CCR4, CCR5, CCR6, CCR7, CCR9, CCR2b, CXCR1, CXCR2, and CXCR4.

21. The chimeric protein construct of claim 20, wherein the NK-cell-activating receptor comprises: (a) an extracellular domain (ED) of the NK-cell-activating receptor or a functional variant thereof, (b) a transmembrane domain (TMD) of the NK-cell-activating receptor or a functional variant thereof, and (c) an intracellular domain (ICD) of the NK-cell-activating receptor or a functional variant thereof; and wherein, optionally, a hinge or linker is included among the extracellular domain of the NK-cell-activating receptor or a functional variant thereof, the transmembrane domain of NK-cell-activating receptor or a functional variant thereof, and/or the intracellular domain of the NK-cell-activating receptor or a functional variant thereof.

22. The chimeric protein construct of claim 21, wherein the NK-cell-activating receptor is selected from the group consisting of NKG2D, NKG2C, NKG2E, NKG2F, NKG2H, CD94, KIR2DL4, KIR2DS1, KIR2DS2, KIR2DS4, KIR3DS1, a natural cytotoxic receptor, TRAIL, DNAM-1, CD16a, 2B4, NTB-A, CRACC, and NKp80.

23. The chimeric protein construct of claim 21 or 22, wherein the NK-cell-activating receptor is complexed with a CNK signal transduction module.

24. The chimeric protein construct of any one of claims 18-23, wherein the at least one co-expression moiety is linked to the chimeric protein construct by a cleavable linker.

25. A nucleic acid molecule encoding the chimeric protein construct of any one of claims 1-24.

26. The nucleic acid molecule of claim 25, wherein the nucleic acid molecule is deoxyribonucleic acid (DNA), ribonucleic acid (RNA) (for example, mRNA, circular RNA, ccRNA), threose nucleic acid (TNA), glycol nucleic acid (GNA), peptide nucleic acid (PNA), locked nucleic acid (LNA, including LNA with β-D-ribose configuration, α-LNA with α-L-ribose configuration (diastereoisomers of LNA), 2'-amino-LNA with 2'-amino-functionalization and 2'-amino-α-LNA with 2'-amino-functionalization), ethylene nucleic acid (ENA), cyclohexenyl nucleic acid (CeNA), and/or chimeras and/or combinations thereof.

27. A vector comprising the nucleic acid molecule of claim 25 or 26, wherein the nucleic acid molecule is operably linked to at least one polynucleotide regulatory element to express a chimeric protein construct encoded by the nucleic acid molecule.

28. The vector of claim 27, wherein the vector is selected from the group consisting of: a plasmid, a nanoplasmid, a cosmid, a viral vector (e.g., an oncolytic viral vector), a minicircle, a RNA vector, or a linear or circular DNA (e.g., a transposon DNA) or RNA molecule.

29. The vector of claim 28, wherein the vector is a viral vector selected from the group consisting of retrovirus, lentiviral vector, adenovirus, parvoviruse (e.g., adeno-associated virus), adeno-associated virus (AAV) vector, coronavirus, negative-strand RNA virus such as orthomyxovirus (e.g., influenza virus), rhabdovirus (e.g., rabies and vesicular stomatitis viruses), paramyxovirus (e.g., Machi and Sendai), positive-strand RNA Virus such as picornavirus and alphavirus, and double-stranded DNA virus, wherein the double-stranded DNA virus comprises adenovirus, herpesvirus (e.g., herpes simplex viruses I and II, Epstein-Barr virus, cytomegalovirus) and pox virus (e.g., vaccinia, fowlpox, and canarypox), Norwalk, togavirus, flavivirus, reovirus, papovavirus, hepadnavirus, baculovirus, and hepatitis virus , virus-like particle (VLP).

30. The vector of claim 29, wherein the viral vector is a retroviral vector.

31. The vector of claim 30, wherein the retroviral vector is selected from the group consisting of: avian leukoproliferative-sarcoma, mammalian C-type, B-type virus, D-type virus, HTLV-BLV collection, lentivirus, bubble virus.

32. The vector of claim 29, wherein the viral vector is a lentiviral vector.

33. The vector of claim 32, wherein the lentiviral vector is selected from the group consisting of HIV-1, HIV-2, SIV, FIV, BIV, EIAV, CAEV or ovine demyelinating leukoencephalitis lentivirus.

34. The vector of any one of claims 27-33, wherein the vector is further combined with other vectors comprising other nucleic acid molecules encoding at least one co-expression moiety.

35. An engineered cell expressing the chimeric protein construct of any one of claims 1-25, or comprising the nucleic acid molecule of claim 26, or comprising the vector of any one of claims 27-33.

36. The engineered cell of claim 35 expressing the chimeric protein construct of any one of claims 1-18, and a co-expression moiety.

37. The engineered cell of claim 35 or 36, wherein the cell is an immune cell selected from the group consisting of: a T cell, a natural killer (NK) cell, a B cell, a macrophage, a monocyte, a dendrites cell, a neutrophil, or a γδT cell.

38. The engineered cell of claim 37, wherein the T cell is selected from the group consisting of: CD8+ T cell, CD4+ T cell, cytotoxic T cell, terminal effector T cell, memory T cell, naive T cell, cell group composed of regulatory T cell, natural killer T cell, gamma-delta T cell, cytokine-induced killer (CIK) T cell, and tumor infiltrating lymphocyte.

39. A method for producing the engineered cell of any one of claims 35-38, comprising introducing the vector of any one of claims 27 to 34 into a starting cell, under a condition suitable for expressing the nucleic acid molecule of claim 25 or 26.

40. The method of claim 39, wherein the starting cell is a stem cell or a cell differentiated from a stem cell.

41. The method of claim 38, wherein the stem cell is a hematopoietic progenitor cell (e.g. T cell progenitor, NK cell progenitor, macrophage progenitor), a hematopoietic stem cell (HSC), a CD34+ cell, an embryonic cell line, a mesenchymal stem cell or an iPSC cell.

42. The method of claim 37, wherein the cell is an immune cell.

43. The method of claim 40, wherein the immune cell is a T cell, a natural killer (NK) cell, a B cell, a macrophage, a monocyte, or a dendritic cell.

44. The method of claim 41, wherein the cell is a T cell selected from the group consisting of: a CD4+ T cell, a CD8+ T cell, a cytotoxic T cell, a terminal effector T cell, a memory T cell, a naive T cell, cells composed of regulatory T cells, a natural killer T cell, a gamma-delta T cell, a cytokine-induced killer (CIK) T cell, and a tumor infiltrating lymphocyte.

45. A cell population produced ex vivo by the method of any one of claims 39-44.

46. A pharmaceutical composition or kit, comprising:
(i) the chimeric protein construct of any one of claims 1-24, or the nucleic acid molecule of claim 25 or 26, or the vector of any one of claims 27-34, or the engineered cell of any one of claims 35-38 or the cell population of claim 45, and
(ii) pharmaceutically acceptable medium.

47. A method for degrading a target protein, comprising delivery of the vector of any one of claims 27-34 into a cell expressing the target protein.

48. The method of claim 47, wherein the cell is selected from the group consisting of: a tumor cell, a virus-infected cell, a neural cell, a transplanted cell, and an immune cell.

49. The method of claim 47 or 48, wherein the delivery comprises delivery by a viral vector (e.g., an oncolytic virus).

50. The method of claim 49, wherein the vector expresses the chimeric protein construct of any one of claims 1-24 in the cell.

51. The method of claim 50, wherein the chimeric protein construct is capable of simultaneously binding a target protein and an ERAD mechanism protein, thereby degrading the target protein.

52. A method of treating a condition or disease in a subject in need thereof, comprising:
administering a therapeutically effective amount of the pharmaceutical composition of claim 46 to the subject.

53. The method of claim 52, wherein the disease comprises various solid tumors and hematological tumors, viral infectious diseases, autoimmune diseases, and neurological and degenerative diseases (e.g., Alzheimer's disease), metabolic diseases (e.g., diabetes (e.g., type II diabete), lipid metabolism-related diseases (e.g., tumor lipid metabolism, non-alcoholic fatty liver), atherosclerosis (AS), tumor glucose metabolism);
preferably, the solid tumor is selected from the group consisting of nervous system tumors, head and neck tumors, chest tumors, digestive system tumors, genitourinary system tumors, soft tissue and skin tumors, bone tumors, etc.;
preferably, the nervous system tumors comprise diffuse glioma, diffuse astrocytoma and anaplastic astrocytoma, glioblastoma, oligodendroglioma, oligoastrocytoma, diffuse Gliomas, other astrocytomas, ependymomas, neuronal and mixed neuronal-glial tumors, medulloblastoma, other embryonal tumors, schwannomas, meningiomas, solitary fibrous tumors and hemangiopericytoma, etc.;
preferably, the head and neck tumors comprise malignant tumors of the nasal cavity and sinuses, nasopharyngeal cancer, oral cancer, laryngeal cancer, salivary gland tumors, intracranial tumors, thyroid cancer, tongue cancer, etc.;
preferably, the thoracic tumors comprise lung cancer, esophageal cancer, cardia cancer, breast cancer, mediastinal tumors, etc.;
preferably, the digestive system tumors comprise gastric cancer, colorectal cancer, sigmoid colon and rectal cancer, liver cancer, pancreatic cancer and periampullary cancer, biliary tract cancer, malignant tumors of the small intestine, etc.;
preferably, the genitourinary system tumors comprise kidney cancer, prostate cancer, bladder cancer, testicular cancer, penile cancer, cervical cancer, endometrial cancer, ovarian cancer, etc.;
preferably, the soft tissue and skin tumors comprise malignant fibrous histiocytoma, rhabdomyosarcoma, synovial sarcoma, malignant melanoma of the skin, etc.;
preferably, the bone tumors comprise osteosarcoma, Ewing's sarcoma, etc.;
preferably, the colon cancer is colon adenoma;
preferably, the breast cancer is a triple-negative breast cancer cell;
preferably, the liver cancer is hepatocellular carcinoma;
preferably, the disease is a hematological tumor selected from the group consisting of leukemia, lymphoma (HL), multiple myeloma (MM), myelodysplastic syndrome (MDS), etc.;
preferably, the leukemia is B-cell acute lymphoblastic leukemia, T-cell acute lymphoblastic leukemia, acute myeloid leukemia, etc.;
preferably, viral infectious diseases comprise: respiratory viral diseases, gastrointestinal viral diseases, liver viral diseases, skin and mucous membrane viral diseases, ocular viral diseases, central nervous system viral diseases, lymphocytic viral diseases, insect-borne viral diseases, lentivirus infection diseases, etc.;
preferably, the respiratory viral diseases comprise infections of rhinovirus, adenovirus, respiratory syncytial virus, parainfluenza virus, and coronavirus; influenza; mumps, etc.; preferably, the gastrointestinal viral diseases comprise polio; cooksackie virus infection; ECHO virus infection; and viral gastroenteritis including rotavirus gastroenteritis, Norwalk virus gastroenteritis, adenovirus gastroenteritis, astrovirus gastroenteritis, coronavirus gastroenteritis and calicivirus gastroenteritis, etc.;
preferably, the liver viral diseases comprise viral hepatitis A, viral hepatitis B, viral hepatitis C, viral hepatitis D, viral hepatitis E, Epstein-Barr viral hepatitis, and cytomegalovirus hepatitis, etc;
preferably, the skin and mucous membrane viral diseases comprise measles, rubella, infantile acute rash, chickenpox and herpes zoster, smallpox, herpes simplex virus infection, rabies and foot-and-mouth disease, etc.;
preferably, the ocular viral diseases comprise epidemic keratoconjunctivitis, follicular conjunctivitis and herpetic keratoconjunctivitis, etc.;
preferably, the central nervous system viral diseases comprise Japanese encephalitis, western equine encephalitis, eastern equine encephalitis, St. Louis encephalitis, Venezuelan equine encephalitis, Murray Valley encephalitis, Californian encephalitis, forest encephalitis and lymphocytic choroid plexus meningitis, etc.;
preferably, the lymphocytic viral diseases comprise infectious mononucleosis, cytomegalovirus infection and acquired immunodeficiency syndrome, etc.;
preferably, the insect-borne viral diseases comprise viral hemorrhagic fevers including epidemic hemorrhagic fever, yellow fever, Crimean-Congo hemorrhagic fever, Rift Valley fever, Argentine hemorrhagic fever, Bolivian hemorrhagic fever, Lassa fever, Omu Scrubs hemorrhagic fever, Marburg disease and Ebola hemorrhagic fever, etc.; dengue fever and dengue hemorrhagic fever; West Nile fever; Colorado tick heat transfer; and sandfly fever, etc.; preferably, the lentiviral infection diseases comprise subacute sclerosing panencephalitis, Kuru disease, progressive multifocal leukoencephalopathy and subacute spongiform encephalopathy (corticostriatal spinal cord degeneration), etc.;
preferably, the autoimmune diseases comprise organ-specific autoimmune diseases and systemic autoimmune diseases;
preferably, the organ-specific autoimmune diseases comprise chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritic syndrome, vulgaris Pemphigus, pemphigoid, primary biliary cirrhosis, multiple sclerosis, acute idiopathic polyneuritis, etc.;
preferably, the systemic autoimmune diseases comprise systemic lupus erythematosus, rheumatoid arthritis, systemic vasculitis, scleroderma, pemphigus, dermatomyositis, mixed connective tissue disease, autoimmune hemolytic anemia, thyroid autoimmune diseases, ulcerative colitis, etc.;
preferably, the neurological diseases comprise nervous system diseases, peripheral nerve diseases such as trigeminal neuralgia, facial paralysis, hemifacial spasm, vestibular neuronitis, glossopharyngeal neuralgia, mononerve disease, brachial plexus neuralgia, multiple mononeuropathy, multiple Neuropathy, acute inflammatory demyelinating polyneuropathy, chronic inflammatory demyelinating polyneuropathy; spinal cord diseases, e.g., myelitis, compressive myelopathy, subacute combined degeneration of the spinal cord, syringomyelia, spinal cord vascular disease, spinal cord arachnoiditis, etc.; cerebrovascular diseases, e.g., transient ischemic attack, cerebral infarction, cerebral hemorrhage, subarachnoid hemorrhage, intracranial venous system thrombosis, etc.; infectious diseases of the central nervous system, e.g., meningitis, encephalitis, etc. caused by infections of viruses, bacteria, fungi, or parasites, etc., and lentiviral encephalitis caused by lentiviral infections; central nervous system demyelinating diseases, e.g., multiple sclerosis, neuromyelitis optica, acute disseminated encephalomyelitis, leukodystrophy, etc.; movement disorders, e.g., Parkinson's disease, chorea, hepatolenticular degeneration, dystonia, essential tremor, tardive dyskinesia, etc. Epilepsy; headaches such as migraines, tension headaches, cluster headaches, etc.;
nervous system degenerative diseases, e.g., motor neuron disease, Alzheimer's disease, Lewy body dementia, frontotemporal dementia, multiple system atrophy, etc.; inherited nervous system diseases, e.g., hereditary ataxia, hereditary spastic paraplegia, Charcot-Marie-Tooth disease, neurofibromatosis, tuberous sclerosis, cerebral hemangiomatosis, etc.;
neurodevelopmental disorders, e.g., congenital hydrocephalus, cerebral palsy, basilar depression, cerebellar subtonsillar disease, etc.; neuromuscular junction and muscle diseases, e.g., myasthenia gravis, periodic paralysis, polymyositis, progressive muscular dystrophy, myotonic myopathy (myotonic dystrophy, myotonia congenita), metabolic myopathy (mitochondrial myopathy, lipid storage myopathy, glycogenosis), etc.; autonomic nervous system diseases, e.g., Raynaud's disease, erythromelalgia, hemifacial atrophy, generalized autonomic insufficiency, spontaneous hyperhidrosis, progressive lipodystrophy, etc.; nervous system tumors, e.g., glioma, lymphoma, meningioma, etc.:
paraneoplastic syndromes of the nervous system, e.g., paraneoplastic cerebellar degeneration, paraneoplastic encephalomyelitis, subacute necrotizing myelopathy, subacute motor neuron disease, paraneoplastic sensory neuron disease, etc.;
preferably, metabolic diseases comprise diabetes, hyperlipidemia, gout and the like.

54. A method of stimulating an immune response in a subject, said method comprises administering an effective amount of the pharmaceutical composition of claim 46 to the subject.
